# EUROPEAN PATENT APPLICATION

(11) **EP 4 717 351 A1**
(43) Date of publication of application: **01.04.2026**
(21) Application number: 24203113.6
(22) Date of filing: 27.09.2024
(51) Int. Cl.: B01J 20/32, A61M 1/36, B01D 15/38

(54) **A WHOLE-BLOOD COMPATIBLE DOUBLE-PEGYLATED MATRIX FOR EXTRACORPOREAL BLOOD TREATMENT**

(71) Applicant: Gambro Lundia AB, 220 10 Lund (SE); Rapp Polymere GmbH, 72072 Tuebingen (DE)
(72) Inventor: Kalmutzki, Markus Johannes, 72379 Hechingen (DE); Loercher, Joachim, 72116 Mössingen (DE); Storr, Markus, 70794 Filderstadt (DE); Rempfer, Martin, 72810 Gomaringen (DE); Schuetz, Stefanie, 72458 Albstadt (DE); Schowner, Roman, 72076 Tübingen (DE); Rapp, Wolfgang, 72070 Tübingen (DE); Boschetti Castro, Adriana, 72379 Hechingen (DE)
(74) Representative: Hertin und Partner Rechts- und Patentanwälte PartG mbB

(57) **Abstract**

The invention relates to a whole blood compatible matrix for coupling a ligand and use in extracorporeal blood treatment, said matrix comprising a solid substrate, said substrate comprising a first layer of polyethylene glycol (PEG) and at least a second layer of polyethylene glycol, wherein said at least second layer is positioned outside the first layer and is bound to said first layer, and wherein the polyethylene glycol of the at least second layer comprises a terminal functional group for coupling said ligand to said polyethylene glycol. The invention further relates to a blood treatment device in the form of an adsorption cartridge or column, comprising the matrix according to the present invention, wherein the matrix is whole blood compatible. The invention further relates to the matrix of the invention comprising a ligand coupled thereto (apheresis matrix). The invention further relates to a method of preparing an apheresis matrix, comprising coupling the polyethylene glycol of the at least second layer of the matrix to the ligand. The invention further relates to a ligand coupled to the polyethylene glycol of the apheresis matrix according to the invention for use in the treatment of a medical condition by extracorporeal blood treatment.

## Description

The invention relates to the field of matrices and solid phases for ligand coupling, intended for use in extracorporeal blood treatment.

The invention relates to a whole blood compatible matrix for coupling a ligand and use in extracorporeal blood treatment, said matrix comprising a solid substrate, said substrate comprising a first layer of polyethylene glycol (PEG) and at least a second layer of polyethylene glycol, wherein said at least second layer is positioned outside the first layer and is bound to said first layer, and wherein the polyethylene glycol of the at least second layer comprises a terminal functional group for coupling said ligand to said polyethylene glycol (hereinafter also termed "double-PEGylated matrix").

The invention further relates to a blood treatment device in the form of an adsorption cartridge or column, comprising the matrix according to the present invention, wherein the matrix is whole blood compatible.

The invention further relates to the matrix of the invention comprising a ligand coupled thereto (herein termed apheresis matrix).

The invention further relates to a method of preparing an apheresis matrix, comprising coupling the polyethylene glycol of the at least second layer of the matrix to the ligand, wherein the polyethylene glycol can be linear, branched, Y-shaped, or forked.

The invention further relates to a ligand coupled to the polyethylene glycol of the apheresis matrix according to the invention for use in the treatment of a medical condition by extracorporeal blood treatment, wherein said extracorporeal blood treatment comprises transporting blood from the patients' vascular system to said apheresis matrix without further separating the blood in its components and returning the treated blood back to the patient.

### BACKGROUND OF THE INVENTION

A broad range of therapeutic approaches involving blood treatment are known in the art, which typically remove, manipulate, or utilize blood components for various medical purposes. Blood treatment strategies include both direct and indirect manipulation methods. Direct manipulation involves modifying blood components like red blood cells, platelets or plasma, immune modulation, or removing unwanted or pathological molecules or cells from the blood. Indirect manipulation methods typically use the blood as a platform to deliver therapeutic agents or diagnostic tools to other parts of the body. Blood treatment approaches are applicable in the treatment of a wide range of diseases, including for example inflammatory conditions, organ support or replacement such as hemodialysis, autoimmune diseases, cancer, infectious diseases, organ transplantation, and degenerative diseases. The advantages of blood treatment include, amongst others, targeted therapy that avoids systemic administration of drugs and its side-effects, enhanced efficacy, potential for long-lasting effects, and personalized medicine tailored to individual disease characteristics.

Apheresis is a medical technology in which the blood of a subject is passed through an apparatus that separates or modifies particular constituents and returns the remainder of blood to the circulation. Apheresis techniques rely on different processes depending on the desired outcome. For example, selective adsorption facilitates the precise targeting and removal of specific blood components through immobilization. Alternatively, molecular targets can undergo modification through enzymatic reactions or conversion. In the process of dialysis, the selection of components for removal from blood is typically determined by their size or other physical properties, and these components are extracted through a semipermeable membrane within the dialyzer.

Selective whole-blood apheresis procedures represent a specialized approach designed to target specific molecules, antibodies, cellular elements, or other blood components, in various diseases. These procedures offer a notable advantage over conventional therapeutic plasmapheresis because they are a targeted, less invasive approach having reduced complexity as well as improved overall efficacy and safety.

Hemocompatibility of apheresis matrices, on the other hand, is a critical prerequisite for biomedical materials employed in apheresis. The interaction of biomaterials with blood can trigger platelet adsorption/immobilization and aggregation, potentially initiating the coagulation cascade culminating in fibrin structure formation. The demand for diverse biomaterials, particularly hemocompatible polymers, is imperative in medical applications. Hemocompatible polymers play a pivotal role in preventing thrombin formation upon contact with blood, ensuring the functionality and duration of use of medical devices, and the safety of patients. These polymers find applications in critical medical devices such as heart valves, vascular grafts, hemodialysis membranes, extracorporeal blood circulation systems, and stents, that necessitate unimpeded blood flow over their surfaces.

Polyethylene glycols (PEGs) play an important role in various biomedical applications due to their non-toxic nature and versatility. Widely employed in bioconjugation and surface functionalization, PEGs find applications in biomedical research, drug delivery, tissue engineering, and the food and cosmetics industries. The process of coupling PEG polymer chains to molecules and surfaces is known as PEGylation. PEGylation enhances biocompatibility, solubility, stability, and pharmacokinetic properties of therapeutics. In biomedicine, PEGs serve as biologically inert and non-immunogenic chemicals, labeling tags, and crosslinkers, improving the water solubility of proteins. The distinct properties of PEG, such as non-toxicity, hydrophilicity, and flexibility, make it an invaluable component in various biological, chemical, and pharmaceutical settings. The availability of defined-length PEG derivatives with specific functional groups further enhances their precise and versatile application in medical materials.

Several limitations exist regarding the blood compatibility of blood treatment devices. Individual variations in blood components, such as genetics and blood type, can lead to compatibility issues, resulting in immunogenic reactions and alloimmunity. Moreover, manipulating blood components for therapeutic purposes can cause activation of the complement system, hemolysis, and coagulation problems. Strategies to improve blood compatibility include personalized medicine, biocompatible materials, minimally invasive modifications, and rigorous testing and monitoring.

Developments towards improving hemocompatibility of solid phase materials have been described in the art. Kovach et al (6) describe that uncoated PDMS (Polydimethylsiloxane) microchannel networks rapidly adsorb high levels of fibrinogen in blood contacting applications, thereby initiating platelet activation, resulting in an increase in pressure across microchannel networks, rendering them useless for long term applications. Kovach employs an oxygen plasma pretreatment and siloxane-bound polyethylene glycol grafting approach to reduce fibrinogen and platelet adhesion to PDMS microchannel networks.

Bacal et al (4) provide a review of recent progress across apheresis technologies relating to advances in surface science and materials engineering to address challenges in selective removal or extraction of blood components in apheresis technologies. In summarizing adsorbent apheresis technology, the authors conclude that although adsorption is efficient and promising, the process requires improvement and novel surface chemistries must be developed to enable more selective capture mechanisms with sufficient hemocompatibility, which remains a significant challenge.

Jianhua Lv et al (7) described using PEG, either in linear or looped form, grafted onto a Styrene ethylene butylene styrene (SEBS) carrier via polydopamine (PDA) as a spacer, to generate a hemocompatible surface. Looped and linear PEGs with the same grafting density were assessed with respect to unwanted non-specific protein adsorption and the authors conclude that the looped PEG conformation is superior in showing lower amounts of adsorbed proteins, thereby improving hemocompatibility of SEBS.

US11752250 teaches methods of treating blood to provide increased shelf life and maintaining freshness of blood by removing undesirable molecules from the blood through contacting the blood with a sorbent comprising a cross-linked polymeric material having a plurality of zwitterionic moieties and oligo(ethylene glycol) moieties attached to the surface. PEGylation is indicated as sub-optimal for selective bioactivity compared low fouling, biocompatible, zwitterionic polymers based on methacrylate derivatives of betaine monomers, such as sulfobetaine and carboxybetaine methacrylate.

Thus, despite some advances in improving the hemocompatibility of solid substrates and matrices, difficulties still remain in providing such safe and effective whole-blood compatible materials for extracorporeal blood treatment.

### SUMMARY OF THE INVENTION

In light of the prior art, the technical problem underlying the invention was providing alternative or improved means for a whole blood compatible matrix and device for coupling a ligand and use in extracorporeal blood treatment with excellent hemocompatibility. The present invention seeks to provide such means while avoiding the disadvantages known in the prior art.

A further object of the invention was to provide alternative or improved means for extracorporeal blood treatment of medical conditions such as inflammatory conditions, autoimmune diseases, cancer, conditions associated with organ failure and organ transplantation, and/or degenerative diseases.

A further object of the invention was to provide alternative or improved means for selectively absorbing and/or modifying target molecules in whole blood without the need to separate a patient's blood components before or during therapy

A further object of the invention was to provide alternative or improved means for a whole blood compatible matrix for use in extracorporeal blood treatment with excellent ligand coupling efficiency.

A further object of the invention was to provide alternative or improved means for a whole blood compatible apheresis matrix for use in extracorporeal blood treatment with a high target molecule binding capacity.

A further object of the invention was to provide alternative or improved means for a whole blood compatible matrix for use in extracorporeal blood treatment with low levels of unwanted unspecific protein adsorption, and/or low levels of unwanted adsorption of other whole blood components.

A further object of the invention was to provide alternative or improved means for a whole blood compatible apheresis matrix for extracorporeal blood treatment suitable for sterilization without loss of ligand or ligand activity and/or target molecule binding properties.

A further object of the invention was to provide alternative or improved means for a whole blood compatible matrix for extracorporeal blood treatment enabling simple ligand coupling techniques.

These problems are solved by the features of the independent claims. Preferred embodiments of the present invention are provided by the dependent claims.

The present invention, in one aspect, relates to a whole-blood compatible matrix for coupling a ligand and use in extracorporeal blood treatment, said matrix comprising a solid substrate, said substrate comprising a first layer of polyethylene glycol (PEG) and at least a second layer of polyethylene glycol, wherein said at least second layer is positioned outside the first layer and is bound to said first layer, and wherein the polyethylene glycol of the at least second layer comprises a terminal functional group for coupling said ligand to said polyethylene glycol.

As described in more detail below, in embodiments, the first (inner) layer of polyethylene glycol (PEG) is bound to at least a second (outer) layer of polyethylene glycol, and such matrices may also be termed as "double-PEG" matrices or a "double-PEGylated matrix".

In embodiments, a third, fourth, of fifth layer can be added to the second, third, or fourth layer, respectively. The principles and methods for adding such third, fourth, or consecutive layer correspond to the addition of the at least second layer to the first layer as disclosed herein. Accordingly, when using the expression "the at least second layer", further layers that can be added according to the same methods and principles, such as a third, fourth, or further layer, are envisioned. The expressions "double PEGylated" and "double PEGylation" as used herein therefore encompass beads and matrices having a third, fourth, or further layer and methods for adding same.

In embodiments, beads and matrices having a first layer of polyethylene glycol and a second layer of polyethylene glycol (i.e., there is no third or further polyethylene glycol layer present) are preferred even though additional layers beyond a second polyethylene glycol layer are feasible, since the intended effect can generally already be achieved with a second polyethylene glycol layer. However, uses can be envisioned where the addition of a third or fourth or further polyethylene glycol layer may be beneficial.

The whole blood compatible matrix of the present invention allows effective ligand coupling and thus the selective targeting of specific components that should be removed from the blood by immobilization and/or modified e.g., by enzymatic conversion, to treat or ameliorate various health conditions. Preferably, for successfully using such a matrix with a ligand bound thereon (the "apheresis matrix"), the molecular targets are present in the bloodstream and must be able to be immobilized by the ligand of the apheresis matrix, which can comprise proteins, such as antigens or antibodies, or nucleic acids, such as DNA or RNA aptamers, that are coupled to the matrix. When immobilized to the ligands of the apheresis matrix, target molecules are sequestered from the bloodstream, mitigating their potential to induce or exacerbate specific diseases. Thus, the target molecules are immobilized in a manner that suppresses or removes their activity from circulating blood, including enzymatic functions, or binding interactions with other molecules.

Furthermore, the invention advantageously enables an improved hemocompatibility of the matrix and offers diverse surface chemistry, facilitating the covalent attachment of ligands, such as antibodies, enzymes, or other biological molecules. Unlike beads comprising a "single PEGylation", the "double PEGylated" matrix which is characterized by at least a second layer of polyethylene glycol provides a higher density of chemical surface functionalities, potentially allowing for a greater ligand loading capacity.

As described in more detail in the examples, an advantageous and unexpected increased blood compatibility can be obtained through double PEGylation of the matrix.

For example, double PEGylation may enable a higher PEG density on the bead surface compared to single PEGylation. In embodiments, the first PEG layer in double PEGylation may be denser than the PEG layer achieved by single PEGylation. In embodiments, this may be obtained by employing a shorter length of the first PEG in the double PEGylation. Furthermore, a higher density of surface functionalities can be obtained by double PEGylation compared to single PEGylation. Furthermore, in embodiments, the use of shorter PEG chains in the first PEGylation results in better accessibility of pores and thus also a better coating of porous beads in the pore volume when using two-step PEGylation.

By way of example, the experimental evidence presented herein shows that, for example, two-step PEGylation ("double PEGylation") leads to equally efficient or improved blood compatibility compared to single PEGylation (PEGylation in one step) with the same or lower molecular weight PEG entities.

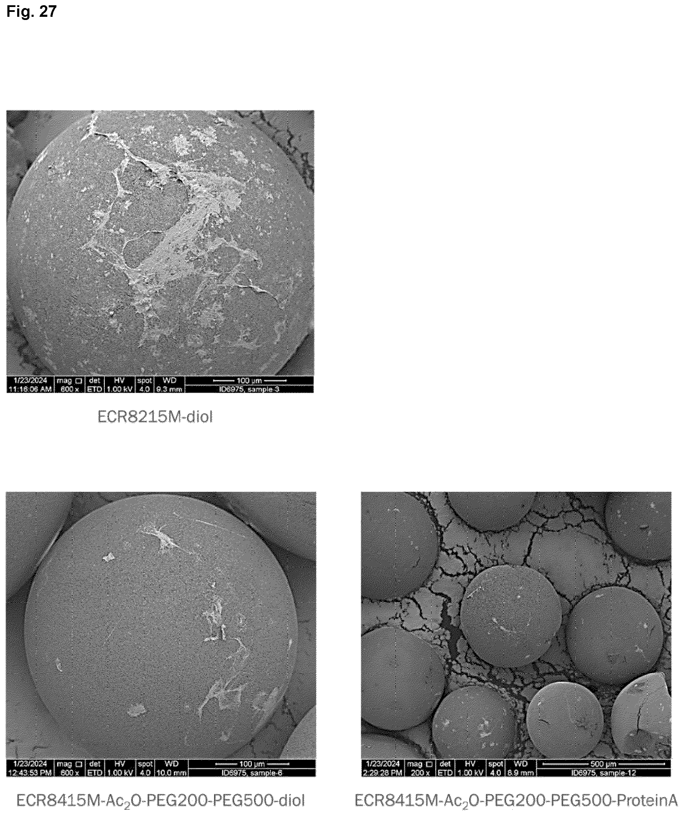

Furthermore, double PEGylation may enable the prevention of non-specific protein accumulation. As can be seen from the scanning electron microscope (SEM) images shown in Figure 27, "naked" beads without PEGylation lead to heavily covered beads, coated with adhering cells and protein deposits, whereas beads with a double PEGylation and beads coated with ligand-couple double PEGylation (for example double PEGylated beads coupled to protein A as an example ligand) show a much less pronounced deposition of cellular components and proteins. Thus, the double PEGylation represents a surprisingly advantageous approach towards treating beads that are employed in an apheresis matrix after ligand coupling.

Furthermore, the ligand coupling to the double PEGylated beads shows no detrimental effect on blood compatibility tests. By way of example, Protein A as a ligand does not cause any loss in blood compatibility compared to PEGylated beads without coupled ligand.

Furthermore, the immobilized ligands maintain their function post-immobilization, making the construct suitable for selectively adsorbing target molecules from whole blood in apheresis treatment. The composite, comprised of porous polymer beads and multiple layers of PEG chains with various chemical functionalities, forms a versatile platform for attaching ligands of different chemical and/or biological nature.

In one embodiment, the present invention relates to a whole blood compatible matrix for coupling a ligand and use in extracorporeal blood treatment, said matrix comprising a solid substrate, said substrate comprising a first layer of linear polyethylene glycol (PEG) and at least a second layer of linear polyethylene glycol, wherein said at least second layer is positioned outside the first layer and is bound to said first layer, and wherein the linear polyethylene glycol of the at least second layer comprises a terminal functional group for coupling said ligand to said linear polyethylene glycol.

In embodiments, the target molecules (in whole blood) can undergo enzymatic modification or conversion, if the ligand attached to the matrix is an enzyme. In embodiments, enzymes may be immobilized on the matrix as described herein. In embodiments, these enzymes may encompass a range of enzymes, including, without limitation, oxidoreductases, transferases, hydrolases, lyases, isomerases and/or ligases. Such enzymatic reactions can specifically target molecules implicated in, contributing to, or causing a disease. For instance, alkaline phosphatase immobilized on the matrix may catalyze the conversion of extracellular ATP, ADP, AMP, or lipopolysaccharides.

In embodiments, it is imperative that the matrix effectively carries the ligand, ensuring coupling, sterilization, and storage without compromising functionality. Accordingly, in embodiments, the matrix is configured to allow effective covalent binding of such ligands. According to the aspect described above, the whole-blood compatible matrix is designed for coupling a ligand, wherein the linear polyethylene glycol comprises a terminal functional group for coupling the ligand to said linear polyethylene glycol. Said terminal functional group is preferably selected to fulfill one or more of the desired properties enabling good ligand coupling efficiency, ligand stability, sterilizability, and other properties, as described in more detail herein.

In embodiments, the matrix is designed to optimize coupling efficiency, thereby optimizing the ligand density on the matrix. Accordingly, in embodiments, the matrix exhibits a high coupling efficiency, allowing for stable covalent binding of the ligand while preserving its structural and functional integrity.

In another embodiment, the binding capacity of the resulting apheresis matrix is optimized to facilitate the capture and/or processing of a therapeutically effective quantity of a target molecule within a reasonable time. Furthermore, in embodiments, the activity of the ligand remains intact following coupling to the matrix. However, a high ligand density can sometimes lead to the loss of ligand activity, often attributed to steric hindrance. Thus, one embodiment involves designing the matrix to minimize steric hindrance of the ligand.

The matrices of the present invention are highly hemocompatible and suitable for coupling a ligand and use of the resulting apheresis matrix in whole blood extracorporeal blood treatment systems. Various matrices known in the art exhibit poor blood compatibility, often triggering the activation of blood cascade systems. This can result in complications such as blood coagulation, platelet depletion, and other compatibility issues, as discussed by Ekdahl et al. (3). Blood compatibility remains a significant concern in extracorporeal blood treatment, particularly in applications such as apheresis. Ideally, matrices capable of direct perfusion with a patients' blood would enable and simplify apheresis procedures. However, achieving this necessitates excellent blood compatibility. As noted by Bacal et al. (4), the hemocompatibility and toxicity of materials used to build chemical adsorbents remains a significant challenge, often leading to avoiding extensive direct blood contact and necessitating expensive and complex cartridge and therapy designs. Hence, in embodiments, the matrix of the present invention is hemocompatible and/or biocompatible and enables apheresis devices and therapies using whole blood.

To the best of the inventors' knowledge, the matrix of the present invention is optimized regarding efficiency and hemocompatibility/biocompatibility and represents an improvement in the combination of the aforementioned advantages and features over other matrices known in the prior art. Without being bound by theory, achieving such optimization was not merely the aggregated result of a simple combination of existing approaches or techniques. Given the multitude of constraints, challenges, and possibilities, arriving at the matrix of the present invention represents an unexpected and beneficial result, defined by a combination of features and resulting advantages not previously disclosed or suggested in the art.

One main advantage of the invention is that it facilitates fine-tuning of the hemocompatibility of porous polymers and enables diverse surface chemistry, facilitating the covalent attachment of ligands, such as proteins, nucleic acids, carbohydrates, or other biological molecules. In one embodiment, the composite structure comprises porous polymer beads coated with carefully designed covalently bound PEG layers, offering a wide range of chemical functionalities for ligand attachment. This flexible platform accommodates ligands of varying chemical nature, ensuring that immobilized molecules retain their functionality. Consequently, the composite material is well-suited for applications requiring selective adsorption of target molecules from whole blood in whole-blood apheresis treatments.

Thus, the covalent coating of porous polymer beads using polyethylene glycols (PEGs) of carefully selected varying lengths and with an optimized coupling chemistry enables the enhancement and adjustment of hemocompatibility while maintaining a high density of chemical surface functionalities. This approach facilitates the subsequent covalent attachment of ligands suitable for selectively adsorbing target molecules from human whole blood.

In embodiments, the hemocompatibility of the matrix is enhanced by tailored double PEGylation. In embodiments, the hemocompatibility of the matrix is enhanced when coated with double-PEG according to the invention in contrast to the single-PEG matrix or uncoated base solid phase. In some embodiments, the improved hemocompatibility is evident from a reduced decline in platelet count (PLT). In some embodiments, the improved hemocompatibility is evident from a reduced increase of platelet factor 4 (PF4).

In embodiments, the non-specific absorbed protein layer is decreased on the PEGylated solid substrate compared to the non-PEGylated solid substrate, and is decreased on the double PEGylated solid substrate compared to a single PEGylated solid substrate.

In embodiments, non-specific protein adsorption is decreased on the PEGylated solid substrate compared to the non-PEGylated solid substrate, and non-specific protein adsorption is decreased on the double PEGylated solid substrate compared to the single PEGylated solid substrate.

In embodiments, the adhesion of activated platelets is decreased on the PEGylated solid substrate compared to the non-PEGylated solid substrate, and the adhesion of activated platelets is decreased on the double PEGylated solid substrate compared to the single PEGylated solid substrate.

The protein layer initially adsorbed onto the surface of biomaterials primarily instigates adverse reactions, including activating coagulation via the intrinsic pathway, stimulating leukocytes leading to inflammation, and adhering and activating platelets (Liu et al., J Mater Chem B, 2014). Consequently, this cascade of events may lead to a decrease in blood cell count and thrombosis formation.

Thus, the main advantage of the present invention is that the PEGylated solid substrate as disclosed herein exhibits decreased non-specific adsorption of proteins compared to non-PEGylated solid substrates, and the double PEGylated solid substrate as disclosed herein exhibits decreased non-specific adsorption of proteins compared to single PEGylated solid substrates.

This reduction in protein adsorption helps minimize the initiation of adverse reactions associated with protein-layer-triggered responses, such as coagulation activation and inflammation. Moreover, in comparison to non-PEGylated or single PEGylated solid substrates, the PEGylated solid substrate of the invention shows less activation of adhered platelets. This reduction in platelet adhesion and activation is beneficial as it reduces the risk of thrombus formation, thus promoting improved blood flow and preventing potential thrombotic complications.

In embodiments, the combination of particle diameter and average pore size of the particles, when coated with, for example, linear PEG of a given length (repeating units) as disclosed herein, enables various beneficial effects, such as high ligand coupling efficiency, high stability of the matrix (and ligand) during and after sterilization and medical use, and low levels of unspecific binding of blood components, thus providing a highly hemocompatible matrix suitable for effective ligand coupling and use in whole blood extracorporeal therapies.

In embodiments, careful optimization of particle diameter, average pore size of the particles, and PEG length, specifically in case of linear PEG, in combination, reveals unexpected improvements in the beneficial properties of the matrix, as described herein.

In embodiments, the solid substrate comprises particles, wherein said particles have a diameter of about 100 µm to about 1000 µm and an average pore size of about 20 nm to about 300 nm.

In embodiments, the particle of the present invention has a diameter of about 100, 200, 300, 400, 500, 600, 700, 800, 900, or 1000 µm. The diameter of the particle may also fall within a size range formed between any two endpoints mentioned in the list.

In embodiments, the specified diameter of the particle plays an important role in optimizing separation efficiency, flow dynamics, and targeted removal of blood molecules within the system. By defining the size or diameter of the particle, efficient interaction with target components is facilitated, enhancing separation resolution and potentially increasing capture efficiency while minimizing non-specific binding. Furthermore, the defined particle size enables smooth blood flow with reduced pressure drop, thereby enhancing patient comfort and potentially mitigating hemolysis and coagulation due to shear stress.

The specification of the particle size is dependent on the intended application. By way of example, larger particles may be preferred for applications requiring or allowing a higher blood flow rate while smaller beads might be more suitable for applications requiring lower flow rates in order to optimize the binding capacity and kinetics of target molecule removal.

In embodiments, the particle of the present invention has an average pore size of about 20, 30, 40, 50, 60, 70, 80, 90, 100, 110, 120, 130, 140, 150, 160, 170, 180, 190, 200, 210, 220, 230, 240, 250, 260, 270, 280, 290 or 300 nm. The average pore size of the particle may fall within a size range formed between any two endpoints mentioned in the list.

In embodiments, particles with smaller pore sizes, such as 20, 30, 40, 50, 60, 70, 80, 90, 100, 110, 120, 130, 140, 150, 160, 170, 180, 190 or 200 nm, are employed for shorter PEGs. In embodiments, particles with larger pore sizes, such as 150, 160, 170, 180, 190, 200, 210, 220, 230, 240, 250, 260, 270, 280, 290 or 300 nm, are employed for longer PEGs.

In embodiments, the present invention demonstrates a surprising effect, wherein significantly enhanced blood compatibility can be achieved when utilizing small pores and shorter PEGs. PEGylating the material can enhance its blood compatibility by making the surface more hydrophilic and reducing the number of sites where proteins can bind. This happens because PEGylation effectively blocks particle pores, reducing the surface area available for protein interaction. As the pores undergo PEGylation, a layer of PEG molecules forms on their surface, preventing proteins from binding and thus decreasing the risk of blood clot formation. Nevertheless, the surface of particles with larger pores can also be effectively shielded, preferably without blocking the pores, but by forming a dense PEG layer on the inner and outer surface, thus reducing non-specific protein interactions and increasing hemocompatibility.

In embodiments, the linear polyethylene glycol of the first layer has a molecular weight of about 100 to 7000 Daltons, preferably 100 to 5000 Daltons, more preferably 100 to 1000 Daltons, and especially preferably from 100 to 500 Daltons, such as, for example, 200, 300, 400, or 500 Daltons.

In embodiments, the polyethylene glycol of the at least second layer has a molecular weight of about 100 to 7000 Daltons, preferably 200 to 3000 Daltons, more preferably 200 to 1000 Daltons, and especially preferably from 300 to 800 Daltons, such as, for example, 500, 600. 700, or 800 Daltons.

In embodiments, the polyethylene glycol of the first and/or at least second layers, each considered individually, has a molecular weight of about 50, 100, 200, 300, 400, 500, 600, 700, 800, 900, 1000, 1100, 1200, 1300, 1400, 1500, 1600, 1700, 1800, 1900, 2000, 2100, 2200, 2300, 2400, 2500, 2600, 2700, 2800, 2900, 3000, 3100, 3200, 3300, 3400, 3500, 3600, 3700, 3800, 3900, 4000, 4100, 4200, 4300, 4400, 4500, 4600, 4700, 4800, 4900, 5000, 5100, 5200, 5300, 5400, 5500, 5600, 5700, 5800, 5900, 6000, 6100, 6200, 6300, 6400, 6500, 6600, 6700, 6800, 6900, 7000 Daltons. The molecular weight of the linear PEG may also fall within a size range formed between any two endpoints mentioned in the list.

The polyethylene glycol can be linear, branched, Y-shaped, or have a multi arm geometry, all of which are commercially available. Said different forms of polyethylene glycol are described, for example, in Santos et al., Brazilian Journal of Pharmaceutical Sciences (2018), 54(spe), e01009. For example, 2-arm or 3-arm branched polyethylene glycol (PEG) can be obtained from NOF America Corp. 3-arm PEGs are PEGs wherein another PEG spacer is introduced to 2-arm branched PEGs (see, for example, US8828373B2), whereas 4-arm branched PRGs (also available from, for example, NOF America Corp.) provide for an even larger number of branching points. Said branched PEGs tend to have a different viscosity compared to linear PEG of the same molecular weight (see, for example, US8071712B2). Forked PEG structures have the advantage of placing two reactive groups at precise distance apart.

According to one embodiment of the invention, linear polyethylene glycol is used for preparing the whole-blood compatible matrix. According to another embodiment of the invention, branched polyethylene glycol is used for preparing the whole-blood compatible matrix, wherein preferably PEGs are used that have three to five PEG chains emanating from the central or core group. According to yet another embodiment of the invention, Y-shaped polyethylene glycol is used for preparing the whole-blood compatible matrix.

In embodiments, the polyethylene glycol of the first and of the at least second layer is linear polyethylene glycol. In embodiments, the polyethylene glycol of the first and/or of the at least second layer is Y-shaped polyethylene glycol.

It was entirely surprising that length and molecular weight of the PEG moieties and their specific combination in the first and at least second PEG layers can be optimized to enhance and tailor both blood compatibility and achievable ligand density and activity, as disclosed herein. Identifying an optimal PEG length for either or both of the first and/or second PEG and/or any additional layer involves balancing protein resistance with the ability to effectively couple ligands that following said coupling retain their ability to bind desired molecules. Shorter and/or less branched chains may offer better access to blood components and allow for a high ligand loading, but less protein resistance, while longer and/or branched or Y-shaped chains may provide stronger protein resistance but potentially hinder a high ligand loading and the effective immobilization or modification of target molecules. In this regard, Y-shaped PEG used in either the first and/or second layer is a compromise that makes it a useful PEG geometry according to the present invention. Therefore, identifying an optimal PEG length and/or molecular weight resulted in the unexpected outcome of enhanced hemocompatibility, ligand loading, and target molecule binding capacity. In embodiments, combining PEG length with particle and pore size leads to unexpected enhancements in one or more of the properties described herein.

In embodiments, the solid substrate is hydrophilic or hydrophobic, and comprises a synthetic polymer, a natural polymer, and/or inorganic material.

In embodiments, the solid substrate is hydrophilic. In embodiments, the solid substrate is hydrophobic.

The choice between utilizing a hydrophilic or hydrophobic solid substrate may depend on the nature of the target molecule and the concomitant restrains for the solid support with respect to pore size and particle size for optimizing the matrix as described above. Therefore, having the option for either a hydrophilic or hydrophobic solid substrate offers increased flexibility and may help overcome limitations associated with exclusively hydrophilic or hydrophobic bead-based approaches.

A hydrophilic solid substrate may facilitate interactions with water-soluble molecules, such as those in blood plasma. This property enables efficient binding and removal of target molecules readily dissolving in the blood. Additionally, due to their affinity for water, hydrophilic substrates typically exhibit lower non-specific interactions with other blood components. Conversely, coupling discrete PEG moieties to a hydrophobic solid is more challenging and non-specific interactions with other blood components are increased. Both hydrophilic and hydrophobic solid substrates can be modified with specific functional groups to increase their affinity for discrete PEG moieties according to the invention and as further described herein.

In embodiments, the solid substrate comprises a synthetic polymer, a natural polymer, and/or inorganic material.

One advantage of a solid substrate comprising a synthetic polymer is that it can be chemically modified to achieve specific functionalities, like desired pore size and/or surface chemistry In other embodiments, natural polymers may be advantageous regarding their production and disposal after use. In other embodiments, inorganic materials exhibit high stability and durability with increased mechanical strength and resistance to degradation which may allow prolonged use during therapy. Each type of solid substrate offers advantages that can be leveraged depending on specific requirements and can be readily combined with the disclosure made herein for generating matrices for efficient coupling of ligands and use in extracorporeal blood treatment applications.

In embodiments, the solid substrate is a porous synthetic polymer particle, preferably comprising a methacrylate polymer.

In one embodiment, the solid substrate takes the form of particles or beads. In one embodiment, the solid substrate is selected from Purolite^{®} Lifetech^{™} ECR beads. In embodiments, the solid substrate is a methacrylate bead, preferably an epoxy methacrylate or an amino C6 methacrylate bead. In embodiments, the solid substrate is selected from Purolite^{®} Lifetech^{™} ECR8215M (epoxy methacrylate), ECR8215F (epoxy methacrylate), ECR8415M (amino C6 methacrylate), ECR8415F (epoxy methacrylate), ECR8204M (epoxy methacrylate), ECR8204F (epoxy methacrylate), ECR8404M (amino C6 methacrylate), or ECR8404F (amino C6 methacrylate).

According to one embodiment, Purolite^{®} Lifetech^{™} ECR8215M or ECR8215F epoxy methacrylate beads are used which carry a functional group to which a ligand can be coupled. According to another embodiment, Purolite^{®} Lifetech^{™} ECR8415M or ECR8415F amino C6 methacrylate beads are used, which carry a functional group to which a ligand can be coupled. According to another embodiment, Purolite^{®} Lifetech^{™} ECR8404M amino C6 methacrylate beads are used which carry an epoxy group as a functional group to which a ligand can be coupled.

In embodiments, the average molecular weight of said bound linear polyethylene glycols of the first and at least second layer, combined weight of the first layer PEG coupled to the at least second layer PEG, is in the range of about 100 to 2500 Daltons, 300 to 2000 Daltons, preferably 400 to 1000 Daltons, more preferably the average molecular weight is about 500 to 1000 Daltons. In embodiments, the average molecular weight of said bound linear polyethylene glycols of the first and at least second layer, combined, is about 150, 200, 250, 300, 350, 450, 500, 550, 600, 650, 700, 750, 800, 850, 900, 950, 1000, or 1500 Daltons. The molecular weight of said bound linear polyethylene glycols may also fall within a size range formed between any two endpoints mentioned in the lists herein. In one preferred embodiment, the average molecular weight of said bound linear polyethylene glycol of the first and at least second layer, combined, is 500-1000 Daltons, such as 700 Daltons. In a further preferred embodiment, the average molecular weight of said bound linear polyethylene glycol of the first layer is 200 Daltons and the average molecular weight of linear polyethylene glycol of the at least second layer is 500 Daltons.

In embodiments, the particles have a diameter of about 200 µm to 800 µm and the average pore size of the solid substrate is about 40 to 500 nm, preferably 50 to 300 nm. In embodiments, the particles have a diameter of about 200, 300, 400, 500, 600, 700, or 800 µm and the average pore size of the solid substrate is about 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, or 500 nm. The particle diameter and the average pore size of the solid substrate may also fall within a size range formed between any two endpoints mentioned in the list.

The disclosed particle diameter and pore size of the solid substrate facilitates effective PEGylation, enabling subsequent application in apheresis procedures. In embodiments, the hemocompatibility can be enhanced within this diameter and pore size range.

In embodiments, the solid substrate comprises a synthetic methacrylate polymer particle covalently coupled to linear polyethylene glycols of a first and at least second layer with an average combined molecular weight of 400 to 1000 Daltons, such as about 500, 600, 700, 800, or 900 Daltons. The average combined molecular weight of the linear polyethylene glycols coupled to said synthetic methacrylate polymer particle may also fall within a size formed between any two endpoints mentioned in the list.

In embodiments, the solid substrate comprises a synthetic methacrylate polymer particle covalently coupled to linear polyethylene glycols of a first and at least second layer with an average combined molecular weight of 400 to 6000 Daltons, preferably 500 to 5000 Daltons. The average combined molecular weight of the linear polyethylene glycols coupled to said synthetic methacrylate polymer particle may also fall within a size formed between any two endpoints mentioned before.

In embodiments, the solid substrate has bound thereon in essence a single polyethylene glycol molecular weight form as the first and/or at least second layer. The term "single PEG form" as used herein is thus not limited specifically to using PEG molecules with only the precise length, geometry, or MW indicated, rather the term single PEG form is used to convey coupling of a PEG with an average length or MW at the indicated length or MW, respectively. The "single PEG form" may be elected for the first and/or second PEG layer, the PEG entities may be the same or different for the first and second PEG layers. Accordingly, a "single PEG form" used for creating the first or at least second PEG layer according to the invention may relate to a PEG having the essentially same molecular weight and/or length in one or both layers. In embodiments "single PEG form" may relate to a PEG having the essentially same geometry, such as, for example, linear geometry or Y-shaped geometry, which is used in either the first or the at least second layer or in both, wherein the Y-shaped PEG may also have the same average molecular weight and length. In embodiments, linear PEG is used as the "single PEG form" in both the first and the at least second layer. In embodiments, the first layer essentially is prepared from Y-shaped PEG having the same average molecular weight, whereas the at least second layer is prepared from linear PEG having the same average molecular weight. In embodiments, the first layer essentially is prepared from linear PEG having the same average molecular weight, whereas the at least second layer is prepared from linear PEG having the same average molecular weight.

In embodiments, the solid substrate has a first and at least a second layer of linear PEG, wherein said at least second layer is positioned outside (on top of) the first layer and is bound to said first layer. The linear PEG of the at least second layer comprises a terminal functional group for coupling a ligand to said linear PEG.

In preferred embodiments, the "PEG of the at least second layer" is bound to the first layer of PEG, wherein the first layer and the second layer essentially consist of linear PEG. In some embodiments, the PEG of the at least second layer may be attached directly to the solid substrate (beads). In embodiments, any attachment of the second PEG layer to the substrate is considered inadvertent, and preferred embodiments relate to the second PEG layer being primarily, in essence substantially, or almost completely, or completely, bound to the first PEG layer.

In preferred embodiments, the ligand is coupled to the second PEG layer. In embodiments, the ligand may be coupled to the PEG of the first layer. However, in embodiments, attachment of the ligand to the first PEG layer is considered inadvertent, and preferred embodiments relate to the ligand being primarily coupled to the second PEG layer, in essence substantially or almost completely, or completely, coupled to the second PEG layer.

Commercially available PEG products are often indicated to have an average length or MW, such as "PEG500" (i.e., having an average molecular weight of 500 Dalton), whereas the PEG molecules in the preparation will show some variation in MW, with the majority of PEG molecules having the indicated length or MW. A skilled person is aware of common variation in PEG length or MW in commercial PEG products and is capable of determining the length or MW of PEG coupled to a particle using established analytical techniques such as mass spectrometry or other suitable approaches.

In embodiments, the solid substrate comprises a synthetic methacrylate polymer particle covalently coupled to a linear polyethylene glycol of the first layer with an average molecular weight of about 200 to 2500 Daltons and the linear polyethylene glycol of the at least second layer is coupled to the linear polyethylene glycol of the first layer and has an average molecular weight of about 200 to 2500 Daltons.

In embodiments, the solid substrate comprises a synthetic methacrylate polymer particle covalently coupled to a linear polyethylene glycol of the first layer with an average molecular weight of about 100 to 1000 Daltons and the linear polyethylene glycol of the at least second layer is coupled to the linear polyethylene glycol of the first layer and has an average molecular weight of about 300 to 2000 Daltons.

In embodiments, the solid substrate comprises a synthetic methacrylate polymer particle covalently coupled to a liner PEG of the first layer with an average molecular weight of about 100, 200, 300, 400, 500, 600, 700, 800, 900, 1000, 1100, 1200, 1300, 1400, 1500, 1600, 1700, 1800, 1900, or 2000 Daltons and the linear PEG of the at least second layer is coupled to the linear PEG of the first layer and has an average molecular weight of about 200, 300, 400, 500, 600, 700, 800, 900, 1000, 1100, 1200, 1300, 1400, 1500, 1600, 1700, 1800, 1900, 2000, 2100, 2200, 2300, 2400, or 2500 Daltons. The average molecular weight of the linear polyethylene glycol of the first layer and the average molecular weight of the linear polyethylene glycol of the at least second layer may also fall within a size formed between any two endpoints mentioned in the list.

In embodiments, the solid substrate comprises a synthetic methacrylate polymer particle covalently coupled to a liner PEG of the first layer with an average molecular weight of about 100, 200, 300, 400, 500, 600, 700, 800, 900, 1000, 1100, 1200, 1300, 1400, 1500, 1600, 1700, 1800, 1900, 2000, 2100, 2200, 2300, 2400, 2500 Daltons and the linear PEG of the at least second layer is coupled to the linear PEG of the first layer and has an average molecular weight of about 200, 300, 400, 500, 600, 700, 800, 900, 1000, 1100, 1200, 1300, 1400, 1500, 1600, 1700, 1800, 1900, 2000, 2100, 2200, 2300, 2400, 2500, 2600, 2700, 2800, 2900, 3000, 3500, 4000, 4500, 5000, 5500, 6000 Daltons. The average molecular weight of the linear polyethylene glycol of the first layer and the average molecular weight of the linear polyethylene glycol of the at least second layer may also fall within a size formed between any two endpoints mentioned in the list.

In embodiments, the linear polyethylene glycol of the first layer has a smaller average molecular weight than the at least second layer.

In embodiments, the solid substrate comprises a first layer of in essence a single polyethylene glycol molecular weight form, such as PEG100, PEG200, PEG300, PEG400, PEG500, PEG600, PEG750, PEG2000, and a at least second layer of in essence a single polyethylene glycol molecular weight form, such as PEG100, PEG200, PEG300, PEG400, PEG500, PEG600, PEG750, PEG2000, PEG3000 or PEG5000, wherein the number following the expression "PEG" indicates the average molecular weight of the PEG.

In embodiments, the solid substrate comprises a first layer of in essence a single polyethylene glycol molecular weight form, such as PEG100, PEG200, PEG300, PEG400, PEG500, PEG600, PEG750, PEG1000, PEG1500 or PEG2000, and a at least second layer of in essence a single polyethylene glycol molecular weight form, such as PEG100, PEG200, PEG300, PEG400, PEG500, PEG600, PEG750, PEG 1000, PEG 1500, PEG2000, PEG3000, PEG4000 or PEG5000. The single polyethylene glycol molecular weight forms may also fall within a size formed between any two endpoints mentioned in the list.

In embodiments, the solid substrate comprises a first layer of in essence a single polyethylene glycol molecular weight form, such as polyethylene glycol having an average molecular weight of 100 Daltons, 200 Daltons, 300 Daltons, 400 Daltons, 500 Daltons, 600 Daltons, 700 Daltons, 750 Daltons, 900 Daltons, 1000 Daltons or 2000 Daltons, and at least a second layer of in essence a single polyethylene glycol molecular weight form, such as polyethylene glycol having an average molecular weight of 100 Daltons, 200 Daltons, 300 Daltons, 400 Daltons, 500 Daltons, 600 Daltons, 700 Daltons, 750 Daltons, 900 Daltons, 1000 Daltons, 2000 Daltons, 3000 Daltons, or 5000 Daltons.

In one embodiment, the polyethylene glycol of the first and/or at least second layer is a linear polyethylene glycol.

In embodiments, the linear polyethylene glycol of the first layer has a smaller average molecular weight than the at least second layer. For example, the first layer may comprise or consist of polyethylene glycol having a molecular weight of 100 to 300 Daltons, whereas the at least second layer may comprise or consist of polyethylene glycol having a molecular weight of 400 to 700 Daltons. Taken together, the average molecular weight of the polyethylene glycol of the first and at least second layer may then be, for example, in the range of from 500 to 1000 Daltons.

In embodiments, the method of preparing the matrix involves a two-step covalent coating of porous polymer beads using PEGs of varying lengths. Initially, short-chain PEGs (e.g., PEG200) are utilized for PEGylation, resulting in a high density of chemical functionalities on the bead surface. A second PEGylation step is employed to fine-tune hemocompatibility while maintaining a high density of chemical surface functionalities for subsequent ligand immobilization. Compared to PEGylation with a single, larger PEG molecule of equivalent effective size (molecular weight), this approach advantageously increases the density of available chemical surface functionalities, enabling higher loading with functional molecules after PEGylation, while effectively providing or maintaining hemocompatibility.

In embodiments, the linear polyethylene glycol (PEG) of the first layer is covalently bound to the solid substrate by a chemical reaction having occurred between said PEG of the first layer and a functional group of the solid substrate, wherein said functional group of the solid substrate is selected from an epoxy, acetyl, hydroxyl, amine, thiol, cystamine HCl, carboxy, aldehyde, squaric acid ester, active ester, maleimide, haloacetate, succinimidyl carbonate, octadecyl and/or iminodiacetic group, or a derivative thereof.

In embodiments, the terminal functional group of the linear polyethylene glycol (PEG) of the at least second layer for coupling to the ligand is selected from an amine, cystamine HCl, carboxy, epoxy, thiol, aldehyde, squaric acid ester, active ester, maleimide, haloacetate, nitrophenyl/succinimidyl carbonate, acetyl, hydroxy, haloacetamide, isocyanate, halogen, azide, alkyne, alkene, silane, oxyamine, hydrazide, biotin, sulfonate, acrylate, acrylamide, vinyl sulfone, orthopyridyldisulfide (OPSS)), bromoacetamide and/or iodoacetamide group, or a derivative thereof.

In preferred embodiments, the functional groups used for coupling linear polyethylene glycol (PEG) of the first layer covalently to the solid substrate, and/or for coupling the terminal functional group of the linear polyethylene glycol (PEG) of the at least second layer to the ligand, is selected from amines, cystamine HCl, carboxy, epoxy, thiol, aldehyde, squaric acid ester, active ester, maleimide, haloacetate and succinimidyl carbonate. Experimental support has been generated for these functional groups.

In preferred embodiments, the functional groups used for coupling linear polyethylene glycol (PEG) of the first layer covalently to the solid substrate is selected from amines, cystamine HCl, carboxy, epoxy, thiol, aldehyde, squaric acid ester, active ester, maleimide, haloacetate and succinimidyl carbonate. Experimental support has been generated and summarized below for these functional groups.

In preferred embodiments, the functional groups used for coupling the terminal functional group of the linear polyethylene glycol (PEG) of the at least second layer to the ligand, is selected from amines, cystamine HCl, carboxy, epoxy, thiol, aldehyde, squaric acid ester, active ester, maleimide, haloacetate and succinimidyl carbonate.

Although some chemically difficult groups are more technically challenging, they may be feasibly implemented and are encompassed by the invention, such as alkenes, biotin, hydrazides, oxyamines, sulfonates, halides, silanes, and vinyl sulfones.

In preferred embodiments, the coupling of the first PEG layer to the solid substrate comprises an epoxide functional group of the substrate reacting with an amine group of a PEG (or PEG derivative) to form a covalently bound first PEG layer.

In preferred embodiments, the coupling of the first PEG layer to the solid substrate comprises an amine functional group of the substrate reacting with an epoxide group of a PEG (or PEG derivative) to form a covalently bound first PEG layer.

In preferred embodiments, the first PEG layer comprises a terminal epoxide group for reaction with an amine group of the second PEG layer. In preferred embodiments, the first PEG layer comprises a terminal amine group for reaction with an epoxide group of the second PEG layer.

In preferred embodiments, the second PEG layer comprises a terminal functional group, such as an amine, epoxide or other functional group, for reacting with the ligand.

The versatility of employing a range of functional groups offers substantial advantages in developing and optimizing the present invention. It enables customization to specific materials and applications. The availability of diverse functional groups allows the selection of the most appropriate group based on the particular reactive groups present on the matrix, ensuring efficient and stable coupling. Furthermore, different functional groups can impart varied properties to the PEGylated product, enhancing its versatility and utility.

In embodiments, the solid substrate is capped by chemical modification, preferably by acetylation and/or hydrolysis. Such "capping" means that reactive groups that may remain on the surface of the solid substrate after the coupling of the first PEG layer are chemically deactivated, thereby reducing or eliminating the potential of the matrix to interact or react with the ligand and/or with a component of the treated blood or blood component in any unwanted way. The capping thereby forms an important aspect of rendering the matrix and final apheresis matrix hemocompatible/biocompatible and safe for use with whole blood or a blood component.

In embodiments, the solid substrate is capped through acetylation. In embodiments, the PEGylated solid substrate is capped through acetylation. In embodiments, acetylation is achieved by reacting the primary amines of the solid substrate with acetylating agents under conditions generally known in the art. In embodiments, the acetylating agent is acetic anhydride or acetyl chloride. In embodiments, the solid substrate is capped with acetic anhydride and/or acetyl chloride. In embodiments, the PEGylated solid substrate is capped with acetic anhydride and/or acetyl chloride.

As mentioned above, the acetylation method offers several advantages. For example, primary amines are strong nucleophiles and facilitate the non-specific adsorption of proteins. Capping these amines via acetylation yields a chemically deactivated hydrophilic derivative of the solid substrate starting material having increased bio- and hemocompatibility.

In embodiments, the solid substrate is capped with 1,4-butanediol diglycidyl ether (BDE). In embodiments, the PEGylated solid substrate is capped with 1 ,4-butanediol diglycidyl ether (BDE). In one embodiment, capping using BDE is achieved by reacting the primary amines of the solid substrate in analogy to what is described, for example, in Zeeman et al. 1998, Journal of Biomedical Materials Research 51(4), 541-548, with BDE, wherein the epoxide group(s) of BDE will react with the amine group(s) of the solid substrate in the presence of NaHCO₃, preferably at an alkaline pH, such as >8, resulting in a secondary amine group. The primary amine group is thus "masked" and no longer reactive under conditions relevant to the intended use of the matrix. The remaining epoxide group of BDE is susceptible to being hydrolyzed. This reaction can be used to deactivate said second epoxy group of BDE, resulting in a "capped" solid substrate or matrix.

Capping with BDE offers several advantages, including high reactivity and biocompatibility of BDE. As mentioned above, BDE contains two epoxide groups per molecule, facilitating efficient crosslinking and attachment to diverse substrates. While the first epoxy group of BDE can be used to react with and deactivate, for example, remaining primary amines on the solid substrate, the second epoxy group can either be hydrolyzed and inactivated as suggested above or used for coupling further compounds, thereby allowing for further customization of surface properties of the solid substrate or matrix, such as, for example, hydrophobicity and charge. Hydrolyzation of the second epoxy group of BDE can, for example, be achieved as described below for the superfluous epoxy group of the solid substrate or matrix.

In further embodiments, where the solid substrate carries epoxy functional groups, superfluous epoxy groups of the solid substrate or matrix are capped through hydrolytic modification. In embodiments, the PEGylated solid substrate is capped through hydrolytic modification. Hydrolytic modifications can alter the surface functionalities of the substrate, potentially impacting its hydrophilicity, charge, and reactivity.

In embodiments, said epoxy groups of the solid substrate or matrix are capped or "masked", for example, using a solution containing 0.1 M NaOH and/or 1 M ethanolamine. In embodiments, in synthesis routes starting from the solid substrate bearing epoxy groups, capping can be achieved by reacting said substrate with 0.1 M NaOH or 1 M ethanolamine.

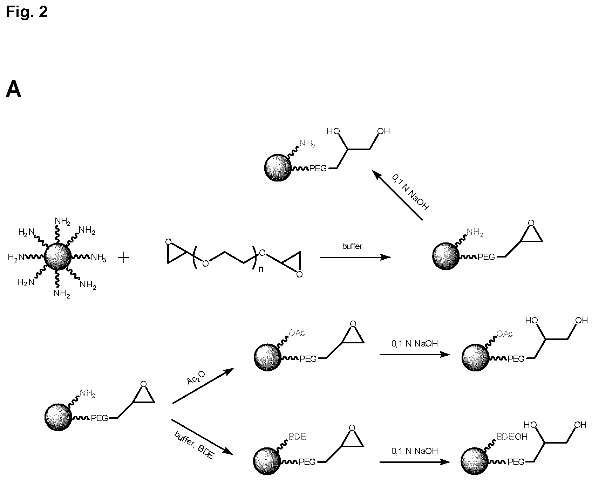

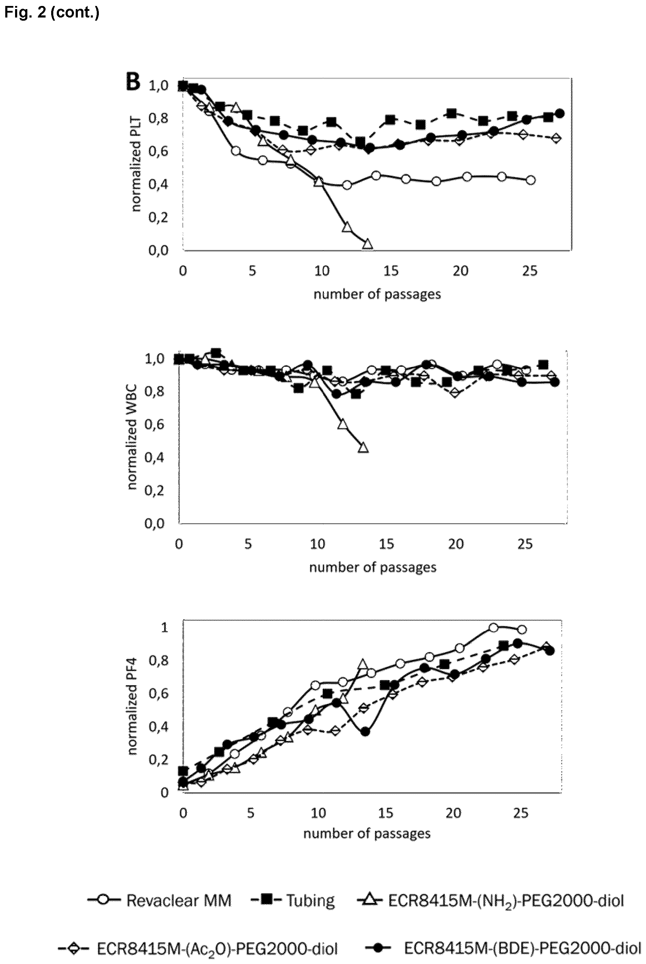

The invention enables diverse capping methods, each offering distinct advantages depending on the intended function and desired characteristics of the matrix. As demonstrated in Figure 2, these distinct capping strategies have been proven to enhance hemocompatibility significantly compared to uncapped substrates. Consequently, in certain embodiments, the capping of the solid substrate improves its hemocompatibility.

In embodiments, the matrix facilitates a ligand loading, obtained by coupling the functional group of the linear polyethylene glycol of the at least second layer to the ligand, of 1 to 200 mg ligand/mL of matrix, preferably 10 to 100 mg ligand/mL matrix.

In embodiments, a ligand can be coupled to the functional group of a linear polyethylene glycol of the at least second layer of the matrix to yield a ligand loading of about 1 to 200 mg ligand/mL of the matrix, preferably 10 to 100 mg ligand/mL of the matrix. In embodiments, the ligand is a peptide or protein, such as, for example, an antibody or an enzyme. In embodiments, the ligand comprises or essentially consists of a carbohydrate, such as, for example, a glycosaminoglycan or ABO(H) antigen. In embodiments, the ligand is a nucleic acid, such as an aptamer, for example, an RNA, a DNA aptamer, and/or mirror-image RNA aptamer.

In embodiments, the ligand loading of said matrix, obtained by coupling the functional group of the linear polyethylene glycol to the ligand is 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, 100, 110, 120, 130, 140, 150, 160, 170, 180, 190, or 200 mg ligand/mL matrix.

In embodiments, the ligand is coupled to the linear polyethylene glycol of the at least second layer, preferably by covalent binding.

In embodiments, the ligand is coupled to the functional group of the linear polyethylene glycol of the at least second layer, preferably by covalent binding. In embodiments, covalent binding of the ligand to the functional group of the linear polyethylene glycol of the at least second layer is achieved through amide bond formation, click chemistry, Schiff base formation, thioester bond formation, hydrazone bond formation, Diels-Alder cycloaddition, Staudinger ligation, reductive amination, and/or Michael addition.

Modification and conjugation techniques are known in the art and are described at length in available literature (for example, G. Hermanson, Chapter 2, Functional Targets for Bioconjugation, Academic Press, 2013). Speaking generally, all conjugation techniques depend on two interrelated chemistries: the reactive functionalities present on the various crosslinking or derivatizing reagents and the functional groups present on the macromolecules to be immobilized.

The solid substrate that forms the basis for generating a matrix according to the invention must provide or facilitate chemical reactivity, thus allowing the chemical coupling of PEG. In equal measure, the matrix, specifically the PEG moieties coupled to the solid substrate, must provide for or facilitate the necessary chemical reactivity for coupling a ligand. Various methods for coupling ligands, such as antibodies or fragments thereof, to solid supports are well-known in the art. In general, the coupling chemistry should result in chemical bonds that are stable over a wide range of pH, buffer conditions, and temperature, resulting in negligible leaching of ligands. The coupling method should avoid improper orientation, multisite attachment, or steric hindrance of the ligand, which may cause masking of the binding sites and, subsequently, lead to loss of activity. Site-directed attachment and/or spacers can be considered for immobilizing the ligand onto the support. The ligand density per volume of the matrix can be optimized to promote target accessibility and binding.

In embodiments, the apheresis matrix comprising the ligand is configured to selectively bind and/or immobilize and/or to enzymatically process a target molecule in whole blood. In embodiments, the ligand is a biomolecule. In embodiments, the biomolecule is configured to selectively bind and/or immobilize and/or to enzymatically process a target molecule in whole blood.

The ligands (preferably biomolecules) of the present invention can effectively and safely interact with target molecules in whole blood, opening up possibilities for various therapeutic and diagnostic applications. The specific requirements may vary depending on the intended use and desired outcome.

In embodiments, the ligand possesses adequate binding sites to effectively immobilize and/ or modify a substantial portion or therapeutically effective portion of the target molecule from the volume of blood being processed. In embodiments, the ligand is configured to bind, immobilize, and/or modify the target molecule more effectively compared to other components in the blood, such as serum proteins or red blood cells, thus minimizing potential off-target interactions and side effects. In embodiments, the ligand is able to distinguish and bind, immobilize, and/or capture specifically a desired target molecule, enabling selective removal of the target molecule. In embodiments, the ligand does not disrupt or interfere with other vital functions of blood components like oxygen transport or clotting factors.

In embodiments, the ligand is non-toxic and non-immunogenic. In further embodiments, the ligand is stable in the blood environment, resisting degradation by enzymes or other blood components over a relevant treatment period, such as from 4 to 72 hours, during which the apheresis matrix comprising the ligand will be exposed to whole blood or a blood component, such as blood plasma. In embodiments, the ligand is adaptable to different target molecules and easily modified for specific applications.

In embodiments, the ligand is a polypeptide, preferably an antigen, antibody or fragment thereof, or an enzyme.

In embodiments, the protein ligand is an affinity reagent, a binder, a binding agent, a binding moiety, or an antigen binding agent. In embodiments, the ligand comprises or essentially consists of a carbohydrate, such as, for example, a glycosaminoglycan or ABO(H) antigen. In embodiments, the ligand is a nucleic acid, such as an aptamer, for example, a DNA aptamer, an RNA aptamer, or a mirror-image RNA aptamer.

One advantage of the present invention is its capability to accommodate various ligands. Different ligands can provide distinct binding profiles, enabling precise targeting of specific molecules or processes. The availability of a diverse library of ligands also facilitates the discrimination between closely related molecules, a crucial aspect of targeted therapies. Consequently, this versatility permits the customization of ligand properties to align with the desired treatment most effectively.

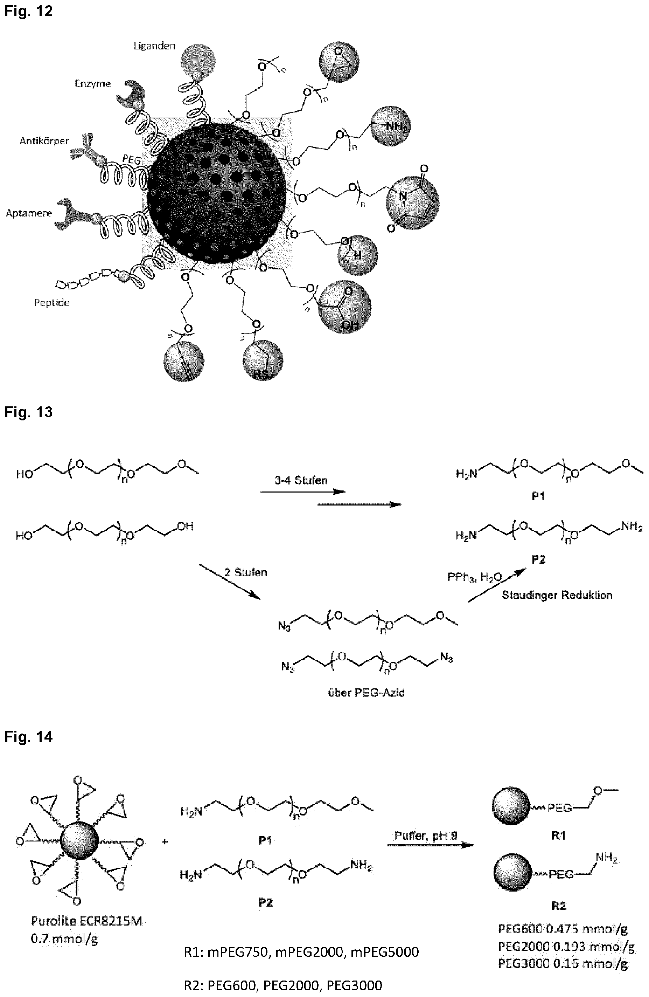

In one embodiment, carboxy-terminated matrices according to the invention, wherein the PEG of the at least second layer carries a carboxyl group (see, for example, Fig. 12) can be coupled through EDC-mediated coupling with amino-functionalized ligands. EDC-mediated coupling is generally known in the art (Hermanson: Bioconjugate Techniques, 3rd Edition, Academic Press; Amsterdam; Chapter 4, p. 259-273). In another embodiment, ligands featuring S-S bridges can be directly coupled to epoxy-functionalized matrices according to the invention (see, for example, Fig. 12) by reducing the S-S bridges to the corresponding thiol using TCEP (tris(2-carboxyethyl)phosphine) during the coupling process. The reduction to thiol groups is necessary to make the ligand available for the crosslinking to the epoxy function of the matrix. The epoxide groups readily react with thiol groups and require a buffered system close to physiological pH. For most applications, 5 to 50 mM TCEP provides sufficient molar excess to effectively reduce peptide or protein disulfide bonds within seconds to a few minutes at room temperature. As TCEP is easily soluble and stable in aqueous solutions and reduces disulfide bonds very effectively, it does not necessarily have to be removed before the crosslinking reaction (see, for example, Thermo Scientific Instructions for TCEP-HCI Number 20490 and 20491, or Urh et al. Affinity Chromatography: General Methods, in Methods in Enzymology, Vol. 463, Burlington, Academic Press (2009), pp. 417-438).

In embodiments, the ligand is a polypeptide selected from an antigen, or antibody or fragment thereof, configured to selectively bind a target protein in whole blood, wherein said matrix has a target protein binding capacity of 0.01 to 20 mg protein/mL of matrix, preferably 0.1 to 10 mg protein/mL matrix.

In embodiments, the ligand is a polypeptide selected from an enzyme, antigen, antibody, or fragment thereof, configured to selectively capture and/or modify a target molecule from whole blood, wherein the resulting apheresis matrix has a target molecule binding capacity, where the target molecule is a protein, of 0.01 to 20 mg protein/mL of apheresis matrix, preferably 0.1 to 10 mg protein/mL apheresis matrix. In embodiments, said apheresis matrix has a target molecule binding capacity, where the target molecule is a protein, of 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20 mg protein/mL.

Advantageously, the apheresis matrix can be designed to possess varying binding capacities depending on the specific target molecule. In certain embodiments, a defined binding capacity of the apheresis matrix ensures precise control over the level of target molecule binding and/or removal from circulation. For instance, in some embodiments, the apheresis matrix may exhibit a high binding capacity for the target protein. Alternatively, in another embodiment, the apheresis matrix may be designed to have a moderate binding capacity for the target protein. This flexibility allows optimizing the apheresis matrix to suit diverse applications and desired outcomes.

In embodiments, the ligand is a nucleic acid molecule, such as an RNA or DNA aptamer, including mirror-image aptamers (all referred to herein as "aptamers").

Aptamers exhibit high selectivity and affinity for their target molecules, owing to their unique three-dimensional structure. This characteristic often results in more efficient removal of the target molecule from the blood compared to other ligands. Additionally, aptamers offer versatility as they can be readily modified and designed to target molecules of interest. Another advantage of aptamers is that they are considered non-immunogenic (Vaganov et al. (2022), Molecules 27(23): 8593).

Another aspect of the invention relates to a method for preparing an apheresis matrix as described herein, comprising coupling, preferably by covalent binding, the linear polyethylene glycol of the at least second layer of the matrix as described herein to the ligand.

In embodiments, the solid substrate, the matrix, and/or the apheresis matrix, are sterilizable or sterilized, preferably by heat and/or radiation.

In general, common sterilization processes do not compromise the hemocompatibility of the solid substrate, the matrix, and/or the apheresis matrix. In embodiments, the solid substrate, the matrix, and/or the apheresis matrix can undergo radiation sterilization methods such as gamma, X-ray, and/or e-beam. In embodiments, the solid substrate, the matrix, and/or the apheresis matrix are sterilizable or sterilized by radiation at 25 kGy. In further embodiments, radiation sterilization does not result in cytotoxic byproducts.

In another embodiment, the solid substrate, the matrix, and/or the apheresis matrix can undergo steam sterilization without deteriorating and without resulting in the generation of components that would reduce or challenge the bio- or hemocompatibility of the matrix and/or the apheresis matrix. In embodiments, the solid substrate, the matrix, and/or the apheresis matrix are sterilizable or sterilized by steam for about 20 minutes at 120°C. In embodiments, the matrix can undergo steam sterilization without compromising its hemocompatibility. In further embodiments, steam sterilization does not result in cytotoxic byproducts.

Advantageously, the sterilizability of the components, such as the solid substrate, the matrix, and/or the apheresis matrix, ensures the safety and efficacy of said components, while hemocompatibility is not compromised as a result of sterilization. Non-sterile components can harbor foreign proteins, endotoxins, and other bioburden that can have adverse effects in the body, including but not limited to undesired immune responses or sepsis. Hence, sterilization of said compounds minimizes these contaminants, leading to a safe product with less risk of adverse reactions, while the hemocompatibility of the matrix and/or apheresis matrix are maintained.

Another advantage of the invention is that the intended functionality of the matrix for use in the production of an apheresis matrix was not compromised upon sterilization. The matrix, and any device comprising same, can thus be broadly used for the generation of various apheresis matrices, i.e., by coupling a desired ligand, sterilization, and subsequent use in whole blood apheresis applications. Accordingly, the present invention allows to produce a sterile matrix and/or device or container comprising the same, which can be packed, shipped, stored, and provided as a semi-finished product for the subsequent production or generation of an apheresis matrix by the simplified coupling a ligand of choice as described above.

Such final apheresis matrix can then be sterilized for use in an extracorporeal blood treatment circuit without compromising hemocompatibility and efficacy, either after direct coupling of a ligand to the matrix, or after initial sterilization of the matrix for storage and subsequent use. It was demonstrated herein that the final apheresis matrix can be sterilized as disclosed above without loss of functionality of the apheresis matrix and/or loss of efficacy, whereas the hemocompatibility is maintained and not challenged upon sterilization. It was found that the matrix of the invention does not negatively impact the ligand during the sterilization and thus will not reduce or abolish a ligand's functionality if the ligand as such has the potential to be sterilized under certain conditions.

In one aspect, the present invention relates to a matrix that is designed for coupling ligands and use in extracorporeal blood treatment applications, including for use in whole blood treatment applications and for use in blood plasma treatment applications. In another aspect, the present invention relates to a blood treatment device in the form of an adsorption cartridge or column, comprising the matrix according to the present invention. In yet another aspect, the present invention relates to a blood treatment device in the form of an adsorption cartridge or column, comprising the apheresis matrix according to the present invention, wherein a ligand is coupled to the matrix, wherein the blood treatment device comprising the apheresis matrix is designed for use in extracorporeal blood treatment applications, including the treatment of whole blood or blood components, such as blood plasma.

In embodiments, the blood treatment device is an adsorber cartridge.

In embodiments, the adsorber cartridge comprises an apheresis matrix comprising a double PEGylated matrix coupled to a ligand, said comprising a solid substrate, said substrate comprising a first layer of linear polyethylene glycol (PEG) and at least a second layer of linear polyethylene glycol, wherein said at least second layer is positioned outside the first layer and is bound to said first layer, and wherein the linear polyethylene glycol of the at least second layer is coupled (by a terminal functional group) to said ligand.

In embodiments, the adsorber cartridge comprises an apheresis matrix comprising a double PEGylated matrix coupled to a ligand, said comprising a solid substrate, said substrate comprising a first layer of linear polyethylene glycol (PEG) and at least a second layer of linear polyethylene glycol, wherein said at least second layer is positioned outside the first layer and is bound to said first layer, and wherein the linear polyethylene glycol of the at least second layer is coupled (by a terminal functional group) to said ligand, wherein
- the solid substrate comprises particles, wherein said particles have:
   - a diameter of about 100 µm to about 1000 µm and
   - an average pore size of about 20 nm to about 300 nm,
- the linear polyethylene glycol of the first layer has a molecular weight of about 100 - 2500 Daltons, and
- the linear polyethylene glycol of the at least second layer has a molecular weight of about 100 to 7500 Daltons.

Such a device after coupling a ligand can be a part of an extracorporeal circuit for blood treatment, configured to provide hemodialysis, hemodiafiltration, hemofiltration, apheresis, or plasmapheresis.

The device can be the sole blood treatment device within the blood circuit or can be located, for example, upstream or downstream of the dialyzer in a hemodialysis, hemodiafiltration, or hemofiltration circuit. Alternatively, it can be immediately connected to a dialyzer at a blood inlet or outlet, wherein the device is configured to be perfused with whole blood. In embodiments, the matrix before or after coupling of a ligand can be used in combination devices such as described in US 20150320924 A1, wherein the device comprises hollow fibers in the form of a bundle which is embedded in the housing and held at both ends in a molding compound, wherein the upper and lower end of the hollow fibers protruding the molding compound is open for receiving the solution to be treated in a distribution space, wherein said solution, such as whole blood, passes the lumen of the hollow fibers and is discharged at the opposite end of the hollow fibers into a collection space. The filtrate space surrounding the hollow fibers is closed off from the distribution and collection space and comprises particulate material, such as the matrix of the invention before the ligand is coupled, or the apheresis matrix according to the invention, which after sterilization can be used in extracorporeal blood circuits as described above. In such an embodiment, only a component of the blood, such as, for example, plasma, passes the hollow fiber through which whole blood is perfused. The plasma is then immediately contacted with the apheresis matrix of the invention and will be rejoined with the whole blood by passing again the hollow fiber membrane. The hollow fiber membrane can be chosen according to the size and/or fraction of the blood that is intended to be contacted with the apheresis matrix. This approach might be preferable in cases where the ligand should not be contacted with whole blood for reasons of therapy safety or treatment regimen.

In embodiments, a device according to the invention can be a part of an extracorporeal plasmapheresis circuit, wherein the device is perfused with blood plasma or components thereof. The device can also be used in therapeutic plasma exchange (TPE) treatment, wherein the plasma is removed from the patient and is then replaced with a substitute, e.g., fresh frozen plasma or plasma which has been treated by being contacted with an apheresis matrix according to the invention. According to one embodiment of the invention, the device is used to remove a target molecule from the substitute plasma of a donor before or during its administration to the patient.

In embodiments, the blood treatment device is located in an extracorporeal blood circuit through which the blood of a patient passes, and which comprises means for transporting blood from the patients' vascular system to the blood treatment device at a defined flow rate and for returning the treated blood back to the patient.

The apheresis matrix of the present invention may be configured for or present in various blood treatment systems and/or devices. Without limitation, a blood treatment device can therefore be or comprise an adsorption cartridge comprising an apheresis matrix comprising or based on a matrix according to the present invention. Such a device can be a member of an extracorporeal circuit for blood treatment, configured to provide hemodialysis, hemodiafiltration, hemofiltration, apheresis, and/or plasmapheresis. The device can be the sole blood treatment device within the blood circuit or can be located, for example, upstream or downstream of the dialyzer in a hemodialysis, hemodiafiltration or hemofiltration circuit or can alternatively be immediately connected to the dialyzer at the blood inlet or outlet, wherein the device is configured to be perfused with whole blood. The device can also be a member of an extracorporeal plasmapheresis circuit, wherein the device is perfused with blood plasma or components thereof. The device can also be used in therapeutic plasma exchange (TPE) treatment, wherein the plasma is removed from the patient and is then replaced with a substitute, e.g. fresh frozen plasma or treated plasma, wherein such treatment encompasses contacting the plasma with an apheresis matrix according to the invention.

As mentioned above, the device can also be a hybrid filter device which combines e.g., hollow fiber membranes and an apheresis matrix in the filtrate space of the filter, wherein said apheresis matrix comprises a matrix of the invention with a ligand for capturing, immobilizing, modifying, or processing a target molecule. Such a filter can be a member of an extracorporeal circuit configured for performing hemodialysis, hemodiafiltration or hemofiltration, wherein the said filter is located either upstream or downstream of the dialyzer for hemodialysis, hemodiafiltration or hemofiltration, or it can be used as a sole filter device within an extracorporeal blood circuit in the absence of such dialyzer.

Typically, devices according to the invention are designed as cylinders with a cylindrical housing having at least one inlet and at least one outlet for the blood or blood plasma which is treated with it, or are designed as described in further detail in US 20150320924 A1 when combination devices are used.

In one aspect, the present invention further relates to a ligand coupled to the (at least second layer of) linear polyethylene glycol of the matrix according to the invention for use in the treatment of a medical condition by extracorporeal blood treatment, wherein said extracorporeal blood treatment comprises transporting blood from the patient's vascular system to said ligand at a defined flow rate and returning the treated blood back to the patient.

In embodiments, the medical condition is an inflammatory condition, an autoimmune disease, a condition associated with organ failure, transplantation, and/or a degenerative disease.

Another aspect of the invention relates to a method for the treatment of a medical condition, said treatment comprising extracorporeal blood treatment with a ligand coupled to the (at least second layer of) linear polyethylene glycol of the matrix according to the invention.

In embodiments of the method, said extracorporeal blood treatment comprises transporting blood from a patient's vascular system to said ligand at a defined flow rate and returning the treated blood back to the patient. In embodiments, the method of treatment is configured for the treatment of an inflammatory condition, an autoimmune disease, a condition associated with transplantation or cancer, and/or a degenerative disease.

Another aspect of the invention relates to a method for the treatment of a medical condition, said treatment comprising extracorporeal blood treatment with the apheresis matrix according to the invention, which comprises the matrix of the invention and a ligand of interest which is coupled to the (at least second layer of) linear polyethylene glycol of the matrix. In embodiments of the method, said extracorporeal blood treatment comprises transporting blood from a patient's vascular system to said apheresis matrix at a defined flow rate and returning the treated blood back to the patient.

Another aspect of the invention relates to a method for removing, enzymatically converting, or modifying a target molecule from the blood of a patient, said removing, enzymatically converting, or modifying comprising extracorporeal blood treatment with the apheresis matrix according to the invention.

The various aspects of the invention are unified by, benefit from, are based on, and/or are linked by the structural and/or functional features, including functional properties and beneficial technical effects, of the whole blood compatible matrix for coupling a ligand described herein. The features disclosed in the context of the matrix also apply to and are considered disclosed in the context of the blood treatment device, and vice versa. Any features disclosed in any further aspects of the invention, such as the device, the extracorporeal blood circuit, medical use thereof or medical methods, are also considered disclosed in the context of the inventive matrix, and vice versa.

Any embodiment disclosed herein regarding PEGylation and/or ligand coupling as such, may also represent an embodiment of the present invention regarding the double PEGylated matrices. For example, any figures, examples or embodiments disclosed herein regarding coupling of PEG to the substrate, may be considered to apply to the present invention with respect to coupling the first PEG layer to the substrate. By way of further example, any figures, examples or embodiments disclosed herein regarding coupling of a ligand to the PEG (of the first or any other layer), may be considered to apply to the present invention with respect to coupling the ligand to the second PEG layer. For example, the coupling chemistries employed in coupling PEG to the substrate apply directly to coupling the first PEG layer to the substrate. For example, the coupling chemistries employed in coupling PEG to a ligand apply directly to coupling the second PEG layer to the ligand.

### DETAILED DESCRIPTION OF THE INVENTION

The present invention relates to a whole blood compatible matrix for coupling a ligand and use in extracorporeal blood treatment, comprising a solid substrate, said substrate comprising a first layer of polyethylene glycol (PEG) and at least a second layer of polyethylene glycol, wherein said at least second layer is positioned outside the first layer and is bound to said first layer, and wherein the polyethylene glycol of the at least second layer comprises a terminal functional group for coupling said ligand to said polyethylene glycol. The polyethylene glycol can be linear, branches, forked, or Y-shaped.

As used herein, the terms "first layer of polyethylene glycol (PEG)", "first PEG layer", "inner PEG layer", "first PEG", "first layer", "substrate-coupled PEG", and the like, may be used interchangeably. Accordingly, as used herein, the terms "at least second layer of polyethylene glycol (PEG)", "at least second PEG layer", "outer PEG layer", "at least second layer", "at least second PEG", and the like, may be used interchangeably.

All words and terms used herein shall have the same meaning commonly given to them by the person skilled in the art unless the context indicates a different meaning. All terms used in the singular shall include the plural of that term and vice versa.

### Extracorporeal blood treatment

The invention includes devices which are configured to be located in an extracorporeal blood circuit through which the blood of a patient passes, and which comprises means for transporting blood from the patient's vascular system to a blood treatment device at a defined flow rate and then returning the treated blood back to the patient.

According to the invention, the expression "extracorporeal blood treatment" or "extracorporeal blood purification" refers preferably to the process of removing substances from body fluids through their clearance from flowing blood in a diverted circuit outside the patient's body (extracorporeal). Said substances may include, without limitation, endogenous toxins (i.e., uremic toxins), exogenous toxins or poisons (e.g., ethylene glycol, or fungal toxins), administered drugs, viruses, bacteria, antibodies, metabolites, and proteins (i.e., IMHA, myasthenia gravis), abnormal cells (i.e., leukemia), and/or excessive water. Therapeutic procedures include hemodialysis, including intermittent hemodialysis (HD, HDF, HF), expanded hemodialysis, and continuous renal replacement therapy (CRRT); hemoperfusion; plasma exchange; and therapeutic apheresis. Such methods are known to a skilled person, and the invention device can be incorporated accordingly.

"Therapeutic apheresis (TA)" is a medical procedure involving the extracorporeal separation or removal of blood components to reduce or eliminate the presence of harmful substances or pathological agents from the patient's circulation. It is commonly utilized for conditions mediated by high-molecular-weight molecules, such as antibodies, to mitigate disease severity or progression. TA encompasses various modalities, including but not limited to therapeutic plasma exchange (TPE), red blood cell exchange, leukocyte apheresis, and extracorporeal photopheresis, each tailored to target specific disease mechanisms or pathologies. TPE, the most frequently performed TA procedure, selectively removes plasma containing pathogenic antibodies or immunoglobulins implicated in autoimmune diseases or immune-mediated conditions. Other TA modalities focus on eliminating specific blood components, such as certain proteins, peptides, red blood cells or leukocytes, to achieve therapeutic outcomes. The choice of TA modality depends on the underlying disease pathology and the therapeutic goals of the treatment regimen. These methods are familiar to the person skilled in the art and can be selected based on factors such as flow rates, complication rates, and suitability.

In the context of kidney transplantation, TA serves as an adjunctive therapeutic option to immunosuppressive agents, addressing immunological barriers such as "ABO blood group incompatibility" and preformed donor-specific antibodies. Current techniques include therapeutic plasma exchange (TPE), double-filtration plasmapheresis (DFPP), immunoadsorption (IA), and extracorporeal photopheresis (ECP).

In "hemodialysis," blood is circulated in an extracorporeal circuit, and its composition is modified by the mass transfer of solute and water by diffusive and/or convective forces across an interfacing semipermeable membrane. The magnitude and spectrum of the solute transfer are predicated on the nature of the force(s) imposed across the membrane and on the chemical and physical characteristics of the membrane as well as the solute. Hemoperfusion is an adsorptive extracorporeal therapy used to manage endogenous and exogenous intoxications that cannot be cleared efficiently by hemodialysis, wherein the blood or blood components are contacted with an often-unspecific matrix that immobilizes certain types of compounds.

In embodiments, the invention comprises pre-transplant desensitization treatment using the matrix of the present invention wherein an appropriate ligand is coupled to the matrix of the invention, and wherein the ligand can reduce or eliminate the level of antibodies in the patient, thereby lowering the risk of antibody-mediated rejection. "Pre-transplant desensitization" specifically refers to a therapeutic process aimed at removing or reducing preformed donor-specific HLA antibodies (DSA) in sensitized patients awaiting transplantation, particularly kidney transplantation. Sensitization often occurs due to prior transplants, blood transfusions, or pregnancies, leading to the development of DSAs, which pose a significant barrier to successful transplantation by increasing the risk of antibody-mediated rejection (AMR) and allograft loss. Desensitization protocols typically involve therapeutic plasma exchange (TPE) or immunoadsorption to remove circulating antibodies physically, often combined with intravenous immunoglobulin administration to modulate the immune response. Consequently, the aim of desensitization is to achieve a negative crossmatch and reduce DSA levels to improve transplant outcomes.

"Adsorption" is an extracorporeal technique utilized for blood purification and involves the direct capture and immobilization of particles and target molecules from the blood or plasma by binding them to the surface of an adsorbent material within a purification device. Chemical and physical attraction forces are responsible for retaining the adsorbed molecules, with physical forces including Van-der-Waals and hydrophobic interactions, and chemical interactions involving the formation of chemical bonds. Sorbent materials can be natural or synthetic, with activated carbon and inorganic porous materials offering high adsorption capacity but sometimes exhibiting poor biocompatibility and selectivity. Sorbent materials are available in various forms, with solid particles having preferably a high volumetric surface area. The mechanisms of solute adsorption involve external and internal mass transfer, surface diffusion, and adsorption onto adsorption sites of the solid surfaces. Biocompatibility considerations include hardness, mechanical strength (i.e., avoiding the generation of fine particulate material that may enter the blood stream), cytotoxicity, and immune system activation, which can be addressed through surface coating and anti-adhesion modifications. The design of adsorption devices must optimize sorbent utilization, ensure even flow distribution, and account for blood viscosity and turbulent flows.

Adsorption may also be considered as the principle of molecular attachment of a solute, such as a protein, to a material surface (a matrix). In contrast to the physical separation of toxins from blood by diffusive and convective transport mechanisms over a membrane that occurs during hemodialysis, during hemoperfusion blood is exposed directly to an adsorbent with the capacity to selectively or non-selectively bind solutes within the blood path.

The "vascular system," also known as the "circulatory system," encompasses a network of blood vessels and lymphatic vessels that transport blood and lymph fluid throughout the body. Arteries carry oxygenated blood from the heart to the body tissues, while veins return deoxygenated blood to the heart. Capillaries between arteries and veins facilitate the exchange of oxygen, nutrients, and waste products with body cells. This system is vital for delivering nutrients and oxygen to tissues, removing waste products, and maintaining immune function. It also regulates body temperature and assists in various bodily functions, such as digestion and waste elimination. The vascular system is intricately organized, comprising arteries, veins, and capillaries, each serving specific functions within the broader circulatory system. Additionally, the lymphatic system, which is part of the vascular system, aids in draining excess fluid from tissues and contributes to immune function. Understanding the development and function of the vascular system is crucial for addressing various pathological conditions and developing therapeutic interventions to maintain vascular health.

The expression "blood" as used herein refers to whole blood, which contains all components of the blood of an organism, including red cells, white cells, and platelets suspended in plasma. The expression "blood plasma" refers to the fluid, composed of about 92% water, 7% proteins such as albumin, gamma globulin, fibrinogen, complement factors, clotting factors, and 1% mineral salts, sugars, fats, electrolytes, hormones, and vitamins which forms part of whole blood but no longer contains any cellular components such as red and white cells and platelets.

In the context of the present invention, "blood plasma" or simply "plasma" also encompasses specific fractions of the blood plasma, such as blood serum, adhering to its conventional definition. Various established methods can immobilize target molecules, such as those described in the invention. Preferably, such immobilization is specific or selective, primarily targeting the molecule of interest while minimally affecting other proteins and components in whole blood, blood plasma, or samples thereof (*in vitro*).

"Hemocompatibility" or "blood compatibility" refers to the compatibility of materials and medical devices with blood, ensuring they can interact without causing adverse reactions. Testing for hemocompatibility is vital to assess the safety of devices that come into contact with blood, such as catheters, vascular grafts, and heart valves. ISO 10993-4 outlines testing categories, including thrombosis, coagulation, platelets, hematology, and the complement system. Common issues related to hemocompatibility include thromboembolic complications and material erosion. Surface modifications, like heparin-coating, aim to minimize adverse reactions. Various *in-vitro* models, including static and dynamic systems, allow a comprehensive assessment of blood-material interactions. Key parameters evaluated include changes in blood cell counts, hemolysis, coagulation activation, complement system activation, and platelet count.

Platelet count serves as an indicator of thrombosis risk, reflecting platelet activation and consumption. Compared to standard ranges, a low platelet count may suggest increased platelet activation and consumption, indicating a potential for thrombosis. Conversely, a high platelet count, while not directly indicative of hemocompatibility, may signal inflammation.

Platelet factor 4 (PF4), a protein released from activated platelets, plays a role in clot formation and inflammation. Compared to reference ranges, low levels may indicate heparin-induced thrombocytopenia (HIT), an immune response to heparin medication leading to decreased platelet count, while high levels may suggest heightened platelet activation, potentially due to the material's interaction with blood.

The Thrombin-Antithrombin (TAT) complex is a key enzyme for clot formation and a marker for thrombin generation. Compared to reference ranges, a low TAT complex level might suggest hypofibrinolysis, while high levels could indicate hypercoagulability.

The ISO 10993 standard comprises a series of ISO standards designed for the biological evaluation of medical devices. It serves as a guideline for manufacturers of medical devices and testing laboratories. The primary objective of this standard is to provide guidelines for the assessment of the biological compatibility of materials used in medical devices with the human body. This evaluation encompasses finished products and the starting materials used in their manufacturing process. In addition to biological testing, the standard also encompasses physicochemical tests and analyses of dissolved substances and materials. Moreover, it sets forth requirements for compliance with specified limit values for leachable substances. In Germany, ISO 10993 is published as the DIN standard DIN EN ISO 10993. The matrix described here has undergone testing in accordance with ISO 10993 standards. The results of these tests demonstrate sufficient biocompatibility of the matrix.

Although the matrix of the present invention is compatible with whole blood, methods may be employed in which whole blood is not necessarily employed. In some embodiments of therapeutic apheresis, blood is separated into its component fractions, for example, by centrifugation or by plasma filtration by means of a plasma membrane or filter, wherein the fraction containing the solute which shall be removed, generally the plasma fraction, is treated prior to return to the patient. The present invention also provides for an apheresis treatment in which plasma (containing the target molecules) is removed from the patient's flowing blood and, after having been contacted with an apheresis matrix according to the invention, is returned to the patient. Typical plasma flow rates in an extracorporeal circuit wherein the blood treatment device are perfused with blood plasma is in the range of between 7 ml/min and 250 ml/min, but can, in certain cases, reach up to 300, 400, or 500 ml/min.

According to one aspect, the extracorporeal blood circuit according to the invention is configured to additionally provide continuous renal replacement therapy (CCRT), wherein the circuit which is configured to provide CRRT therapy further comprises a device comprising a matrix of the invention to which a ligand of interest has been coupled. Continuous renal replacement therapies (CRRT) as such are slow dialysis treatments that are provided as continuous (up to about 72 h) therapy, mostly to critically ill patients in an ICU setting. Like in intermittent HD for chronic renal failure patients, solute removal with CRRT is achieved either by convection (hemofiltration), diffusion (hemodialysis), or a combination of both these methods (hemodiafiltration) but generally relies on hemodialysis.

According to another aspect, the extracorporeal blood circuit according to the invention is configured to perform hemoperfusion, wherein the device comprising a matrix according to the invention is the only device located in the extracorporeal circuit. Accordingly, the blood treatment device according to the invention is perfused with whole blood and is located within an extracorporeal circuit. According to one aspect of the invention, the device is a cartridge comprising a matrix according to the invention which bears a ligand having an affinity for a target molecule. According to another aspect, the cartridge can be located downstream or upstream of a hemodialyzer, which is configured to perform hemodialysis on the blood of a patient and can be operated in different treatment modes selected from hemodialysis, hemodiafiltration, and hemofiltration.

### PEG and PEGylation

"Polyethylene glycol (PEG)" is a polymer typically with the general formula C₂ₙH₄ₙ+2Oₙ+1, widely used across various industries due to its liquid or solid state depending on chain length, chemical inertness, water solubility, and non-toxic nature. The PEG basic unit comprises monomers (-CH₂-CH₂-O-) with a molecular weight of 44 g/mol, making its derivatives consist of integer multiples of this weight plus the molecular weight of water and, where applicable, of terminal functional groups. Chemically, PEG is a polyether of ethylene glycol. PEGylation, the process of covalently attaching PEG chains to molecules and surfaces, enhances their properties. PEG comes in various forms, including linear, branched, and comb-shaped configurations, with different molecular weights and geometries affecting their applications. In connection with the present invention, linear, Y-shaped, and branched PEG variants will preferably be used, whereof linear PEGs or Y-shaped PEGs are preferred options. PEG solubility in water and organic solvents and its non-toxicity make it suitable for bioconjugation, drug delivery, tissue engineering, and more. Various PEG derivatives and linkers are available to tailor their functionality to specific applications, offering a wide range of molecular weights, terminal functionalities, and polymer architectures. These derivatives offer advantages such as high cytocompatibility, protein repellency, and tunable degradation kinetics. Commonly, PEG-based hydrogels are prepared via free radical polymerization of polyethylene glycol) diacrylates, although this method has limitations such as low swelling ratio and lack of injectability. Alternative crosslinking strategies have been developed to address these issues, including covalent *in-situ* gelation achieved through crosslinking chemistries like Michael addition, disulfide bond formation, and hydrazone condensation. Injectability is facilitated by co-extruding PEG derivatives functionalized with complementary groups. Degradability can be controlled through enzymatic mechanisms or photoinduced degradation using UV or visible light. Furthermore, modifying the chain structure of PEG, such as attaching biodegradable hydrophobic polymer blocks or organizing PEG chains into star or branched configurations, allows for the creation of hydrogels with tunable properties. Overall, PEG derivatives offer significant potential to enhance the performance of hydrogels in tissue engineering and cell delivery applications by improving mechanics, injectability, and cell-hydrogel interactions.

Part of the rationale for using PEG polymers in bioconjugation applications includes a dramatic increase in the water solubility of modified molecules, a decrease in immunogenicity due to the shielding of modified molecules from the immune system, protection of modified protein and peptides from digestion by proteolytic enzymes, and effectively increasing their hydrodynamic volume and decreasing clearance rates by renal filtration. Coupling of affinity molecules to surfaces can also be enhanced by the use of PEG, in some embodiments as PEG linkers. The PEG linkers on surfaces may provide lower non-specific binding character than alkyl linkers, when preparing surfaces for affinity interactions. Further, the extremely low non-specific binding character of PEG enables creating structured surfaces that other modifiers or linkers do not provide. Surfaces on metals, such as gold particles or planar arrays, have also been enhanced in their performance and properties through the use of PEG-containing modifications (G. Hermanson, Chapter 8, Bioconjugate Techniques, Academic Press, 2013).

In embodiments, the whole blood compatible matrix for coupling a ligand and use in extracorporeal blood treatment comprises a solid substrate having bound thereon two layers of polyethylene glycol (PEG), PEG derivatives, and/or PEG co-polymers. The term PEG as used herein comprises PEG derivatives and/or PEG co-polymers. In embodiments, reference to PEG may include reference to PEG comprising a functional group for the coupling reactions disclosed herein. Reactions between two PEG moieties, such as between the PEG of the first layer and PEG of the at least second layer, may inherently refer to a PEG or PEG derivative that comprises a reactive group suitable for the chemical reaction. In embodiments, the PEG is linear in both the first and the at least second layer.

Polyethylene glycol derivatives refer to polyethylene glycols with modified terminal functional groups, such as ethylamine, propylamine, ethylthiol, propylthiol, ethyl epoxide, various acids, and more. Non-limiting examples include PEG acetic acid, PEG propionic acid, PEG succinate, PEG glutarate, PEG glutaramide, PEG succinamide, PEG ethylamine, PEG propylamine, and so forth.

Functional groups within the polymer chain can also serve as potential sites for modification, enabling the creation of terminal functionalities that might otherwise be unstable, such as those sensitive to hydrolysis or oxidation. For instance, functional groups like S-S bonds or ester bonds can be incorporated into the chain, which can later be cleaved by hydrolysis or reduction (*in-situ* activation), offering flexibility in tailoring the properties and functionality of the polymer.

Copolymers continue to be readily available in industrial applications. For instance, Clariant provides corresponding products such as polyalkylenes or polyglycols, including copolymers like PEG-PPG (a copolymer of polyethylene glycol and polypropylene glycol). Additionally, compounds like Jeffamines, also known as polyetheramines, can be used.

Another example is a family of PEG copolymers featuring amine end groups. These copolymers consist of varying proportions of polyethylene glycol (PEG) and polypropylene glycol (PPG), sometimes with only a few units of propylene glycol at the chain's end, accompanied by amine end groups.

Furthermore, copolymers like PEG-lactic acid (PEG-PLA) and similar variations such as PEG-polycaprolactone (PEG-PCL) also might be employed. These copolymers are biodegradable, making them suitable for specific applications. Despite their limited scope, they hold significant potential for particular applications.

The coupling of all these polymer chains can be executed using the same methods described earlier, which may include capping procedures to ensure chemical deactivation and elimination of unwanted reactivity and thus appropriate hemocompatibility of the resulting matrix.

As described herein, the term "polyethylene glycol (PEG)" also encompasses PEG derivatives as well as PEG copolymers, even when not explicitly mentioned.

As used herein, "PEGylation" is a process that involves attaching polyethylene glycol (PEG), PEG derivatives, and/or PEG (co-)polymer chains to other molecules such as drugs, therapeutic proteins, or vesicles, and includes solid surfaces and support materials used in the medical industry. This attachment can be achieved through covalent or non-covalent means, resulting in what is referred to as PEGylated derivatives. It is known that covalent attachment of PEG to a molecule can "mask" it from the immune system, increase its hydrodynamic size, and/or improve water solubility. This process has significant pharmacological advantages, including improved drug solubility, reduced dosage frequency, extended circulating life, increased drug stability, and enhanced protection from degradation. PEGylation can be achieved through various chemical methods, including amine conjugation, thiol conjugation, and oxidized carbohydrate or N-terminal conjugation, all of which are known in the art.

Reversible PEGylation techniques control the release of proteins *in vivo.* PEGylation has been widely used in pharmaceuticals to improve the treatment of various diseases. The choice of PEG size, structure, and conjugation method plays a pivotal role in determining the properties and effectiveness of the resulting PEGylated product. PEG size refers to the molecular weight of the polymer, which can significantly impact factors such as circulation time, solubility, and immunogenicity. The structure of PEG, including linear, branched, or comb-shaped configurations, influences how effectively the PEGylated molecule interacts with its target and navigates physiological barriers. Moreover, the choice of conjugation method can affect the site specificity and stability of the conjugated product. By optimizing these parameters, PEGylation can enhance the pharmacokinetics, bioavailability, and therapeutic efficacy of the drug or therapeutic protein.

The creation of bioconjugate reagents with spontaneously reactive or selectively reactive functional groups forms the basis for simple and reproducible crosslinking or tagging of target molecules (G. Hermanson, Chapter 3, Bioconjugate Techniques, Academic Press, 2013). Some reagents may be used to form active intermediates with another functional group. These active intermediates can subsequently be coupled to a second molecule that possesses the correct chemical constituents, which allows bond formation to occur. Major reactions used in bioconjugation along with the appropriate reaction partners are known to a skilled person in the art.

### Immobilization

The invention includes devices that are configured to be located in an extracorporeal blood circuit through which the blood of a patient passes and which comprises means for transporting blood from the patient's vascular system to a blood treatment device at a defined flow rate and then returning the treated blood to the patient, and wherein the device is further configured to capture and/or modify compounds from/in whole blood thereby chemically or structurally changing them and/or ultimately removing them from the blood of the patient.

The "matrix," as used herein, thus refers to a material inside the blood treatment device that provides an internal material or surface through or over which blood or plasma is passed, wherein the matrix preferably is functionalized with a ligand which is coupled to the matrix (resulting in the "apheresis matrix") making it suitable for use in the targeted treatment of a medical condition by extracorporeal blood treatment. The matrix or apheresis matrix can be utilized for a range of applications, including filtration, cell separation, scaffolding, adsorption, and/or immobilization, depending on the specific needs of the application. The matrix, as used in the context of the present invention, preferably comprises a solid substrate having bound thereon two layers of polyethylene glycol (PEG), which is specifically tailored for use in whole blood applications and to increase the hemocompatibility of the matrix as well as.

Features or embodiments related to the matrix may also relate to the apheresis matrix and vice versa. For example, defining features of the matrix apply equally to the apheresis matrix, which additionally comprises a ligand.

The "solid substrate," as used herein, refers to the portion of the matrix that serves as the "substrate", "base matrix" or "support material" to which the PEG of the first layer, according to the invention, is coupled. Such support or support material is sometimes also referred to as "adsorption material" or "adsorber", as used in an unspecific "adsorption column", "column", or "adsorption cartridge". Suitable support materials, according to the present invention, should be uniform, hydrophilic, mechanically and chemically stable over the relevant pH and temperature range with no or negligible leaching of the PEG during use, have good flow characteristics for whole blood and/or blood plasma, and provide a large surface area for PEG attachment as well as subsequent attachment of a ligand for the generation of an apheresis matrix. As is shown in the context of the present invention, it is also possible to use hydrophobic support materials if adequately modified with PEG according to the invention. In embodiments, the solid substrate is in the form of a particle. In another embodiment, the solid substrate is in the form of a bead. In another embodiment, the solid substrate is a porous bead. Using particles or beads facilitates efficient packing in adsorber devices. Moreover, the size, shape, and material composition of the particles or beads can be designed to achieve specific functionalities.

In embodiments, the size of the column is 5 to 500 mL, preferably 50 to 350 mL, most preferably 100 to 250 mL. In embodiments, the size of the column is 5, 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500 mL, but may also have individual sizes in between any of the explicitly mentioned sizes.

The expression "coupling" of or "to couple" a ligand to the matrix for providing an apheresis matrix, which in some embodiments can be used in a device according to the invention, as used herein refers to a non-covalent or covalent interaction that holds two molecules together. According to one embodiment of the invention, the expression refers to a covalent interaction, i.e., to couple ligands covalently. Non-covalent interactions include but are not limited to, hydrogen bonding, ionic interactions among charged groups, van derWaals interactions, and hydrophobic interactions among non-polar groups. One or more of these interactions can mediate the coupling of two molecules to each other. Coupling may otherwise refer to specific, selective, or unspecific binding.

"Binding", "capturing", or "immobilization" generally refers to the physical association between a target molecule and a binding molecule such as a ligand. The association typically depends upon the presence of a particular structural feature of the target molecule, such as an antigenic determinant, epitope, binding pocket, or cleft, recognized by the ligand.

As used herein, the" binding capacity" of an apheresis matrix refers to the maximum amount of target molecules that the apheresis matrix can capture before reaching saturation. This parameter is critical in determining the effectiveness and efficiency of the final apheresis matrix, which is determined to a significant extent by the design of the underlying matrix.

"Polymers" are substances or materials comprising macromolecules consisting of multiple repeating subunits. They exhibit a diverse range of properties, making them indispensable in various applications. These macromolecules are formed through polymerization, wherein numerous monomers are linked together covalently. Polymers possess distinctive physical properties, including, in some cases, toughness, high elasticity, and the ability to form amorphous or semicrystalline structures. They can be broadly classified into two types: natural and synthetic. Natural polymers may include collagen, hyaluronic acid, chitosan, and alginate. Non-limiting examples of synthetic polymers are polyethylene, poly (lactic-co-glycolic acid) (PLGA), polycaprolactone (PCL), polyhydroxyalkanoate (PHA), silicone, polytetrafluoroethylene (PTFE), polyetheretherketone (PEEK), hydrogels, conductive polymers, and polyethylene glycol) (PEG). Polymerization processes can be categorized into step-growth and chain polymerization methods. Polymers' physical properties, such as mechanical strength and transport properties, are heavily influenced by factors like their molecular weight, microstructure, arrangement, and chemical nature of monomers. The morphology of polymers, describing their spatial arrangement at the microscopic level, further affects their macroscopic properties and behavior.

"Linear polymers" are characterized by a continuous arrangement of monomer units along the polymer chain without branching or cross-linking. These polymers typically exhibit a semi-crystalline structure in the solid state, with regularly folded molecular chains forming dense chain packs called crystallites or lamellae. Examples of linear polymers include polyethylene and isotactic polypropylene, where the molecular chains are arranged in a zigzag conformation or helix, respectively. Linear polymers with periodic structures and low branching often display semi-crystalline properties, leading to enhanced stiffness, density, and resilience. The absence of significant branching allows for a more orderly arrangement of chains, contributing to the polymer's overall strength and thermal properties.

In embodiments, the PEG of the first layer coupled to the substrate is linear PEG. In embodiments, the linear PEG is coupled at both termini to the substrate, thereby forming a PEG loop. In embodiments, the linear PEG of the first layer is not coupled at both termini to the substrate, but only at one terminus, thus enabling coupling of a second PEG layer to said (inner) first layer.

"Poly(methyl methacrylate)" (PMMA) is a transparent thermoplastic derived from methyl methacrylate. Commonly known as acrylic, it finds use as an alternative to glass due to its lightweight and shatter-resistant properties. Its properties include high light transmission, good impact strength, and ease of processing. The synthesis of PMMA encompasses a range of methods, including emulsion polymerization and bulk polymerization. Its properties are customizable by adding comonomers, plasticizers, and fillers, allowing for the tailoring of PMMA to meet specific application needs. PMMA is widely utilized as a biomaterial across various applications due to its biocompatibility, strength, durability, and cost-effectiveness.

"Acetylation" is an organic esterification reaction involving introducing an acetyl group into a chemical compound.

"Hydrolysis" entails breaking one or more chemical bonds within a molecule. It encompasses various processes such as substitution, elimination, and solvation reactions, wherein water acts as a nucleophile.

A "covalent bond" is a chemical bond in which electrons are exchanged to form electron pairs between atoms; in chemistry, it refers to the interatomic bond formed by sharing electron pairs between two atoms.

"Functional groups" in organic chemistry refer to specific arrangements of atoms within molecules that impart characteristic chemical properties to those molecules. These groups can undergo predictable reactions regardless of the surrounding molecular structure. Functional groups also play a crucial role in determining the solubility, reactivity, and other chemical and biological properties of organic molecules. Functional groups are essential building blocks in organic chemistry, providing a framework for understanding the vast array of organic compounds.

In embodiments of the present invention, coupling can be carried out via common functional groups. In embodiments, the functional groups used herein are selected from amines, cystamine HCl, carboxy, hydroxy, epoxy, thiol, aldehyde, squaric acid ester, active ester, maleimide, haloacetamide, haloacetate, isocyanate, halogen, azide, alkyne, alkene, silane, oxyamine, hydrazide, biotin, nitrophenyl/succinimidyl carbonate, sulfonate, acrylate, acrylamide, vinyl sulfone.

As mentioned above, the ligand according to the invention is preferably bound covalently to the matrix via the terminal functional groups of the PEG of the at least second layer. The solid substrate which forms the basis for the generation of a matrix to which said ligand can be attached covalently must provide or facilitate chemical reactivity, thus allowing the chemical coupling of the PEG, to which the ligand is coupled. Also understood under coupling is the attachment of PEG to the solid substrate. As described herein, the matrix of the present invention comprises a PEG coupled to the substrate, wherein said PEG has bound thereon at least a second layer of PEG, and wherein the PEG of the at least second layer has a terminal functional group for coupling a ligand to said PEG.

Many coupling methods for immobilizing molecules, such as the PEG or ligands, such as antibodies or fragments thereof, to their respective substrate, are well known in the art. In general, the bond resulting from coupling two components should be stable over a wide range of pH, buffer conditions and temperatures resulting in negligible leaching of PEGs and/or ligands. The coupling method should avoid improper orientation, multisite attachment or steric hindrance of the PEG or ligand, which may cause masking of binding sites and, subsequently, lead to loss of activity. Site-directed attachment and/or spacers can be considered for immobilizing a ligand or PEG onto the substrate. The ligand and/or PEG density per volume of matrix can be optimized to promote target accessibility and binding.

The coupling is preferably carried out via common functional groups, including amines, alcohols, carboxylic acids, aldehydes, and epoxy groups. Additional embodiments are provided herein. Methods of preparing substrates according to the invention are known in the art and are described, for example, in US 8,142,844 B2, US 2015/0111194 A1, and US 2014/0166580 A1, which are incorporated by reference. These references also describe spacer groups (or "linker" groups) which can advantageously be used in generating the matrix according to the invention.

According to one embodiment of the invention, the ligand is coupled to PEG of the at least second layer through its first terminus of the PEG, and the PEG of the first layer is coupled to the solid substrate, optionally using a spacer, via an amine function on the second terminus of the PEG molecule. In a first step, an amine group is introduced onto the solid substrate or PEG, respectively. Various known methods can be used to introduce amine groups according to the invention. For example, addition of aminated polymers (e.g., aminated polyvinylalcohols) to the polymer solution prior to membrane precipitation or post-treatment of membranes such as silanization of a membrane containing hydroxyl or/and carboxyl groups using APTMS ((3-aminopropyl)trimethoxysilane-tetramethoxysilane), simple adsorption of PEI (polyethylene imine)) or other polymers onto the membrane surface, or plasma treatment of the membranes with ammonium or other organic amine vapors can effectively be used to introduce amine groups. In a second step, carbodiimide compounds can, for example, be used to activate carboxylic groups of proteins for direct conjugation to the primary amines provided on the matrix via amide bonds. The most commonly used carbodiimides are the water-soluble EDC (1-ethyl-3-(-3-dimethylaminopropyl) carbodiimide) for aqueous crosslinking and the water-insoluble DCC (N', N'-dicyclohexyl carbodiimide) for non-aqueous organic synthesis methods.

According to another embodiment of the invention, hydroxyl groups can be introduced to the substrate. According to one specific embodiment, hydroxyl groups can be introduced by hydrolyzing epoxide groups. For example, solid substrates carrying epoxide groups for further chemical modification or coupling of molecules are widely available. Solid substrates based on polysaccharides, for example, cellulose or cellulose derivatives, already carry OH-groups on the surface. Hydroxy groups can also be introduced to the substrate, for example, by plasma treatment with oxygen or air. After acylation of the hydroxy group with succinic anhydride, the resulting O-succinate can react with the amine of the protein with amide bond formation in the presence of carbodiimide or other coupling reagents.

According to yet another embodiment of the invention, carboxylic acid groups can be introduced to the solid substrate. Carboxylate groups can be introduced on said substrates by plasma treatment with carbon dioxide. For protein immobilization, carbodiimide/succinimide coupling chemistry can be used for surface attachment via amine groups of the ligand. According to yet another embodiment of the invention, carbonyl groups (aldehydes, ketones) can be introduced for the subsequent coupling of a ligand. Aldehydes can be created on polysaccharide-based solid substrates by oxidation of OH-groups using periodic acid. The primary amines of proteins (N-terminus of polypeptides and the side chain of lysines) can react with aldehydes via reductive amination and/or the formation of a Schiff base. The formation of a Schiff base is reversible in aqueous solutions. The reduction of the Shiff base irreversibly yields an alkylamine linkage. Sodium cyanoborohydride is a mild reducing agent that induces this reaction without also reducing other chemical groups of proteins.

According to still another embodiment of the invention, epoxy groups can be introduced to the solid substrate. Several pre-activated resins coated with high density epoxy functional groups on the surface are available commercially, see herein. The epoxide group which reacts with nucleophiles in a ring-opening process reacts with primary amines, thiols, or hydroxyl groups of proteins to form stable secondary amines, thioesters, and ether bonds, respectively. The epoxide groups readily react with thiol groups and require buffered systems close to physiological pH (pH 7.5-8.5). The epoxide groups require high pH conditions (pH 11-12) for reacting with hydroxyl groups and moderate alkaline conditions (pH >9) for reaction with amine groups. In each case, spacers of varying chain length can be introduced between the matrix and the affinity ligand.

There are several types of solid substrates, as mentioned above and below, that can be advantageously utilized to couple, for example, PEGs to form a matrix according to the invention, which subsequently can be transformed into an apheresis matrix by coupling a ligand. Affinity substrates can be based on materials such as polysaccharides. Suitable polysaccharides are, for example, cellulose, nitrocellulose, chitosan, collagen, starch, and cross-linked polysaccharide gels such as agarose, Sephadex, or Sepharose. Methods for preparing derivatives of polysaccharide solid substrates or base matrices have long been known and are, for example, described in US 4,411,832 or US 3,947,352, which are incorporated by reference. The solid substrates can also be based on synthetic organic substrates. Synthetic polymeric matrices comprise hydrophilic and hydrophobic synthetic polymers and combinations thereof. Synthetic solid substrates comprise substrates selected, for example, from the group of substrates consisting of polyacrylamide substrates or derivatives thereof; polymethacrylate substrates or derivatives thereof; polystyrene substrates or derivatives thereof; or polyethersulfone substrates or derivatives thereof. Otherwise, derivatized silica, glass, or azlactone beads can be used in devices according to the invention. Such devices preferably make use of organic substrates. The use of beads may be advantageous in the context of the present invention.

According to one aspect of the present invention, the solid substrates carry specific functional groups which are needed for coupling PEG thereto. For example, many functionalized resins are commercially available and known to a person with skill in the art. Preactivated resin substrates which already carry a reactive group for the coupling of PEG with or without a spacer are available commercially and eliminate many of the steps of chemical activation of the solid substrate prior to use as mentioned before, i.e., prior to the coupling of PEG. Such substrates are generally resins as defined before, whereas for membrane and/or non-woven substrates the step of activation generally must be performed before coupling. A wide range of coupling chemistries, involving primary amines, sulfhydryls, aldehydes, hydroxyls, and carboxylic acids are available in said commercial substrates for covalently attaching PEG. Examples for commercially available activated resins are: UltraLink lodoacetyl resin, CarboLink Coupling resin, Profinity^{™} Epoxide resin, Affi-Gel 10 and 15, Epoxy-activated Sepharose^{™} 6B, Tresyl chloride-activated agarose, Tosoh Toyopearl^{®} AF Amino or Epoxy 650-M, ChiralVision Immobead^{™} 350, Resindion ReliZyme^{™} EXE 135 or SEPABEADS^{™}, Purolite^{®} Lifetech^{™} methacrylate polymers functionalized with epoxy or amino groups, and ChiralVision IB-COV and IB-ANI beads methacrylate polymers functionalized with epoxy, amino and/or hydroxyl groups.

According to one embodiment of the invention, the solid substrate used for the coupling of PEG is epoxy functionalized because epoxy groups form very stable covalent linkages with different chemical functional groups such as, for example, -NH₂ or nucleophiles (amino, thiol, phenolic). In addition, coupling can be performed under mild conditions with respect to pH and temperature.

According to another embodiment, primary amino functionalized resins such as Lifetech^{™} ECR8409F with a particle size of from 150 to 300 µm can be used for the coupling of PEG to the base matrix (see also Lifetech™ ECR Enzyme Immobilization Procedures Application Guide (2019)).

According to another embodiment of the invention, the substrate takes the form of beads. According to another embodiment of the invention, the substrate is an epoxy-functionalized methacrylate polymer. According to yet another embodiment of the invention, the substrate is selected from the group of substrates consisting of Tosoh Toyopearl^{®} Epoxy 650-M, ChiralVision Immobead^{™} 350, Resindion ReliZyme^{™} EXE 135, Resindion SEPABEADS^{™}, and Purolite^{®} Lifetech^{™}. According to one aspect, Purolite^{®} Lifetech^{™} ECR8209F epoxy methacrylate beads are used which carry an epoxy group as a functional group to which PEG can be bound. They have a mean pore diameter of between 600 and 1200 Å and a particle size of between 150 and 300 µm. According to another aspect, Purolite^{®} Lifetech^{™} ECR8215M epoxy methacrylate beads are used which carry an epoxy group as a functional group to which PEG can be bound. They have a mean pore diameter of between 1200 and 1800 Å and a particle size of between 300 and 710 µm. According to another aspect, Purolite^{®} Lifetech^{™} ECR8215F epoxy methacrylate beads are used which carry an epoxy group as a functional group to which PEG can be bound. They have a mean pore diameter of between 1200 and 1800 Å and a particle size of between 150 and 300 µm.

According to yet another embodiment, the substrate according to the invention comprises magnetic beads. Magnetic beads are prepared by entrapping magnetite within agarose or other polymeric material, on which PEG and subsequently the ligand according to the invention is coupled. Following the interaction of ligand and target molecule, under the influence of a magnet, rapid separation can be achieved. The use of magnetic beads is especially indicated in extracorporeal applications which are *in vitro* applications and wherein the apheresis matrix for capturing the target molecule is configured for monitoring the presence and/or concentration of the target molecule in a blood or blood plasma sample or in any other *in vitro* application comprising the target proteins, for screening or other analytical purposes and is not part of the extracorporeal blood circuit.

### Ligands

As disclosed herein, a "ligand" is a molecule, atom, or ion that binds reversibly to a target molecule, forming a complex to serve a specific biological purpose, whereby enzymes are also included under ligands. Ligand-target interactions occur through weak intermolecular forces such as ionic bonds, hydrogen bonds, Van-der-Waals forces, and hydrophobic effects. Ligands include substrates, inhibitors, activators, signaling lipids, and neurotransmitters, amongst many other molecules, and they can trigger conformational changes in a protein, altering functional state. Affinity measures the strength of the interaction between a ligand and its binding site on the target molecule, with high-affinity binding indicating strong attraction between the molecules.

In embodiments, the ligand selectively binds or enzymatically processes a target molecule in whole blood. In embodiments, the ligand is a polypeptide, preferably an antigen, antibody, or fragment thereof, or an enzyme. In embodiments, the ligand is a nucleic acid molecule, such as an aptamer. In embodiments, the ligand is a DNA, RNA, enzyme, carbohydrate, and/or a subset thereof.

In further embodiments, the ligand is a primary and/or secondary metabolite. Non-limiting examples of primary metabolites are carbohydrates, amino acids, nucleotides, organic acids, and fatty acids. Non-limiting examples of secondary metabolites are alkaloids, terpenes, phenolics, glycosides, and antibiotics. In another embodiment, the ligand is a hormone, neurotransmitter, vitamin, signaling molecule.

In some embodiments, the strong bond formed during ligand coupling ensures minimal loss of ligands. In other embodiments, the coupling chemistry ensures a stable covalent binding of the ligand while preserving its structural and functional integrity. In further embodiments, the ligand is characterized by its non-toxicity, biocompatibility, and hemocompatibility.

Different ligands offer varying degrees of selectivity, kinetics, and stability. Additionally, different coupling chemistry methods provide varying levels of coupling efficiency. Various molecules have different spatial requirements, leading to varying maximum loading densities. Consequently, depending on the target molecule, specific classes of ligands may be advantageous. This flexibility enables rapid adaptation to new indications. A person skilled in the art is able to select a ligand appropriate for a particular target molecule.

An "antigen (Ag)" refers to a compound, composition, or substance bound by an antibody. An antigen stimulating an "immune response" or "immune reaction," such as the production of antibodies or a T-cell response in an animal or human subject, is also referred to as an "immunogen". Antigens and/or immunogens usually comprise protein, lipid, and/or carbohydrate structures; however, nucleic acids may also represent autoantigens.

As used herein, an "antibody" generally refers to a protein consisting of one or more polypeptides substantially encoded by immunoglobulin genes or fragments of immunoglobulin genes. Where the term "antibody" is used, the term "antibody fragment" may also be considered to be referred to. The recognized immunoglobulin genes include the kappa, lambda, alpha, gamma, delta, epsilon, and mu constant region genes and the myriad immunoglobulin variable region genes. Light chains are classified as either kappa or lambda. Heavy chains are classified as gamma, mu, alpha, delta, or epsilon, defining the immunoglobulin classes IgG, IgM, IgA, IgD, and IgE. The basic immunoglobulin (antibody) structural unit is known to comprise a tetramer or dimer. Each tetramer is composed of two identical pairs of polypeptide chains, each pair having one "light" (L) (about 25 kD) and one "heavy" (H) chain (about 50-70 kD).

Herein, "nucleic acid" may preferably refer to DNA (deoxyribonucleic acid), gDNA (genomic deoxyribonucleic acid), RNA (ribonucleic acid), gRNA (genomic ribonucleic acid), mRNA (messenger ribonucleic acid) and cDNA (complementary deoxyribonucleic acid synthesized from RNA template), or any combination thereof, further also encompassing L-DNA and L-RNA forming so-called mirror-images of the naturally occurring conformation of DNA. As used herein, "nucleic acid" shall mean any nucleic acid molecule, including, without limitation, DNA, RNA, and hybrids or modified variants thereof, including L-DNA and L-RNA.

In embodiments, the synthetic oligonucleotide includes oligonucleotides containing modified backbones or non-natural internucleoside linkages. The invention encompasses oligonucleotides with at least one structural modification that provides improved stability and/or half-life of said oligonucleotide post-administration in a cell and/or organism compared to a structurally unmodified oligonucleotide of the same sequence. Preferred modified oligonucleotide backbones include, for example, phosphorothioates, chiral phosphorothioates, phosphorodithioates, phosphotriesters, aminoalkylphosphotriesters, methyl and other alkyl phosphonates. Various salts, mixed salts, and free acid forms are also included.

In some embodiments of oligonucleotide variants, both the sugar and the internucleoside linkage, i.e., the backbone, of the nucleotide units are replaced with non-natural groups. The base units are maintained for hybridization with an appropriate nucleic acid target compound. One such oligomeric compound, an oligonucleotide mimetic that has been shown to have excellent hybridization properties, is referred to as a peptide nucleic acid (PNA).

"Aptamers" are short sequences of artificial DNA, RNA, XNA, or peptides designed to bind specific target molecules or families of molecules, exhibiting affinities in the pM to µM range. They are akin to chemical antibodies but differ structurally, being mostly oligonucleotides, in contrast to protein-based antibodies. They identify disease markers, serve as drugs, drug delivery vehicles, or controlled release systems and perform molecular engineering tasks. The process of aptamer discovery typically involves SELEX, where aptamers are iteratively selected from a diverse pool of nucleic acid sequences. Aptamers can be optimized for specific characteristics such as target binding specificity, resistance to degradation, and controlled release kinetics. Aptamers offer advantages over antibodies in terms of ease of sequencing, lower production costs, and reduced immunogenicity. Peptide aptamers, a subtype of aptamers, are synthetic proteins engineered to bind specific targets. Peptide aptamers are artificial proteins consisting of peptide loops displayed by a protein scaffold selected or engineered to bind specific target molecules. They offer unique advantages in molecular binding due to their constrained structural diversity.

"Biomolecules" encompass various molecules crucial for biological processes such as cell division and metabolism. They include large macromolecules like proteins, carbohydrates, lipids, and nucleic acids and smaller molecules such as vitamins and hormones. Proteins, consisting of amino acid chains, play roles in structural support, enzymatic reactions, and cellular signaling. Nucleic acids, including DNA and RNA, encode genetic information and facilitate protein synthesis. Carbohydrates are energy sources and structural components, while lipids function in energy storage, membrane formation, and signaling. These biomolecules exhibit intricate structures and functionalities, enabling the complexity and diversity observed in living organisms.

"Small molecules," with a molecular weight of less than 900 Daltons, constitute a diverse group of organic compounds that play essential roles in various biological processes and pharmacological applications. Unlike larger molecules such as proteins and nucleic acids, small molecules can regulate biological functions by binding to specific targets, including receptors and enzymes. These compounds can exert their effects by modulating signaling pathways, inhibiting enzymatic activity, or disrupting protein-protein interactions. Non-limiting examples of small molecules are metabolites (e.g., sugars), signaling molecules (e.g., hormones, neurotransmitters, cytokines), natural products (e.g., alkaloids, glycosides, terpenes, antibiotics), drugs, toxins, poisons, lipids (e.g., triglycerides, phospholipids, cholesterol), carbohydrates (e.g., glucose, starch, cellulose), amino acids, nucleotides (e.g., adenine, guanine, thymine, cytosine), heterocyclic compounds (e.g., alkaloids, purines, pyrimidines), and/or aromatic compounds (e.g., benzene, toluene, naphthalene).

In embodiments, the ligand of the present invention is a small molecule, preferably glycosides, alkaloids, synthetic molecules, and/or synthetically derived compounds.

"Sterilization" refers to the process of eliminating or deactivating all forms of life, particularly microorganisms such as bacteria, fungi, spores, and viruses, as well as other biological agents, from a surface, object, or fluid. This can be achieved through various methods, including heat, chemicals, irradiation, high pressure, and filtration. Unlike disinfection or sanitization, which reduce but do not eliminate all forms of life, sterilization ensures complete eradication of microorganisms. Objects rendered sterile are referred to as aseptic. Sterilization methods include heat sterilization using steam or dry heat, chemical sterilization with agents like ethylene oxide or hydrogen peroxide, radiation sterilization using gamma rays, X-rays, or electron beams, and sterile filtration using membrane filters. Unsterilized biopolymers can cause severe infections in the human body when used for biomedical applications. Hence, biopolymers are required to undergo sterilization. The biopolymers that have been sterilized include both natural and synthetic biodegradable polymers. Sterilization methods that can be applied to biopolymers include but are not limited to steam-autoclaving, dry heat sterilization, irradiation (gamma (γ)-rays, X-rays, ultraviolet, and electron beams), chemical treatment (ethylene oxide), gas plasma, and supercritical fluid sterilization.

In some embodiments, the solid substrate, the matrix, and/or the apheresis matrix can undergo radiation sterilization methods such as gamma, X-ray, and/or e-beam. In further embodiments, radiation sterilization does not result in cytotoxic byproducts. According to one embodiment, the solid substrate, the matrix, and/or the apheresis matrix are sterilized by e-beam treatment According to another embodiment, the solid substrate, the matrix, and/or the apheresis matrix are sterilized by gamma irradiation.

In other embodiments, the solid substrate, the matrix, and/or the apheresis matrix can undergo steam sterilization. In further embodiments, steam sterilization does not result in cytotoxic byproducts.

In further embodiments, the whole blood compatible matrix for coupling a ligand and use in extracorporeal blood treatment, comprising a solid substrate having bound thereon polyethylene glycol (PEG), is sterilizable or sterilized. In embodiments, the apheresis matrix comprising the matrix with a ligand coupled thereto is sterilizable or sterilized. In embodiments, the blood treatment device comprising the matrix for coupling a ligand and use in extracorporeal blood treatment, and the apheresis matrix described herein are sterilizable or sterilized.

In further embodiments, the whole blood compatible apheresis matrix is used in immunoaffinity chromatography. The term "immunoaffinity chromatography" (IAC) is generally used for an affinity chromatography method in which the apheresis matrix comprises an antibody or an antigen-binding fragment thereof (Moser et al., Bioanalysis (2010) 2:769-790). In the context of the present invention, the apheresis matrix comprises, in embodiments, a ligand that captures and/or processes a target molecule. The selective capture of a target molecule by the ligand is a result of a large variety of non-covalent interactions that can occur between an antibody and an antigen such as the target molecule according to the invention and can result in association equilibrium constants in the range of 10⁵-10¹² M⁻¹.

By way of example, due to the generally large size of naturally occurring antigens, antibodies that bind to several different regions of the antigen with a range of binding affinities are often generated. Each individual location on an antigen/target molecule that can bind to an antibody is called an epitope. According to one aspect of the present invention, an antibody can bind to any epitope, provided the association equilibrium constants of the interaction are in the range of 10⁵-10¹⁵ M⁻¹. According to another aspect of the invention, an antibody is selected which binds to a specific epitope, wherein binding inhibits the target molecule's activity once it is immobilized on the apheresis matrix but is still present in the bloodstream of the patient. According to another aspect of the invention, the association equilibrium constants of the interaction are in the range of 10⁶-10¹² M⁻¹, are in the range of 10⁶-10¹⁰ M⁻¹, are in the range of 10⁸-10¹⁰ M⁻¹, or are in the range of 10⁸-10¹² M⁻¹.

According to one embodiment of the invention, the matrix may comprise affinity tags for immobilizing a ligand on the matrix. Affinity tags can be used for purifying a protein during production. In the context of the present invention, affinity tags are used for binding a ligand to the matrix of the present invention. Different types of affinity tags are well known in the art (Terpe, Appl Microbiol Biotechnol (2003) 60:523-533), wherein polyhistidine or Hiss-tags are especially well described and are one option for binding ligands according to the invention to the matrix. Affinity tags which can otherwise be used for coupling the ligand to the matrix can be selected from the group comprising C-myc-tags, FLAG-tags, and Hemagglutinin (HA)-tags.

According to another embodiment of the invention, a ligand is covalently attached to the solid support via PEG, as further detailed below and/or as described in the prior art (Cuatrecasas, J Biol Chem (1970) 245:3059-3065; Nisnevitch et al., J Biochem Biophys Methods (2001) 49:467-480), wherein PEG acts as a linker as well as a means for providing hemocompatibility of the matrix and apheresis matrix, for optimizing the coupling efficiency of the matrix towards a ligand, and for providing an optimized ligand density. Covalent coupling generally includes either covalent non-site directed attachment of the ligand which is based on utilizing functional groups on either the matrix and/or the ligand (Nisnevitch et al., J Biochem Biophys Methods (2001) 49:467-480, Section 2.3). According to another embodiment of the invention the covalent attachment of ligands is a site-directed attachment of the ligand (Nisnevitch et al., J Biochem Biophys Methods (2001) 49:467-480, Section 2.4; Makaraviciute et al., Biosensors and Bioelectronics (2013) 50:460-471). Essentially, technical guidance for attaching a ligand to a matrix, via PEG, as is carried out in immunoaffinity approaches, may be applied to immobilize the ligand via PEG on the support.

### Diseases to be treated

"Inflammation" is a fundamental biological response initiated by the body's tissues in reaction to harmful stimuli like pathogens, injury, or irritants. It involves a complex interplay of immune cells, blood vessels, and molecular mediators to eliminate the initial cause of injury, clear damaged cells and tissues, and initiate tissue repair. Acute inflammation is the immediate response to injury, involving increased movement of leukocytes into the affected tissues. Chronic inflammation, on the other hand, persists over months or years, leading to a shift in cell types present at the site of inflammation and simultaneous tissue destruction and repair. While acute inflammation is a protective mechanism, chronic inflammation is associated with various diseases like atherosclerosis, arthritis, and allergies. Inflammation can be classified based on the type of cytokines and helper T cells involved, and it is essential to distinguish between inflammation and infection, as they are often correlated but can have different underlying causes. Causes of inflammation include physical, biological, chemical, and psychological factors, with acute inflammation typically triggered by exposure to substances, injury, or infection. In contrast, chronic inflammation may result from hypersensitivity, autoimmune disorders, or persistent acute inflammation.

"Autoimmune disorders" or "immunological diseases," refer to conditions in which the immune system malfunctions, either by overactivity (autoimmune disorders) or underactivity (immunodeficiency disorders). Non-limiting examples of autoimmune disorders are Rheumatoid arthritis, Lupus (Systemic Lupus Erythematosus), Lupus nephritis, Multiple sclerosis, Guillain-Barré syndrome, chronic inflammatory demyelinating polyneuropathy (CIPD), Myasthenia gravis, Goodpasture's syndrome, and Pemphigus.

"Hematological disorders" encompass various conditions that affect the blood and blood-forming organs, including red blood cells, white blood cells, platelets, bone marrow, lymph nodes, and spleen. These disorders can be genetic or acquired and may manifest as anemia, clotting disorders, or abnormalities in blood cell production. Examples include but are not limited to sickle cell disease, thalassemia, hemophilia, and von Willebrand disease. Symptoms vary depending on the disorder but may include fatigue, weakness, abnormal bleeding or bruising, and increased susceptibility to infections. Diagnosis typically involves blood tests and sometimes a bone marrow biopsy. Treatment options include medications, blood transfusions, and surgical interventions such as splenectomy.

"Degenerative diseases" encompass a spectrum of conditions marked by the progressive deterioration of tissues or organs over time. These diseases can affect various bodily functions, including movement, cognition, and organ function. While some degenerative diseases have known causes, such as genetic factors or environmental toxins, others may arise spontaneously without a clear etiology. Neurodegenerative diseases, in particular, involve the malfunctioning or death of cells within the central nervous system, leading to debilitating conditions such as Alzheimer's disease and Parkinson's disease. Non-limiting examples of other degenerative diseases include various forms of muscular dystrophy, cardiovascular diseases like atherosclerosis, and certain cancers. Although treatments may help manage symptoms, many degenerative diseases lack a cure, emphasizing the importance of comprehensive care and management plans tailored to individual needs. Thus, in embodiments, the present invention enables the treatment of aforementioned degenerative diseases.

"Cancer" is a term that refers to a group of diseases in which abnormal or transformed cells divide without control and can invade nearby tissues, including disease forms where cancer cells spread to other parts of the body through the blood and lymph systems. cfDNA, which originates from such cancer cells or tumor microenvironment and may circulate in the blood of a patient, is known to have the potential to enter neighboring or distal cells and may be capable of altering the biology of recipient cells. This phenomenon has been implicated in the oncogenic transformation of normal cells and the development of metastases (Bronkhorst et al. (2019), Biomolecular Detection and Quantification 17:100087.

In embodiments, the whole blood compatible matrix may be used, for example, in the treatment of above-mentioned conditions, but is not limited to the diseases listed herein.

### Treatment

As used herein, the terms "individual", "patient", and "subject" are often used interchangeably and refer to a human or animal that exhibits a symptom of a disease, disorder, or condition that can be treated with the whole blood compatible matrix, the ligand coupled thereto, and/or the blood treatment device comprising same. In preferred embodiments, a subject includes a human being or animal that exhibits symptoms of a disease, disorder, or condition that can be treated with methods disclosed herein.

As used herein, the terms "(medical) disease," "(medical) disorder," and "(medical) condition" are often used interchangeably.

As used herein, "treatment" or "treating" includes any beneficial or desirable effect on the symptoms or pathology of a disease or pathological condition and may include even minimal reductions in one or more measurable markers of the disease or condition being treated. Treatment can optionally involve either the reduction or amelioration of symptoms of the disease or condition or the delaying of the progression of the disease or condition. "Treatment" does not necessarily indicate complete eradication or cure of the disease, condition, or associated symptoms. The phrase "therapeutically effective" is intended to include, within the scope of sound medical judgment, excessive toxicity, irritation, and/or other problems or complications but commensurate with a reasonable benefit/risk ratio.

As used herein, "prevent" and similar words such as "prevented", "preventing", or "prophylactic", etc., indicate an approach for preventing, inhibiting, or reducing the likelihood of the occurrence or recurrence of a disease or condition. It also refers to delaying the onset or recurrence of a disease or condition or delaying the occurrence or recurrence of the symptoms of a disease or condition. As used herein, "prevention" and similar words also include reducing the intensity, effect, symptoms, and/or burden of a disease or condition prior to the onset or recurrence of the disease or condition.

The instant disclosure also includes kits, sets, packages, and multi-container units containing the herein-described whole blood compatible matrix, the apheresis matrix, and/or the device comprising the same, and further including, where applicable, means for mounting same on equipment for providing extracorporeal blood treatment to a subject in need.

It will be understood that particular embodiments described herein are shown by way of illustration and not as limitations of the invention. The principal features of this invention can be employed in various embodiments without departing from the scope of the invention. Those skilled in the art will recognize or be able to ascertain, using most routine study, numerous equivalents to the specific procedures described herein. Such equivalents are considered to be within the scope of this invention and are covered by the claims. All publications and patent applications mentioned in the specification indicate the skill level of those skilled in the art to which this invention pertains. All publications and patent applications are herein incorporated by reference to the same extent as if each individual publication or patent application was specifically and individually indicated to be incorporated by reference.

The use of the word "a" or "an" when used in conjunction with the term "comprising" in the claims and/or the specification may mean "one," but it is also consistent with the meaning of "one or more", "at least one", and "one or more than one". The term "or" in the claims is used to mean "and/or" unless explicitly indicated to refer to alternatives only or the alternatives are mutually exclusive. However, the disclosure supports a definition of only alternatives and "and/or". Throughout this application, where relevant, the term "about" indicates that a value includes the inherent variation of error for the device, the method employed to determine the value or the variation among the study subjects.

### FIGURES

The invention is demonstrated by way of example in the following figures. The figures are to provide a further description of potentially preferred embodiments that enhance the support of one or more non-limiting embodiments of the invention.

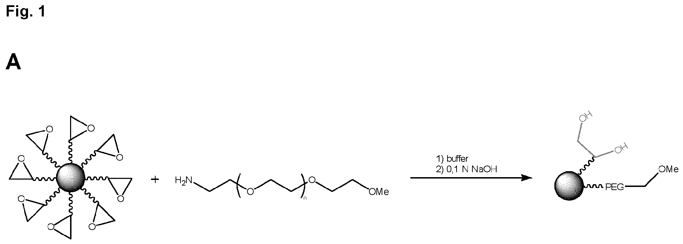

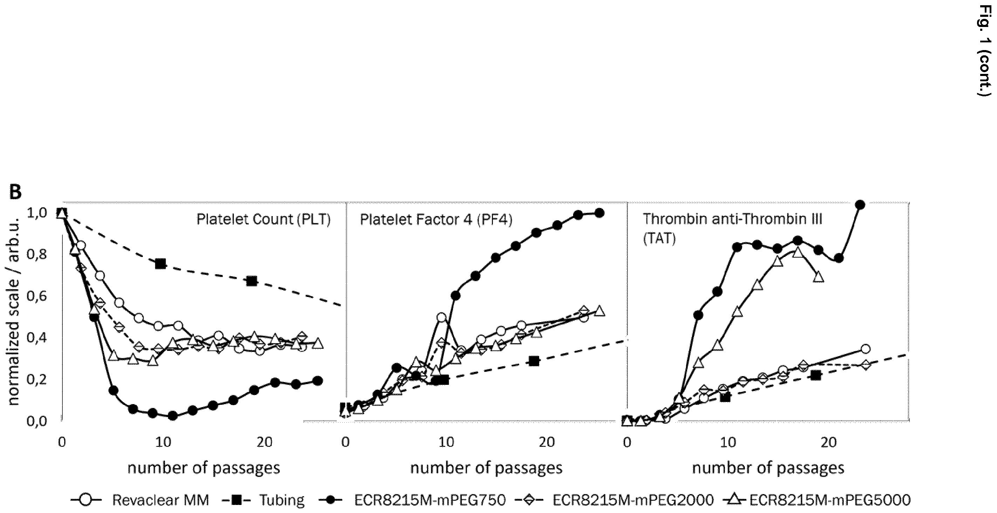

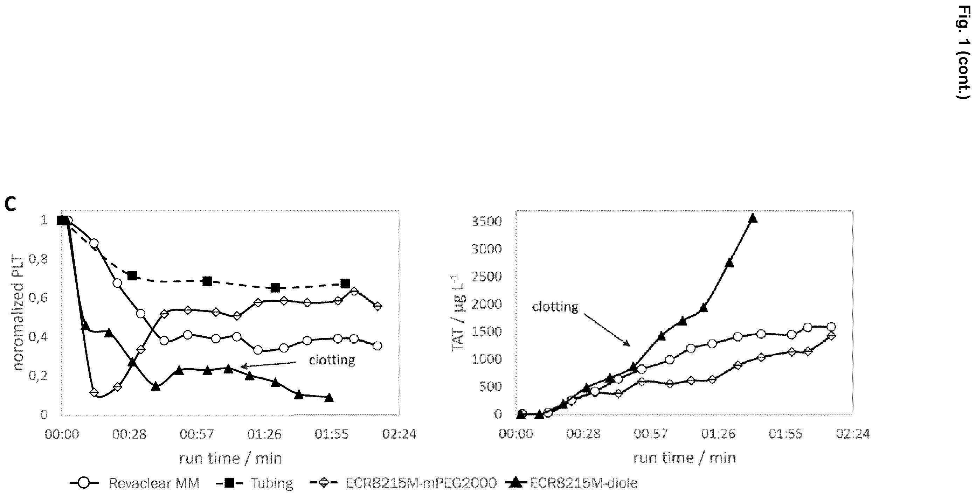

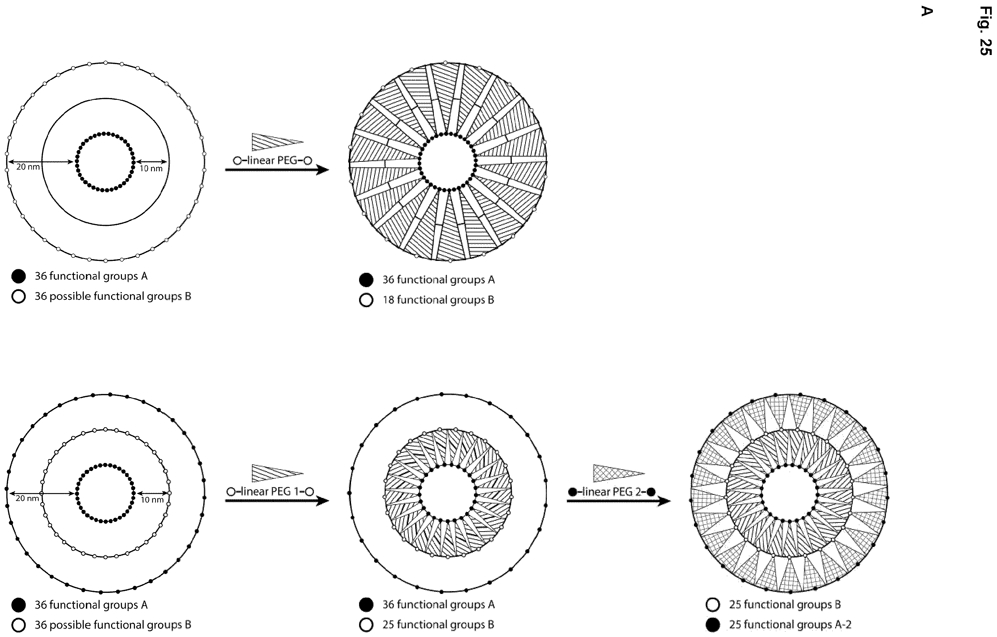

Figures 1 to 24 represent precursor technologies that represent groundwork for the claimed technology of the present invention. Figures 25 to 28 serve as illustrative examples of the present invention.

Any examples from figures 1-24 regarding PEGylation and/or ligand coupling may also represent embodiments of the present invention regarding the double PEGylated beads. For example, any figures, examples or embodiments disclosed herein regarding coupling of PEG to the substrate, may be considered to apply to the present invention with respect to coupling the first PEG layer to the substrate. By way of further example, any figures, examples or embodiments disclosed herein regarding coupling of a ligand to the PEG (of the first or any other layer), may be considered to apply to the present invention with respect to coupling the ligand to the second PEG layer. For example, the coupling chemistries employed in coupling PEG to the substrate apply directly to coupling the first PEG layer to the substrate. For example, the coupling chemistries employed in coupling PEG to a ligand apply directly to coupling the second PEG layer to the ligand.

### Brief description of the figures:

**Figure 1****:** Hemocompatibility testing.
**Figure 2****:** Capping strategies enhance hemocompatibility of PEGylated beads.
**Figure 3****:** PEGylation prevents non-specific protein adsorption.
**Figure 4****:** Evaluation of Factor XII adsorption on beads.
**Factor 5:** Influence of pore size.
**Figure 6****:** Influence of the diameter of the particle.
**Figure 7****:** Enhancing blood compatibility through PEGylation of various matrices.
**Figure 8****:** Sterilizability of the PEGylated matrix with X-ray and E-beam.
**Figure 9****:** Sterilizability of the PEGylated matrix with Gamma, X-ray, and E-beam.
**Figure 10****:** Steam sterilization of the PEGylated matrix.
**Figure 11****:** Hemocompatibility of the construct.
**Figure 12****:** Schematic of PEG-coated solid phase

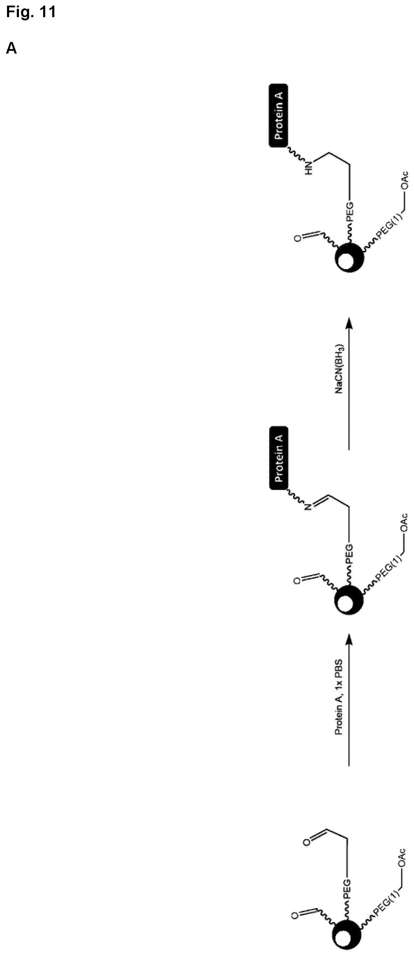

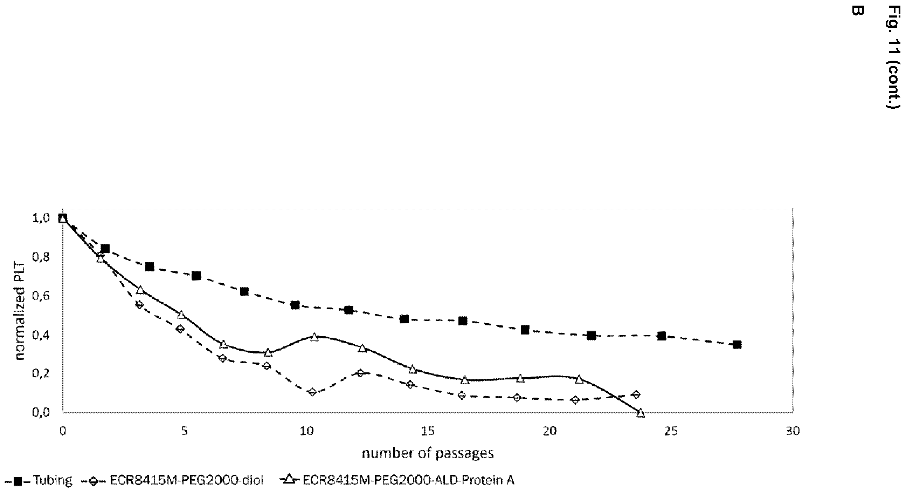

**Figure 11****:** Hemocompatibility of the construct.
**Figure 12****:** Schematic of PEG-coated solid phase.
**Figure 13****:** Synthesis of functionalized PEGs for immobilization on epoxy resins.
**Figure 14****:** PEGylation of Purolite Epoxy Resin.

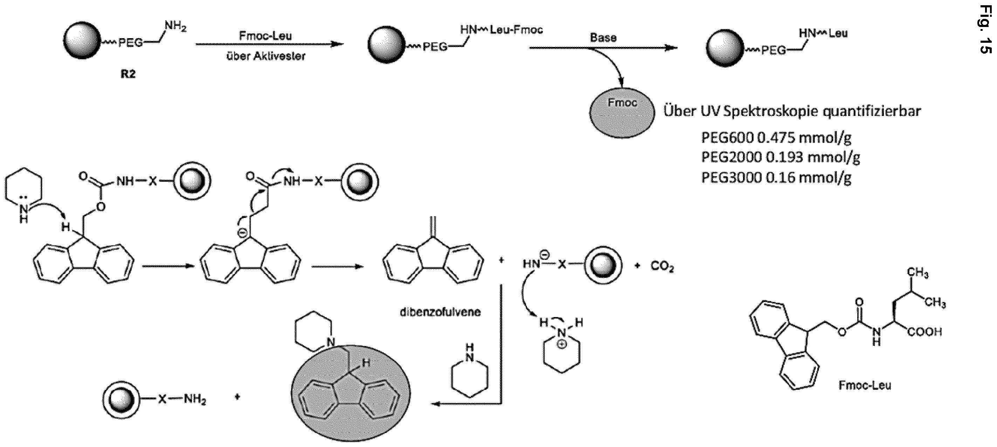

**Figure 15****:** Determination of the amine content of the PEGylated resin.

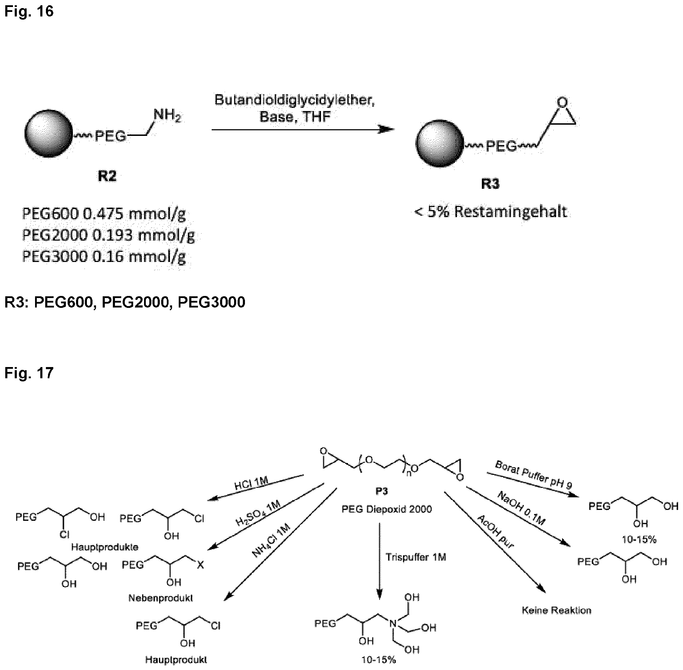

**Figure 16****:** Functionalization of the PEGylated Purolite resins.
**Figure 17****:** Stability of epoxide end groups (on PEG).

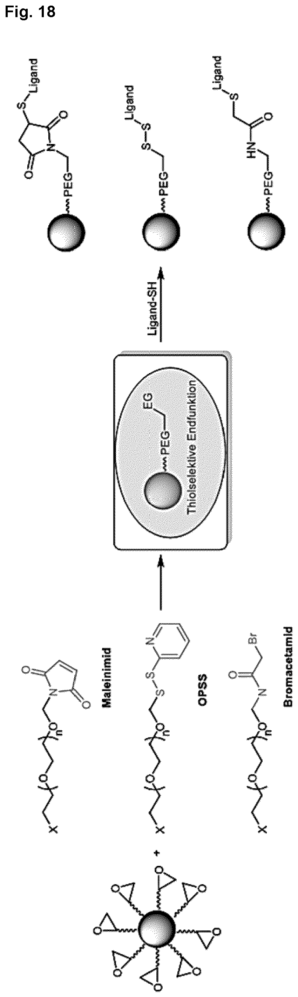

**Figure 18****:** Pathways to further selective adsorbent materials.

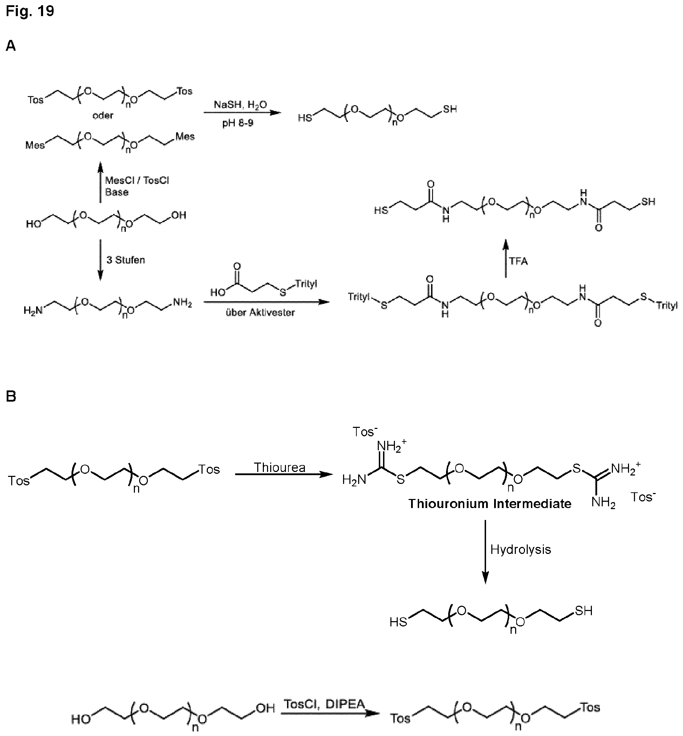

**Figure 19****:** Synthesis of functionalized PEGs for immobilization on epoxy resins.

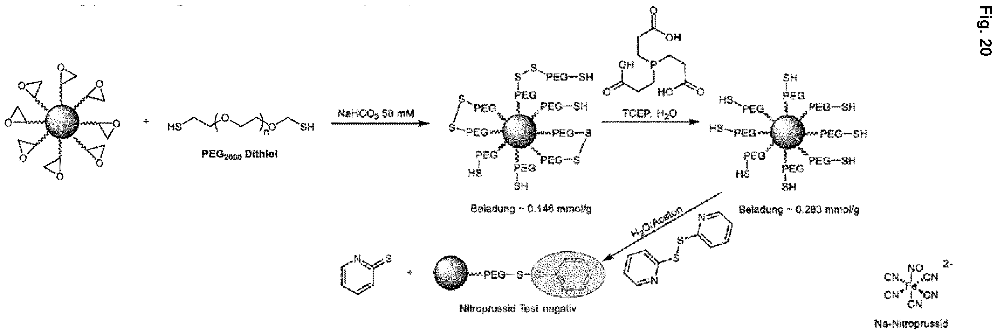

**Figure 20****:** PEGylation of Purolite epoxy resin with PEG thiols.

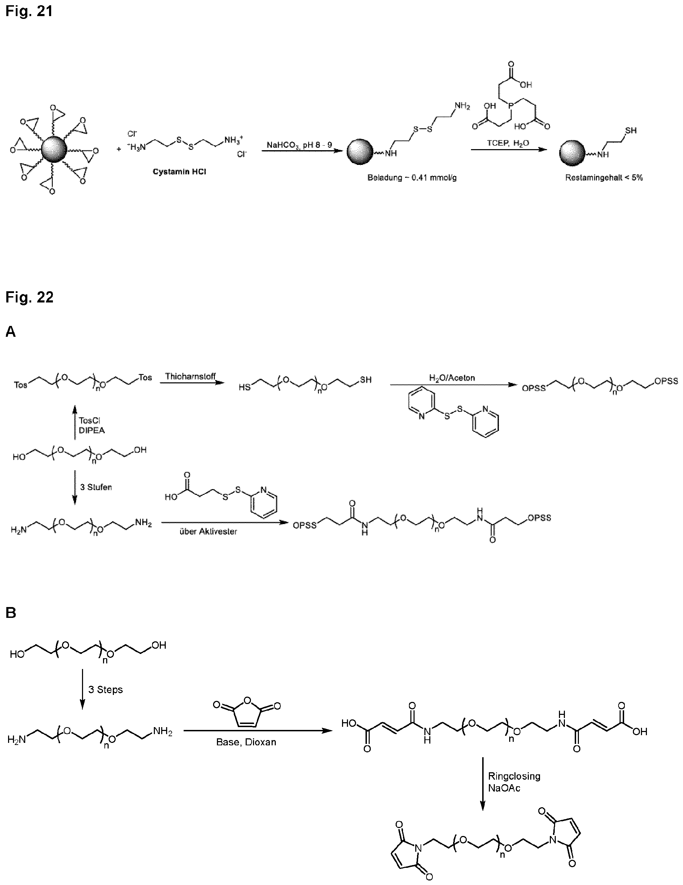

**Figure 21****:** Synthesis of Purolite resin with thiol surface.
**Figure 22****:** Synthesis of thiol-selective PEGs for immobilization on thiol resins.

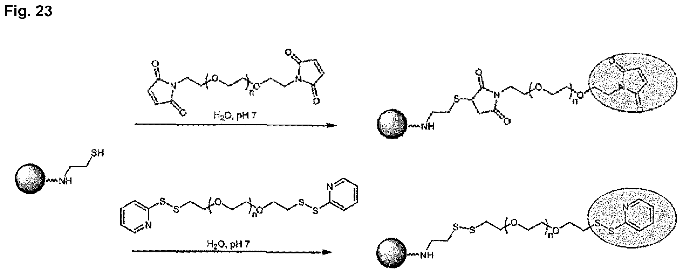

**Figure 23****:** Synthesis of thiol-selective Purolite Resin.

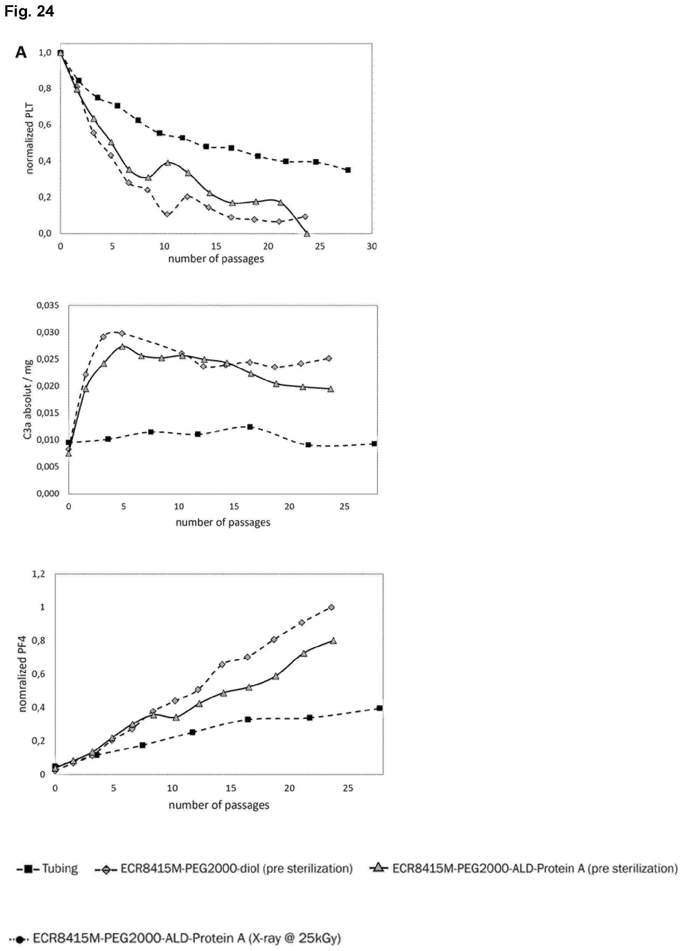

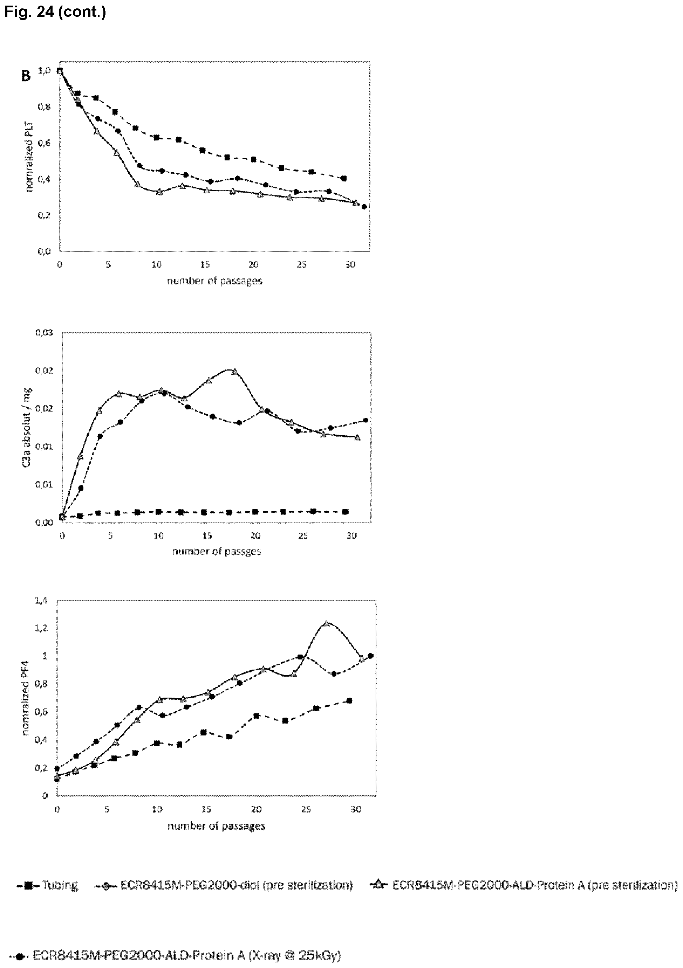

**Figure 24****:** Comparison of PEGylated Beads (ECR8415M-PEG2000) with Protein A loaded PEGylated beads (ECR8415M-PEG2000-ProteinA) in relation to a tubing/system reference.
**Figure 25****:** Schematic illustration of the solid substrate with two layers of PEG.

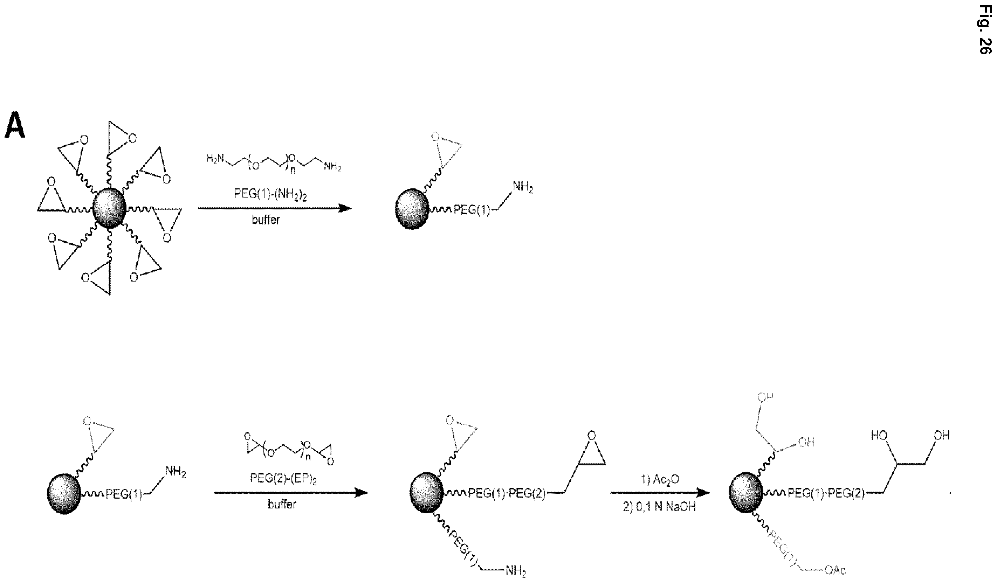

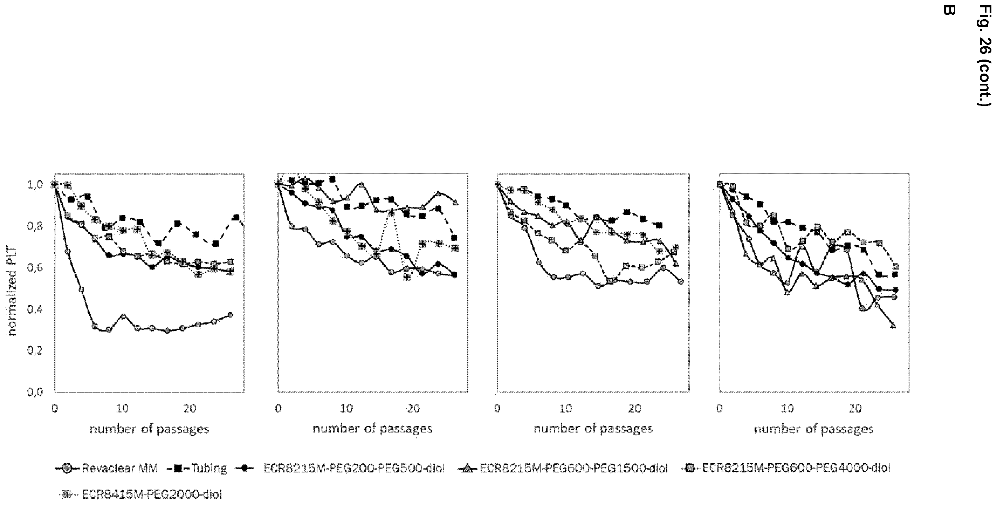

**Figure 26****:** Hemocompatibility testing of the solid substrate with two layers of PEG.
**Figure 27****:** PEGylation prevents non-specific protein adsorption of the solid substrate with two layers of PEG.

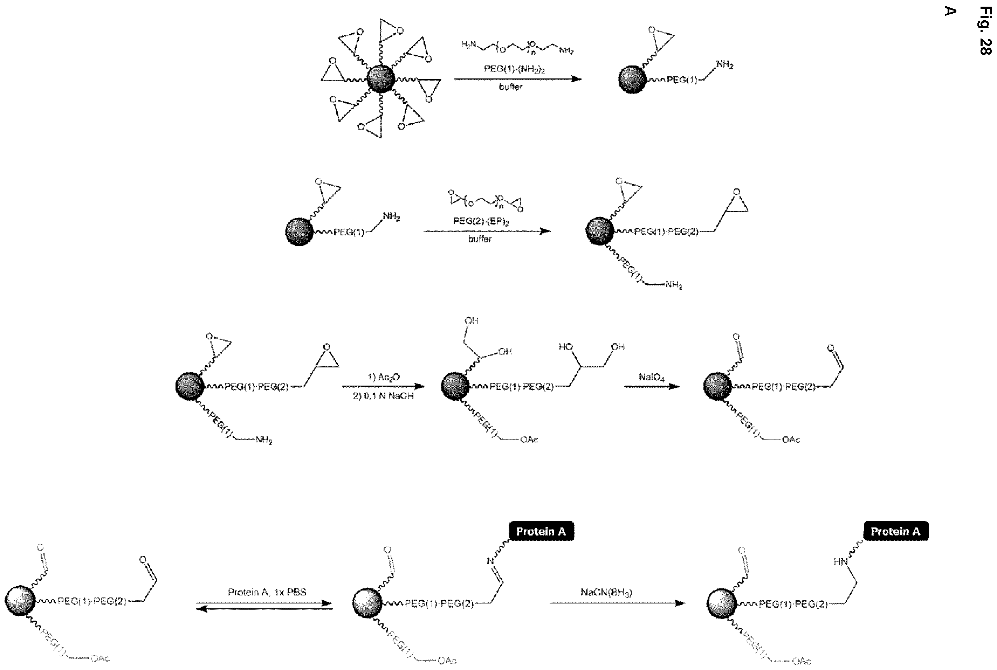

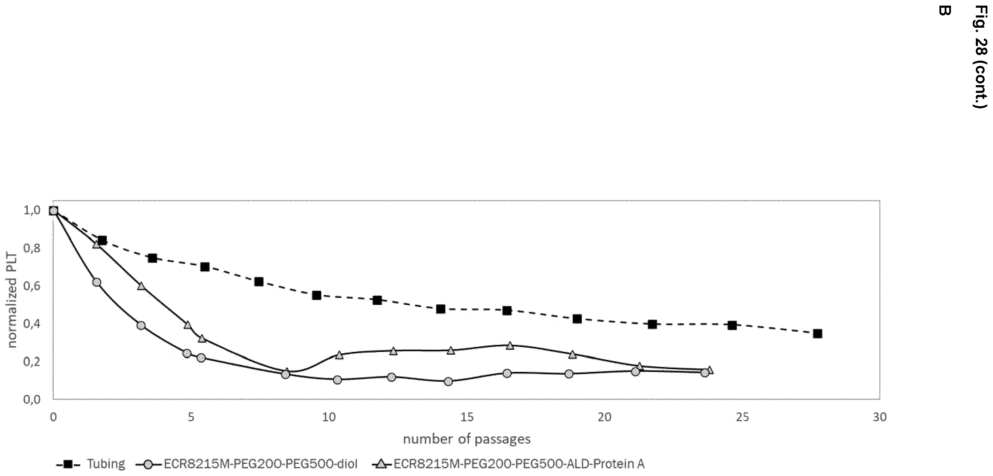

**Figure 28****:** Hemocompatibility of the construct with the double PEGylated solid substrate.

### Detailed description of the figures:

**Figure 1****: Hemocompatibility testing.** (A) Illustration depicting the surface functionalization of base beads with a methoxy-functionalized (CH₃O-, OMe) PEG. (B) Comparison of results of a dynamic perfusion experiment using human whole blood. The change in platelet count (PLT), platelet factor 4 (PF4) and Thrombin-anti-Thrombin III TAT is plotted versus the number of passages. The comparison shows beads coated with methoxy-functionalized PEG750, PEG2000, and PEG5000, a miniaturized Revaclear dialyzer, and a tubing reference. The drop in platelet count is less pronounced the longer the PEG chain and on a similar level as the miniaturized Revaclear dialyzer, indicating enhanced blood compatibility. The initial "dip" in platelet count observed is attributed to primary (reversible) hemostasis, likely influenced by the presence of OMe functionalities on the bead surface (as shown in panel A). The increase in platelet factor 4 (PF4) measured for beads coated with PEG2000 or PEG5000 is comparable to that recorded for the tubing reference and the miniaturized Revaclear dialyzer, while TAT is elevated in beads coated with PEG750 and PEG5000 compared to the references. (C) Comparison of the hemocompatibility between "naked" and PEGylated beads. The change in PLT (left) and TAT (right) is plotted against time. The comparison shows beads coated with methoxy-functionalized PEG2000, diole, a miniaturized Revaclear dialyzer, and a tubing reference.

**Figure 2****: Capping strategies enhance Hemocompatibility of PEGylated beads.** (A) Illustration depicting various capping strategies for PEGylated beads. (B) Capping the functionalities of the base beads (hydrolysis of epoxides and acetylation of amines, among others) influences blood compatibility. Primary unreacted amines on the ECR8415M solid substrate/solid support? result in poor blood compatibility despite PEGylation. Acetylation is achieved by reacting PEGylated beads with an excess of acetic anhydride (4 times the volume of beads) for over 16 hours. Capping using BDE (1,4-butanediol diglycidyl ether) is accomplished by treating PEGylated beads with BDE in a NaHCOa buffer at alkaline pH and subsequent hydrolysis of the epoxides using 0.1 N NaOH (for 12 hours). Capping of the terminal epoxide functional groups of the PEG can be achieved by reaction with 0.1 M NaOH (for 2 hours) or 1M ethanolamine (pH 9, 16 hours).

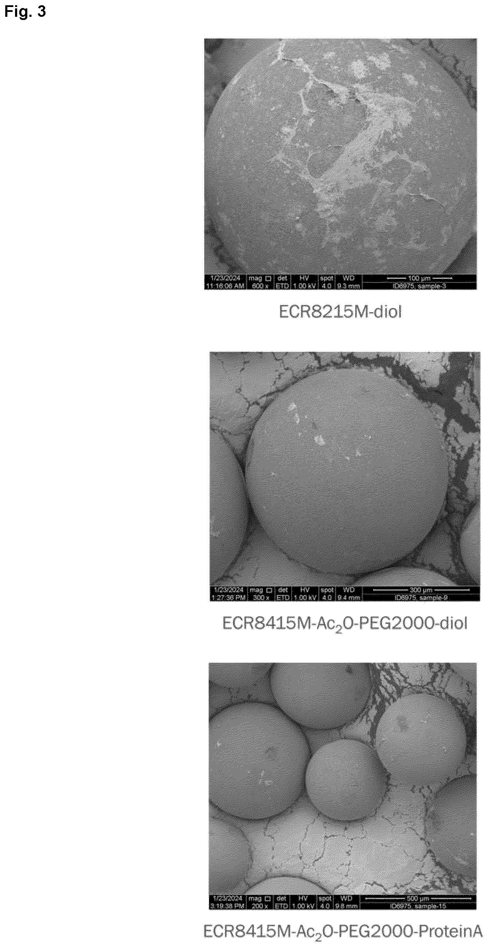

**Figure 3****: PEGylation prevents non-specific protein adsorption.** As depicted in the SEM images, the "naked" beads are covered by fibrin and activated platelets. In contrast, beads coated using PEG2000, as well as beads coated with PEG2000 and having Protein A coupled to the PEG surface, exhibit almost no adhesion of activated platelets or fibrin, which is less pronounced in mPEG2000 beads. To produce the shown samples, 0.5 mL beads were incubated with 4 mL of whole blood in 5 mL polypropylene tubes at 37°C on a roller mixer. Subsequently, the beads were washed with 0.9% NaCl solution, followed by rinsing with a 1% glutaraldehyde solution, and left overnight (>16 hours) in the refrigerator. The beads were then washed several times with deionized water and dried in a freeze-dryer.

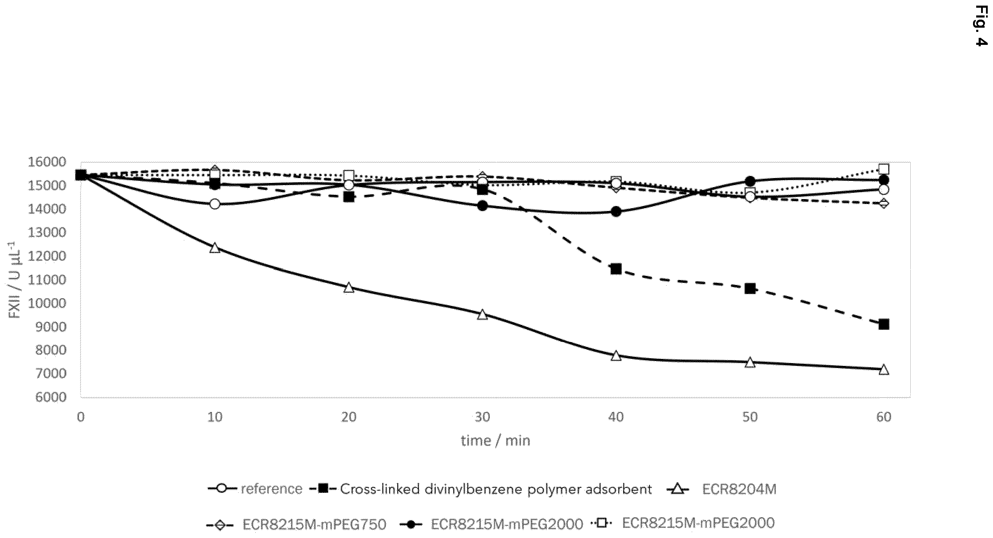

**Figure 4****: Evaluation of Factor XII adsorption on beads.** Factor XII (coagulation factor) adsorption/non-specific adsorption and/or absorption effects were further investigated in another test. For this purpose, 1 mL of beads was transferred to 5 mL polypropylene tubes (suspended in 0.9% NaCl solution). To these beads, 0.1 mL of 0.9% NaCl and 3.9 mL of human pooled plasma were added, and the tubes were incubated on a roller mixer at 37°C for various durations. The collected samples were analyzed using Factor XII ELISA. It is evident that uncoated beads (ECR8204M) as well as cross-linked divinylbenzene polymer adsorbent as used in adsorber cartridges today, adsorb Factor XII, whereas all PEGylated candidates show no adsorption of Factor XII.

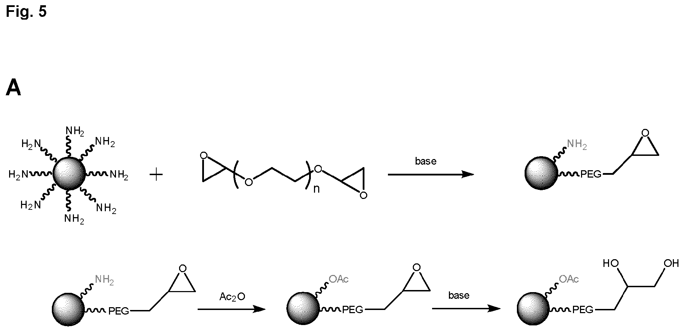

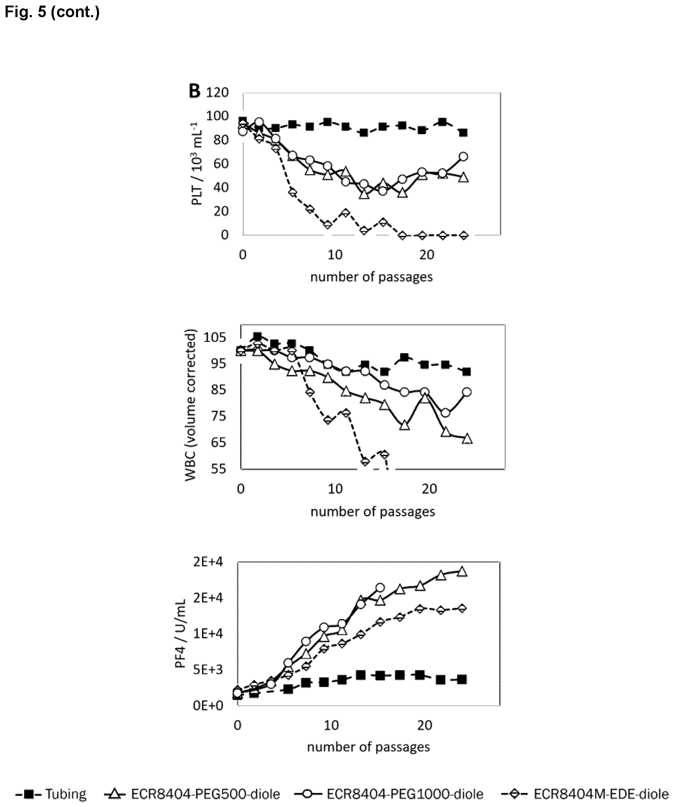

**Figure 5****: Influence of pore size.** (A) Illustration depicting the synthesis route for PEGylated beads capped through acetylation. (B) Distinct beads were evaluated to assess the impact of pore size; ECR8404-PEG500-diole, ECR8404-PEG1000-diole, and ECR8404M-EDE-diole. It is evident that even with small pores (ECR8404M with a diameter dₚₒᵣₑ of about 40 m), good or significantly improved blood compatibility can be achieved, even with shorter PEGs. This can be attributed to the fact that all pores are "blocked" by PEGylation, thereby reducing the effectively accessible surface area for interaction with proteins. In this experiment, the beads were perfused with human whole blood for 2 hours in 1 mL columns at a flow rate of 4 mL/min.

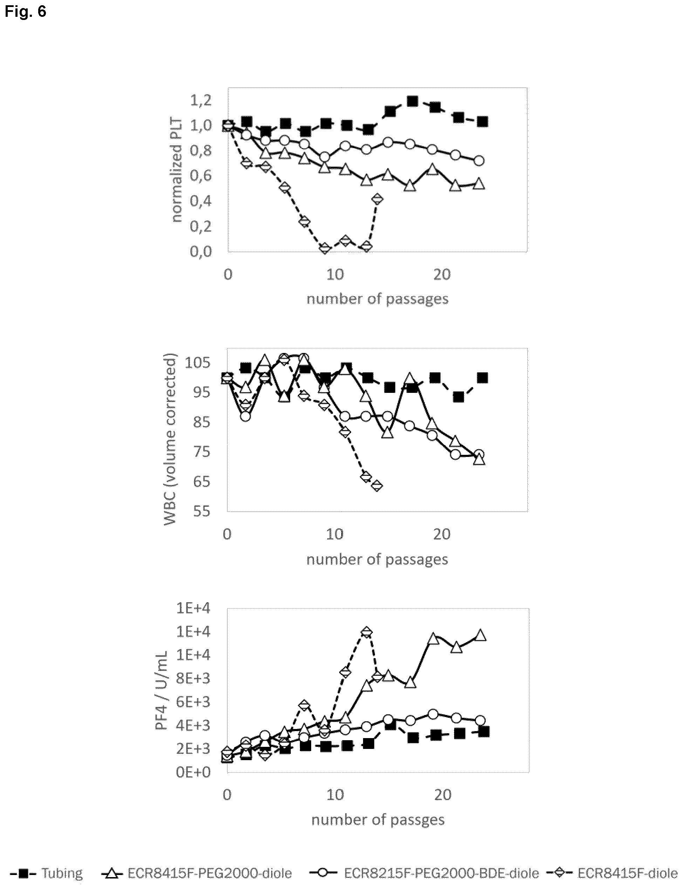

**Figure 6****: Influence of the diameter of the particle.** Finer particles (ECR8415F and ECR8215F d_{Particie} ~ 300 µm [200 - 500 µm]) were employed in this context, undergoing the same PEG2000 coating process as the larger beads (ECR8415M & ECR8215M d_{Particle} ~ 500 µm [200 - 750 µm], see results above). Similarly, a notable enhancement in blood compatibility was observed compared to the non-PEGylated beads. In this experimental setup, the beads were subjected to perfusion with human whole blood for 2 hours within 1 mL columns, operating at a flow rate of 4 mL/min.

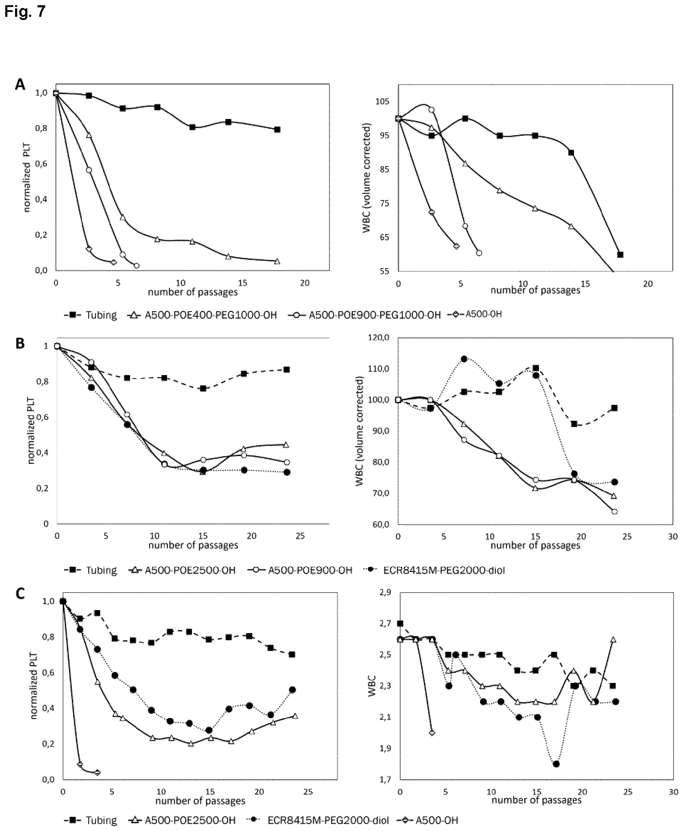

**Figure 7****: Enhancing blood compatibility through PEGylation of various matrices.** Distinct matrices, namely Purolite A500Plus material with PEG of different molecular weights (as indicated in the respective extensions to PEO) grafted thereon, were evaluated to assess hemocompatibility through PEGylation (A) to (C). PEGylation has been shown to enhance the blood compatibility of very hydrophilic PMMA-based beads, such as Purolite ECR beads, and highly hydrophobic DVB crosslinked polystyrene-based beads, like Purolite A-500plus beads. However, in the case of hydrophobic beads, direct grafting of PEG chains may be challenging due to hydrophobic-hydrophilic repulsion. In such instances, a viable approach involves polymerizing a PEG or PEG chain onto the substrate which discrete PEG chains can subsequently be grafted onto, if necessary. Notably, a more significant improvement is typically achieved through the "simple polymerization" of PEO. Processes for grafting of polyethylene oxide onto PS-divinylbenzene are known in the art; methods for grafting PEO on polystyrene-divinylbenzene resins are described, for example, in Lauth et al. (1990) or in US20030087099A1. Alternate materials may serve as a base for these beads, provided that a chemical functionality conducive to PEGylation is sufficiently present on the surface (at a concentration of 0,01 mmol/ml or higher). If needed, existing functionalities can be modified through organic chemistry reactions.

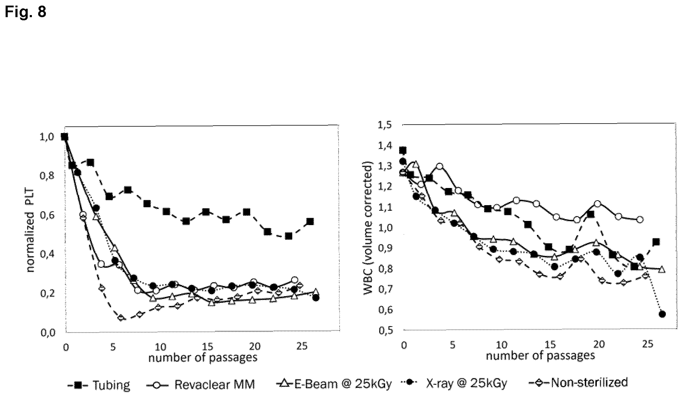

**Figure 8****: Sterilizability of the PEGylated matrix with X-ray and E-beam.** The hemocompatibility of the PEGylated matrix (ECR8415M-Ac2O-PEG2000-diole) is not affected by various radiation sterilization methods, including X-ray and E-beam, all conducted at 25 kGy, and steam sterilization at 120°C for 20 minutes.

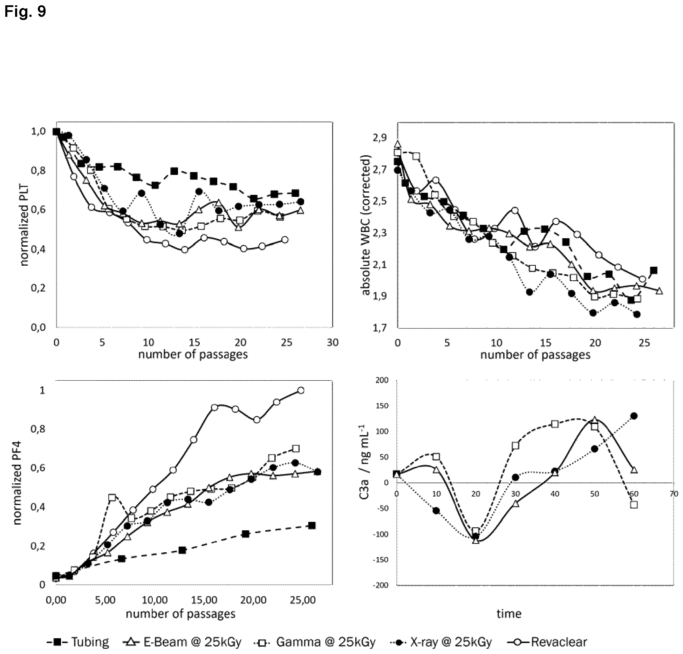

**Figure 9****: Sterilizability of the PEGylated matrix with Gamma, X-ray and E-beam.** The PEGylated matrix (ECR8415M-Ac2O-PEG2000-diole) demonstrates robust compatibility with various radiation sterilization methods, including gamma, X-ray and E-beam, all conducted at 25 kGy, without compromising its blood compatibility.

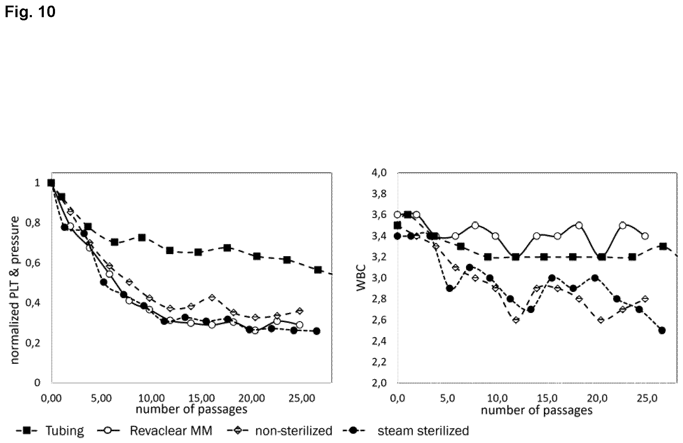

**Figure 10****: Steam sterilization of the PEGylated matrix.** The PEGylated matrix (ECR8415M-Ac2O-PEG2000-diole) maintains blood compatibility when subjected to steam sterilization at 120°C for 20 minutes.

**Figure 11****: Hemocompatibility of the construct.** (A) Illustration depicting the coupling of Protein A to PEGylated beads. (B) Blood compatibility tests comparing protein A-loaded beads (ECR8415MAc2O-PEG2000-Protein A) demonstrate that the bound protein A does not adversely affect blood compatibility.

**Figure 12****: Schematic of PEG-coated solid phase.** Shown is a schematic representation of a substrate (bead) coupled to PEG and said PEG coupled to various ligands according to the present invention. Various coupling chemistries are represented.

### Figure 13: Synthesis of functionalized PEGs for immobilization on epoxy resins.

**Figure 14****: PEGylation of Purolite Epoxy Resin.** Several products were prepared for blood compatibility tests (R1: mPEG750, mPEG2000, mPEG5000; R2: PEG600, PEG2000, PEG3000).

**Figure 15****: Determination of the amine content of the PEGylated resin.** Method of determination shown schematically (quantifiable by UV spectroscopy).

**Figure 16****: Functionalization of the PEGylated Purolite resins.** Provision of the material R3 for further experiments and for initial coupling experiments with model protein or antibody (R3: PEG600, PEG2000, PEG3000).

**Figure 17****: Capping of epoxide end groups (on PEG).** Various options for capping the epoxide end groups of PEG.

**Figure 18****: Pathways to further selective adsorbent materials.** Maleimide end group: established for binding for thiol groups via a thioether bond. Orthopyridyl disulfide end group: "protected thiol" binding of thiols via disulfide bridge. Bromoacetamide/iodoacetamide: Reactive towards strong nucleophiles. Binding of thiols via very stable thioether bond.

**Figure 19****: Synthesis of functionalized PEGs for immobilization on epoxy resins.** (A) Establishment and optimization of the synthesis of PEG thiols. Introduction of thiol groups via amide coupling possible. (B) Efficient synthesis of PEG thiols in two steps using thiourea. Thiol content determined spectroscopically by reaction with Ellmann's reagent (DTNB).

**Figure 20****: PEGylation of Purolite epoxy resin with PEG thiols.** Simple one-step PEGylation. Loading determination by Fmocylation (Fmoc-Leu) via thioester. Significant increase in loading by subsequent reduction of the S-S bridges. Material storable due to OPSS protective group (in the absence of reducing/oxidizing agents).

**Figure 21****: Synthesis of Purolite resin with thiol surface.** Preparation of thiol surface functions by coupling cystamine with subsequent reduction. Determination of loading at the amine and thiol stage is possible (Fmoc-Leu coupling).

**Figure 22****: Synthesis of thiol-selective PEGs for immobilization on thiol resins.** (A) Establishment and optimization of the synthesis of orthopyridyl disulfide (OPSS) PEGs. Synthesis via PEG diamine also possible. Easy to synthesize in three steps via PEG dithiol. (B) Establishment and optimization of the synthesis of maleimido PEGs. Determination of the maleimide content by UV spectroscopy is possible.

**Figure 23****: Synthesis of thiol-selective Purolite Resin.** Binding of the thiol-selective PEGs to the resin surface in just one step.

**Figure 24****: Comparison of PEGylated Beads (ECR8415M-PEG2000) with Protein A loaded PEGylated beads (ECR8415M-PEG2000-ProteinA) in relation to a tubing/system reference.** (A) PLT, C3a & PF4 over the course of ~28 passages (top to bottom). Immobilization of Protein A does not influence any of the shown parameters. (B) PLT, C3a & PF4 over the course of ~28 passages (top to bottom). Sterilization using X-ray radiation at 25 kGy does not influence any of the shown parameters.

**Figure 25****: Schematic illustration of the solid substrate with two layers of PEG.** General schematic illustration of possible configurations of the solid substrate with two layers of PEG. In double PEGylation, the initial PEG layer is notably denser than the PEG layer formed through single PEGylation. Additionally, applying double PEGylation is anticipated to yield a higher density of surface functionalities. The utilization of shorter PEG chains enhances the accessibility of pores, consequently leading to more effective coating of porous beads within the pore volume during the two-step PEGylation process.

**Figure 26****: Hemocompatibility testing of the solid substrate with two layers of PEG.** (A) Illustration depicting the surface functionalization of base beads with multiple PEG layers. (B) Platelet count over time in blood compatibility testing, comparing base beads (ECR8215M) without coating (Revaclear MM) to those coated with PEG200-PEG500-diol, PEG600-PEG1500-diol, PEG600-PEG4000-diol, and PEG2000-diol.

**Figure 27****: PEGylation prevents non-specific protein adsorption of the solid substrate with multiple layers of PEG.** As depicted in the SEM images, the "naked" bead is surrounded by a protein shell (top, left). In contrast, PEG200-PEG500-diol as well as PEG200-PEG500-ProteinA beads exhibit less pronounced adhesion of cellular components and proteins (bottom). To prepare the samples, 1 mL beads were incubated with 3.9 mL human whole blood (1.4 IU/mL heparin) for 2 hours in a 5 mL cryotube on a roller tumbler/rotator. After incubation, the beads were thoroughly washed with 0.9% NaCl and then washed with 1% glutaraldehyde solution and stored in this solution overnight (> 16 h) in the refrigerator (2-8°C). The beads were washed several times with UO water and dried in a freeze-dryer.

**Figure 28****: Hemocompatibility of the construct with the solid substrate with multiple layers of PEG bound to Protein A.** (A) Illustration depicting the double-PEGylated bead coupled to Protein A. (B) Blood compatibility tests comparing protein A-loaded beads (ECR8215M-PEG200-PEG500-ALD-ProteinA) demonstrate that the bound protein A does not adversely affect blood compatibility.

### EXAMPLES

The invention is demonstrated by way of the examples disclosed below. The examples provide technical support and a more detailed description of potentially preferred, non-limiting embodiments of the invention.

### Summary of the Examples

In order to demonstrate the functionality and beneficial properties of the whole blood compatible matrix described herein, the following examples are to be considered:
- Purification of beads before PEGylation.
- Ligand coupling.
- Coupling efficiency and binding capacity.
- Blood and biocompatibility tests.
- Sterilization of the PEGylated matrix.
- Preparation of beads and multiple-layer PEGylation.
- Ligand binding to multi-layer PEGylated beads.
- Blood and biocompatibility tests of multi-layer PEGylated beads.

Examples 1 to 5 represent precursor technologies that represent groundwork for the claimed technology of the present invention. Examples 6 to 8 serve as representative examples of the present invention.

Any of examples 1-5 regarding PEGylation and/or ligand coupling may also represent embodiments of the present invention regarding the double PEGylated beads. For example, any figures, examples or embodiments disclosed herein regarding coupling of PEG to the substrate, may be considered to apply to the present invention with respect to coupling the first PEG layer to the substrate. By way of further example, any figures, examples or embodiments disclosed herein regarding coupling of a ligand to the PEG (of the first or any other layer), may be considered to apply to the present invention with respect to coupling the ligand to the second PEG layer. For example, the coupling chemistries employed in coupling PEG to the substrate apply directly to coupling the first PEG layer to the substrate. For example, the coupling chemistries employed in coupling PEG to a ligand apply directly to coupling the second PEG layer to the ligand.

### Example 1: Preparation of beads and PEGylation

Epoxy methacrylate beads (Lifetech ECR8215M) and amino C6 methacrylate beads (Lifetech ECR8415M) were obtained from Purolite Ltd (Llantrisant, Wales) as suspensions in water (ca. 85% H₂O). The raw material was suspended in deionized water and degassed @ 30 mbar for 30 - 60 min. The degassed beads were washed in a vertical column by flushing with deionized water (bottom to top, 100 mL water for each mL of beads). The flow rate was selected such that the volume of the bead bed was quadrupled. Flushing was performed until no particles d*_{particle} <* 250 nm could be detected using laser diffraction. Epoxide loading was determined by titration (0.7 mmol/g dry material). The amount of surface amines was determined by UV spectroscopy after coupling Fmoc to surface amines and deprotection with a base (0.7 mmol/g dry material). All PEG derivatives were obtained from Rapp Polymere GmbH (Tübingen, Germany) and used as received. 1 ,4-Butanediol diglycidol ether (>93%, TCI, Belgium), NaHCO₃ (Emprove, Merck, Germany), Acetic Anhydride (99+%, Acros Organics, Switzerland), N,N-Diisopropylethylamin (99.5%, Carl Roth GmbH, Germany), Ethanol (Brenntag, Germany), Diethylether (Brenntag, Germany) were used as received.

### ECR8415M-Ac₂O-PEG2000-EP

ECR8415M (100 mL wet beads = 15 g dry material, 0.7 mmol_{NH2}/g_{beads}, 10.5 mmol NH₂) in H₂O was collected on a 500 mL filter funnel and washed twice with 200 mL 10 mM NaHCO₃. The wet material was transferred into a 1 L flask containing 44.7 g EP-PEG2000-EP (2126 g/mol, 21 mmol, 2 equiv.) with 135 mL 10 mM NaHCO₃ (3 mL/g_{PEG}). The flask was placed in a preheated 27 °C rotary evaporator. The suspension was mixed until all PEG dissolved (ca. 10 min). Then, the mixture was degassed three times. The reaction mixture was left rotating for 15 h. After this time, the beads were filtered off by vacuum filtration and washed 10 times with 100 mL of deionized water. Subsequently, the beads were washed three times with 100 mL ethanol and finally twice with 100 mL diethyl ether. The beads were briefly dried on the filter funnel by applying reduced pressure. The material was transferred back into a 1 L flask and suspended in 100 mL acetic anhydride. The suspension was placed back onto the rotary evaporator and degassed three times. The reaction was left rotating at 27 °C for 4 h. Then, the beads were filtered off on a glass funnel filter and washed thoroughly: 10 times with 100 mL ethanol and twice with 100 mL 20% ethanol/water. The capping of residual amines was tested using the ninhydrin test. The product was stored as a suspension in 20% ethanol/water.

### ECR8215M-PEG2000-BDE-EP

ECR8215M (100 mL wet beads = 15 g dry material, 0.7 mmol_{epoxide}/g_{beads}, 10.5 mmol epoxide) in H₂O, was collected on a 500 mL filter funnel and washed twice with 200 mL 10 mM NaHCO₃. The wet material was transferred into a 1 L flask containing 41.3 g NH₂-PEG2000-NH₂ (1967 g/mol, 21 mmol, 2 equiv.) with 125 mL 10 mM NaHCO₃ (3 mL/g_{PEG}). The flask was placed in a preheated 27 °C rotary evaporator. The suspension was mixed until all PEG dissolved (ca. 10 min). Then, the mixture was degassed three times. The reaction mixture was left rotating for 48 h. After this time, the beads were filtered off by vacuum filtration and washed 10 times with 100 mL of deionized water. Subsequently, the beads were washed with three times with 100 mL ethanol and finally twice with 100 mL diethyl ether. The beads were briefly dried on the filter funnel by applying reduced pressure. The material was transferred back into a 1 L flask and suspended in 100 mL dry THF. 2.7 g DIPEA (129 g/mol, 21 mmol, 2 equiv.) and 85 g 1,4-butanediol diglycidyl ether (202 g/mol, 420 mmol, 20 equiv.) were added to the suspension. The suspension was placed back onto the rotary evaporator and was degassed three times. The reaction mixture was left rotating at 40 °C overnight. The beads were filtered off on a glass funnel filter and washed thoroughly: 10 times with 100 mL ethanol and twice with 100 mL 20% ethanol/water. The capping of residual amines was ca. 95% complete according to the ninhydrin test. The final product was stored as a suspension in 20% ethanol/water.

Additional PEGylated beads were manufactured, as outlined briefly below.

### Summary ECR8215M (epoxy) beads:

PEGs of different lengths capped with methoxy groups (OMe) were attached to substrates. Qualitative detection of PEGylation, partially determinable by NMR spectroscopy, was carried out, and blood compatibility tests (described below) were carried out to determine optimal chain length.

The following examples were produced:
- MeO-PEG750-NH₂
- MeO-PEG2000-NH₂
- MeO-PEG5000-NH₂

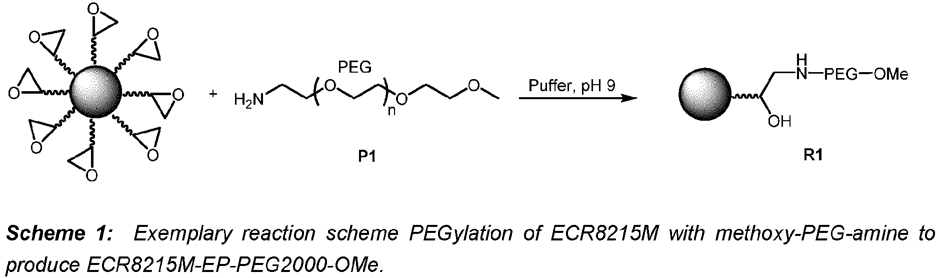

Coupling of PEG diamines of different chain lengths and qualitative and quantitative spectroscopic determination of the amine groups was also carried out.
- NH₂-PEG600-NH₂
- NH₂-PEG2000-NH₂
- NH₂-PEG3000-NH₂

ECR8215M-EP-PEG2000-NH₂ was further reacted with butanediol diglycidyl ether to reintroduce an epoxy function. This material was also subjected to further functional testing. The material was reacted with glutaric anhydride to introduce carboxylic acid groups. The material was also reacted with glutardialdehyde to introduce aldehyde groups.

Coupling of PEG dithiols and qualitative and quantitative spectroscopic determination of the thiol groups was also carried out. SH-PEG2000-SH was coupled. The material was reacted with butanediol diglycidyl ether (BDE) analogous to ECR8215M-EP-PEG2000-NH₂ in order to introduce an epoxy function.

### Transformation of the surface epoxies

ECR8215M beads were reacted with cystamine (2,2-dithiobisethanamine) for the introduction of thiol-selective PEG-dimaleimides. The disulfide bridges were then reductively cleaved to obtain free thiols on the bead surface (Scheme 2).

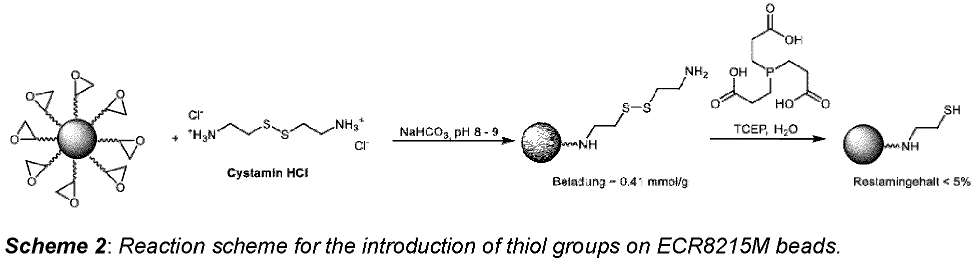

Binding of thiol-selective PEG dimaleimides was successful. Binding of thiol-selective PEG dithiols protected with orthopyridyl sulfide was also completed.

### Further coupling reactions:

In principle, the ECR8215M epoxy beads are PEGylatable with PEGs that carry nucleophilic end groups. Here, amines and thiols are reacted under basic conditions. Alcohols, for example, are also envisaged. Alternatively, the epoxide group can be converted into another functional group using linker molecules. Here, for example, a thiol (through cystamine) can couple to thiol-selective PEGs or amines through hexamethylenediamine, to couple amine-selective PEGs. After PEGylation, the remaining terminal functional groups of the PEG can be used to link to the ligand or can be further modified. In the case of the amine, for example, there are many available linkers that effectively convert the amine group into another functional group to link thiols or other amines to it. The same approach can also be achieved with heterobifunctional PEGs. For example:
NH₂-PEG-N₃ for the generation of azide groups for click chemistry.
NH₂-PEG-alkyne for the generation of alkyne groups for click chemistry.
NH₂-PEG-COOH to produce carboxylic acid group.
NH₂-PEG-S-Trt to produce a protected thiol.
SH-PEG-NH-Boc to produce a protected amine.
NH₂-PEG-Biotin to generate a biotinylated surface for binding of ligands by biotin/streptavidin affinity.
NH₂-PEG-Maleimide for the generation of maleimide functions for selective binding of thiols.

### Summary ECR8415M (Amino) Beads:

Binding of PEG-diepoxides (epoxy = EP) was carried out for coupling ligands with nucleophilic end groups (e.g., thiols, amines)

### EP-PEG2000-EP was generated for further testing.

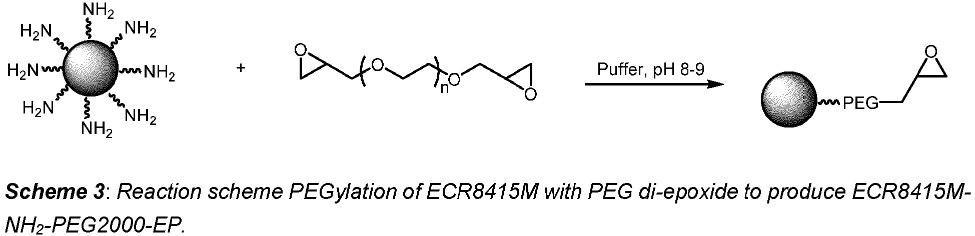

Coupling of PEG-disquaric acid ethylester for the highly selective attachment of ligands bearing primary amine end groups was carried out. SQA-PEG2000-SQA (SQA = squaric acid ethyl ester) was generated for further testing.

Coupling of PEG activated diesters (e.g. NHS esters or tetrafluorophenyl esters) was carried out for the attachment of ligands bearing primary amine end groups. TFP-PEG2000-TFP (TFP = tetrafluorophenyl ester of PEG acetic acid) was generated.

Coupling of PEG dibromoacetamides was carried out for attachment of ligands bearing primary amine or thiol end groups. AcBr-PEG2000-AcBr (AcBr = bromoacetamide) was generated.

### Further reactions:

The same principle applies as for epoxy beads (ECR8415M). Surface amino groups can first be converted into other end groups by direct chemical conversion or by linkers in order to then couple a corresponding PEG to them, or they can be PEGylated with correspondingly functionalized PEGs directly. In addition to squaric acid esters or other active esters, PEG aldehydes, for example, would also be suitable. In addition to the described bromoacetamide PEG derivative for the attachment of ligands bearing nucleophilic end groups, the more reactive iodoacetamide PEG derivative or the corresponding acetate are also envisaged. Analogous to the ECR8215M beads, heterobifunctional PEGs would simplify or shorten the production of the material. Examples of NHS ester PEGs:
NHS-PEG-S-Trt to produce protected thiols.
NHS-PEG-NH-Fmoc to produce protected amine.
NHS-PEG-Alkyne to produce alkynes for click chemistry.

Additional coupling reactions, for PEG to bead coupling, and PEG to ligand coupling, are provided by way of schematics in **Figures 13-23****.**

### Example 2: Ligand coupling

The coupling of ligands to the PEGylated bead surface occurred through covalent bonding. In this process, a variety of different reactions were possible. Examples are provided in "Bioconjugate Techniques, Third Edition. Chapter 2, Functional Targets for Bioconjugation" and "Bioconjugate Techniques, Third Edition. Chapter 3, The Reactions of Bioconjugation".

By way of example, the attachment of Protein A (ProSpec) to the PEGylated beads with terminal aldehyde functionalities (ECR8415M-Ac₂O-PEG2000-ALD) is described. Additionally, carboxy-terminated beads (ECR8415M-Ac2O-PEG2000-COOH) can be coupled through EDC-mediated coupling with amino-functionalized ligands. Ligands featuring S-S bridges can also be directly coupled to Epoxy-functionalized beads (ECR8415M-Ac2O-PEG2000-EP) by reducing the S-S bridges to the corresponding thiol using TCEP during the coupling process.

ECR8415M-Ac₂O-PEG2000-ALD was prepared by hydrolysis and subsequent oxidation of ECR8415M-Ac₂O-PEG2000-EP according to the following protocol: 25 mL of ECR8415M-AczO-PEG2000-EP were washed several times with deionized water and transferred to a 50 mL PP Falcon Tube. Subsequently, the deionized water was exchanged three times for 0.1 N NaOH (PanReac AppliChem ITW Chemicals), and the tube was placed on a roller tumbler for > 16 h at room temperature. After the reaction, the beads were washed several times with deionized water until the pH value of the wash water was neutral. The resulting diol-functionalized beads were designated ECR8415M-Ac₂O-PEG2000-(diol). 10 mL of ECR8415M-Ac₂C-PEG2000-(Diol) beads were measured into a 20 mL tube (made of PP), mixed with 10 mL of a 0.2 M Na(IO₄) (Sigma-Aldrich) solution, and then allowed to react for 15 min on a roller tumbler at room temperature. The oxidized beads were washed several times with MilliQ water; qualitative proof of the oxidation was obtained by purple staining of the beads after adding Schiffs reagent. The oxidized beads produced in this way were designated ECR8415M-Ac₂O-PEG2000-ALD. The attachment of Protein A was carried out via reductive amination. For this purpose, 2 mL of ECR8415M-Ac₂O-PEG2000-ALD were washed with 1X PBS @ pH 7.4, transferred to a 15 mL Falcon tube, and the supernatant was drawn off to the top of the beads. 8 mL of a solution of Protein A in 1X PBS @ pH 7.4 (5 mg_{ProteinA}/mL_{PBS}) was added, and the suspension was mixed for 20 min on a roller tumbler. Then, 0.1 mL of a 5 mol/L Na(CN)BH₃ in 1 mol/L NaOH solution (Sigma-Aldrich) and 0.1 mL of 1N HCl (Sigma-Aldrich) were added. The beads suspended in the reaction solution were placed on a roller tumbler for > 4 h at room temperature. After completion of the reaction, the beads were washed several times with 1 x PBS @pH 7.4. Any remaining unreacted aldehydes were saturated with ethanolamine. For this purpose, the washed beads (2 mL) were mixed with 10 mL 1 M ethanolamine solution (Merck) pH 9 and placed on a roller tumbler for 20 min. Subsequently, 0.1 mL 5mol/L Na(CN)BH₃ in 1 mol/L NaOH and 0.1 mL 1 N HCl were added, and the reaction was carried out for a further 4 h on the roller tumbler. After completion of saturation, the beads were washed several times with 1 M NaCl (ThermoScientific) solution followed by washing with 1 x PBS@ pH 7.4.

### Example 3: Coupling efficiency and binding capacity

An IgG-binding ligand (Ligand 1 or L_1) was coupled to functionalized beads at a concentration of 40 mg/mL beads according to coupling procedures well-established in the literature. This procedure allowed for the creation of the following series of beads:
- ECR8215M-L_1
- ECR8215M-Ac2G-PEG200-PEG500-L_1
- ECR8415M-Ac2O-PEG2000-L_1
- ECR8204-L_1
- IB-COV6-L_1
- IB-COV6-Ac2O-PEG200-PEG500-L_1

The adsorption capacity of IgG was assessed for all types of beads by incubating 0.5 mL of each bead type in a 4 mL solution containing 2 mg/mL of human IgG in 1x PBS buffer at pH 7.4 for a duration of 2 hours. A correlation between the pore size of the beads and the IgG binding capacity was observed through this process.

**Table 1. Correlation between bead pore size and IgG binding capacity.**

| **Type of beads with 40 mg_{L_1} mL_{Beads}⁻¹** | **Binding capacity** / **mg_{IgG} mL_{Beads}⁻¹** | **Theoretical pore size** |
|---|---|---|
| ECR8215M-L_1 | 12,92 | 150 nm |
| ECR8215M-Ac2G-PEG200-PEG500-L_1 | 12,43 | 120 nm |
| ECR8415M-Ac2O-PEG2000-L_1 | 8,97 | 90 nm |
| IB-COV6-L_1 | 5,86 | 80 nm |
| ECR8204-L_1 | 3,76 | 40 nm |
| IB-COV6-Ac2O-PEG200-PEG500-L_1 | 1,97 | 50 nm |

Protein A-loaded beads with concentrations 20 mg ProteinA/mL beads and 40 mg ProteinA/mL beads were employed. The binding capacity of these beads for human IgG (Sigma Aldrich) was evaluated by incubating 0.5 mL of the beads in 4 mL of a solution containing 2 mg IgG/mL in 1x PBS (for a duration of 2 hours). Beads without protein A were utilized for comparison purposes. The results are summarized in the following table:

**Table 2. Binding capacity of Protein A-loaded beads.**

| **Type of beads** | **Loading in mg_{proteinA} mL_{Beads}⁻¹** | **Binding capacity / mg_{IgG} mL_{Beads}⁻¹** |
|---|---|---|
| ECR8415M-Ac₂O-PEG2000-ALD | 20 | n/a |
| ECR8415M-Ac₂O-PEG2000-ALD | 40 | n/a |
| ECR8415M-Ac₂O-PEG2000-ProteinA | 20 | 1,86 |
| ECR8415M-Ac₂O-PEG2000-ProteinA | 40 | 4,84 |
| ECR8415M-Ac₂O-PEG2000 | n/a | n/a |

### Example 4: Blood and biocompatibility tests

Blood and biocompatibility tests were performed in a dynamic setup. For these tests, the investigated beads were placed in cylindrical columns made of polycarbonate and/or polyvinyl chloride with 1- or 5-mL volumes. These columns were perfused in a test stand with 23 or 55 mL of anticoagulated heparinized (1.3 - 1.5 IU/mL) human whole blood tempered to 37 °C, to which CaCl₂ (free Ca²⁺ 1,2 - 1,4 mM/L) were added at the beginning of the experiment (t = 0). To determine the system's influence, one station was operated without a column in each experiment (perfusion of the tubing set used); a miniaturized Revaclear dialyzer was used as an additional reference to the 5 mL columns. Blood samples (0.9 mL each) were taken at predetermined intervals (every 10 - 20 min) and anticoagulated with EDTA (0.1 mol/L, 100 µL) or CTAD () to yield C(Ca2+) < 0,4 mM/L. Subsequently, hematological parameters (PLT, WBC, HCT) were measured directly on a Sysmex XP-300. The remaining blood was centrifuged at 4 °C @2000 rpm for 30 minutes, and the separated plasma was frozen at -70 °C. Further relevant parameters (PF4, C3a, TAT) were determined from this plasma using ELISAs. Differences in blood compatibility were evaluated based on the magnitude of the decrease in platelet count (PLT) and white blood cell count (WBC) as well as the increase in platelet factor 4 (PF4) and thrombin-antithrombin III complex (TAT). In addition, biocompatibility was assessed based on the activation of the complement system by determining the concentration of the central complement factor C3a.

### Example 5: Sterilization of PEGylated matrix

The PEGylated matrix demonstrates robust compatibility with various radiation sterilization methods, including X-ray and E-beam, all conducted at 25 kGy, without compromising its blood compatibility. Furthermore, radiation sterilization does not yield cytotoxic byproducts, as evidenced by the ICG results detailed in Table 3.

**Table 3. Comparison of distinct radiation sterilization methods with respect to inhibition of cell growth (in %).**

| | Aqueous supernatant | Aq. Washed | MEM washed |
|---|---|---|---|
| Gamma | 27 | 13 | 13 |
| X-ray | 8 | 1 | 11 |
| E-beam | 7 | 5 | 7 |

The PEGylated matrix maintains blood compatibility when subjected to steam sterilization at 120 °C for 20 minutes. Notably, steam sterilization does not generate cytotoxic substances, as indicated by the ICG results presented in Table 4.

**Table 4. Results for steam sterilization with respect to inhibition of cell growth.**

| | Aqueous supernatant | Aq. Washed | MEM washed |
|---|---|---|---|
| Steam | 7 | 1 | 6 |

The following examples are representative of the present invention as claimed and show the preparation of beads with multi-layer PEGylation, ligand binding to multi-layer PEGylated beads, and blood and biocompatibility tests of multi-layer PEGylated beads.

### Example 6: Preparation of beads and multiple-layer PEGylation

The preparation of beads with multiple-layer PEGylation was demonstrated by way of example using the method as follows:
Epoxy methacrylate beads (Lifetech ECR8215M & ChiralVision IB-COV-8) and amino C6 methacrylate beads (Lifetech ECR8415M) were obtained from Purolite Ltd (Llantrisant, Wales) as suspensions in water (ca. 85% H₂O) and epoxy hydroxethyl methacrylate beads (ChiralVision IB-COV-6) were obtained from ChiralVision (Den Hoorn, The Netherlands) as suspensions in water (ca. 70% H₂O). The raw material was suspended in deionized water and degassed @ 30 mbar for 30 - 60 mi. The degassed beads were washed in a vertical column by flushing with deionized water (bottom to top, 100 mL water for each mL of beads). The flow rate was selected such that the volume of the bead bed was quadrupled. Flushing was performed until no particles d_{particle} < 250 nm could be detected using laser diffraction. The amount of surface amines was determined by UV spectroscopy after coupling Fmoc to surface amines and deprotection with a base (0.7 mmol/g dry material). Amine PEG derivatives NH₂-PEG-NH₂ (200 g/mol, 600 g/mol) and Epoxide PEG derivatives EP-PEG-EP (500 g/mol, 4000 g/mol) were obtained from Rapp Polymere GmbH (Tübingen, Germany) and used as received. 1,4-Butanediol diglycidol ether (>93%, TCI, Belgium), NaHCO₃ (Emprove, Merck, Germany), Acetic Anhydride (99+%, Acros Organics, Switzerland), N,N-Diisopropylethylamin (99.5%, Carl Roth GmbH, Germany), Ethanol (Brenntag, Germany), Diethylether (Brenntag, Germany) were used as received.

### ECR8215M-PEG200-Ac₂O-PEG500-EP

ECR8215M (110 mL wet beads = 17 g dry material, 0.7 mmol/g Epoxide, 12 mmol) suspended in H2O was collected on a 500 mL filter funnel and washed twice with 200 mL 10 mM NaHCO₃. The wet material was transferred into a 1 L flask with 70 mL 10 mM NaHCO₃ solution (4 mL/gᵣₑₛᵢₙ) containing 13 g NH₂-PEG200-NH₂ (200 g/mol, 60 mmol, 5 equiv.) and 35 mL 10 mM NaHCO₃ (3 mL/g_{PEG}). The flask was placed in a preheated (27 °C) rotary evaporator. The suspension was briefly mixed. Then, the mixture was degassed three times. The reaction mixture was left rotating for 60 h. Then, the beads were filtered off by vacuum filtration and washed 10 times with 100 mL deionized water to remove excess PEG amines and three additional times with 100 mL 10 mM NaHCO₃ solution. The resulting material was transferred back into the flask with 70 mL 10 mM NaHCO₃ (4 mL/gᵣₑₛᵢₙ) and 30 g EP-PEG500-EP (500 g/mol, 60 mmol, 5 equiv.) in 90 mL 10 mM NaHCO₃ solution (3 mL/g_{PEG}) was added. The flask was placed in a preheated (27 °C) rotary evaporator. The suspension was briefly mixed. Then, the mixture was degassed three times. The reaction mixture was left rotating for 24 h. Subsequently, the beads were filtered off by vacuum filtration and washed 10 times with 100 mL deionized water to remove excess PEG, then three times with 100 mL ethanol, and finally two times with 100 mL diethyl ether. The beads were briefly dried on the filter funnel by applying reduced pressure. The material was transferred back into a 1 L flask and suspended in 100 mL acetic anhydride. The suspension was placed onto a rotary evaporator and degassed three times. The reaction was left rotating at 27 °C for 4 h. Then, the beads were filtered off on a glass funnel filter and thoroughly washed 10 times with 100 mL ethanol and two times with 100 mL 20% ethanol/water. The capping of residual amines was confirmed using the ninhydrin test. The product was stored as a suspension in 20% ethanol/water.

### ECR8215M-PEG600-Ac₂O-PEG4000-EP

ECR8215M (150 mL wet beads = 24 g dry material, 0.7 mmol/g Epoxide, 17 mmol) suspended in H2O was collected on a 500 mL filter funnel and washed twice with 250 mL 10 mM NaHCO₃. The wet material was transferred into a 1 L flask with 100 mL 10 mM NaHCO3 solution (4 mL/gᵣₑₛᵢₙ) containing 50 g NH₂-PEG600-NH₂ (600 g/mol, 85 mmol, 5 equiv.) and 150 mL 10 mM NaHCO₃ (3 mL/g_{PEG}). The flask was placed in a preheated (27 °C) rotary evaporator. The suspension was briefly mixed. Then, the mixture was degassed three times. The reaction mixture was left rotating for 60 h. Then, the beads were filtered off by vacuum filtration and washed 10 times with 150 mL deionized water to remove excess PEG amines and three additional times with 150 mL 10 mM NaHCO₃ solution. The resulting material was transferred back into the flask with 100 mL 10 mM NaHCO₃ (4 mL/gᵣₑₛᵢₙ) 135 g EP-PEG4000-EP (4000 g/mol, 34 mmol, 2 equiv.) in 400 mL 10 mM NaHCO₃ solution (3 mL/g_{PEG}) was added. The flask was placed in a preheated (27 °C) rotary evaporator. The suspension was briefly mixed. Then, the mixture was degassed three times. The reaction mixture was left rotating for 24 h. Subsequently, the beads were filtered off by vacuum filtration and washed 10 times with 150 mL deionized water to remove excess PEG, then three times with 150 mL ethanol, and finally two times with 150 mL diethyl ether. The beads were briefly dried on the filter funnel by applying reduced pressure. The material was transferred back into a 1 L flask and suspended in 150 mL acetic anhydride. The suspension was placed onto a rotary evaporator and degassed three times. The reaction was left rotating at 27 °C for 4 h. Then, the beads were filtered off on a glass funnel filter and thoroughly washed 10 times with 150 mL ethanol and two times with 150 mL 20% ethanol/water. The capping of residual amines was confirmed using the ninhydrin test. The product was stored as a suspension in 20% ethanol/water.

### Example 7: Ligand binding on multiple-layer PEGylated beads

The coupling of ligands to the PEGylated bead surface occurred through covalent bonding.

In this process, a variety of different reactions were possible. Examples are provided in "Bioconjugate Techniques, Third Edition. Chapter 2, Functional Targets for Bioconjugation" and "Bioconjugate Techniques, Third Edition. Chapter 3, The Reactions of Bioconjugation".

By way of example, the attachment of Protein A (ProSpec) to the PEGylated beads with terminal aldehyde functionalities (ECR8215M-PEG200-Ac₂O-PEG500-ALD) is described. ECR8215M-PEG200-Ac₂O-PEG500-ALD was prepared by hydrolyzing and subsequently oxidizing ECR8215M-PEG200-Ac₂O-PEG500-EP according to the following protocol:
25 mL of ECR8215M-PEG200-Ac₂O-PEG500-EP was washed several times with UO water and transferred to a 50 mL PP Falcon Tube. The UO water was then exchanged three times for 0.1 N NaOH (PanReac AppliChem ITW Chemicals), and the tube was placed on a roller tumbler for > 16 hours at room temperature. After the reaction, the beads were washed several times with UO water until the pH value of the wash water was neutral. The resulting diol-functionalized beads were designated ECR8215M-PEG200-Ac₂O-PEG500-(diol). 10 mL of ECR8215M-PEG200-Ac₂O-PEG500-(Diol) beads were measured into a 20 mL tube (made of PP), mixed with 10 mL of a 0.2 M Na(IO₄) (SigmaAldrich) solution, and then allowed to react for 15 minutes on a roller tumbler at room temperature. The oxidized beads were washed several times with MilliQ water; qualitative proof of the oxidation was obtained by staining the beads purple after adding Schiffs reagent. The oxidized beads produced in this way were designated ECR8215M-PEG200-Ac₂O-PEG500-ALD. Protein A was bound via reductive amination. For this purpose, 2 mL of ECR8215M-PEG200-Ac₂O-PEG500-ALD was washed with 1X PBS @ pH 7.4, transferred to a 15 mL Falcon tube, and the supernatant was drawn off the top of the beads. 8 mL of a solution of Protein A in 1X PBS @ pH 7.4 (5 mg_{ProteinA}/mL_{PBs}) was added, and the suspension was mixed for 20 minutes on a roller tumbler. Then, 0.1 mL of a 5 mol/L Na(CN)BH₃ in 1 mol/L NaOH solution (SigmaAldrich) and 0.1 mL of 1N HCl (SigmaAldrich) were added. The beads suspended in the reaction solution were placed on a roller tumbler for a further > 16 hours at room temperature. After completion of the reaction, the beads were washed several times with 1X PBS @pH 7.4. Any remaining unreacted aldehydes were saturated with ethanolamine. For this purpose, the washed beads (2 mL) were mixed with 10 mL 1 M ethanolamine solution (Merck) pH 9 and placed on a roller tumbler for 20 minutes. Subsequently, 0.1 mL 5 mol/L Na(CN)BH₃ in 1 mol/L NaOH and 0.1 mL 1 N HCl were added, and the reaction was carried out for a further 4 hours on the roller tumble. After completion of the saturation, the beads were washed several times with 1 M NaCl (ThermoScientific) solution.

Protein A-loaded beads with 20 mg ProteinA/mL beads were thus prepared and analyzed. The binding capacity of these beads for human IgG (SigmaAldrich) was determined by incubating 0.5 mL beads in 4 mL of a 2 mg_{IgG}/mL_{PBS} solution (for 2 hours). For comparison, beads without protein A were used, which showed no adsorption of protein A. The results are shown in the following table. It is clear that the double PEGylation enables a significantly higher binding capacity for IgG, which can be explained by the larger pore volume available.

**Table: Coupling efficiency and binding capacity**

| | **Beads-PEG2000-Protein A** | **Beads-PEG2000-Protein A** | **Beads-PEG200-PEG500-Protein A** |
|---|---|---|---|
| **Loading** / **mg_{ProteinA}mL**⁻¹**_{Beads}** | 20 | 40 | 20 |
| **Binding Capacity** / **mg_{IgG}mL⁻¹_{Beads}** | 2 | 4,9 | 11,9 |

### Example 8: Blood and biocompatibility tests of multi-layer PEGylated beads

Blood and biocompatibility tests were carried out in a dynamic setup. The investigated beads are available in cylindrical columns made of polycarbonate or polyvinyl chloride with a 1- or 5 mL volume. These columns were perfused in a test stand with 23 or 55 mL anticoagulated heparinized (1.3 - 1.5 IU/mL) human whole blood tempered to 37 °C, to which CaCl₂ (free Ca²⁺ 1,2 - 1,4 mM/L) were added at the beginning of the experiment (t = 0).- To determine the system influence, one station was operated without a column in each experiment (perfusion of the tubing set used); a miniaturized Revaclear dialyzer was used as an additional reference to the 5 mL columns. Blood samples (0.9 mL each) were taken at predetermined intervals (every 10 -20 min) and anticoagulated with EDTA (0.1 mol/L, 100 µL). Hematological parameters (PLT, WBC, HCT) were then measured directly on a Sysmex XP-300. The remaining blood was centrifuged at 4 °C @2000 rpm for 30 minutes, and the separated plasma was frozen at -70 °C. Further relevant parameters (PF4, TAT, C3a) were determined from this plasma using ELISA.

The blood compatibility of porous beads is increased by a two-stage PEG coating of the bead.

Differences in blood compatibility were evaluated based on the magnitude of the decrease in platelet count (PLT) and white blood cell count (WBC) as well as the increase in platelet factor 4 (PF4) and thrombin-antithrombin III complex (TAT). In addition, biocompatibility was assessed based on the activation of the complement system by determining the concentration of the central complement factor C3a.

Schematics of double PEGylation are shown in Figure 25. Double PEGylation enables a higher PEG density on the bead surface compared to single PEGylation. Figure 25 also shows that the first PEG layer in double PEGylation is denser than the PEG layer achieved by single PEGylation. This can be explained, in embodiments, by the shorter length of the first PEG in the double PEGylation. Furthermore, a higher density of surface functionalities can be obtained by double PEGylation. Furthermore, in embodiments, the use of shorter PEG chains results in better accessibility of pores and thus also a better coating of porous beads in the pore volume when using two-step PEGylation.

As is shown in Figure 26, an increased blood compatibility through double PEGylation can be obtained.

The examples show that two-step PEGylation ("double PEGylation") leads to a greater increase in blood compatibility compared to single PEGylation (PEGylation in one step) with the same total molecular weight. The capping of the functionalities of the base bead (hydrolysis of epoxides) or non-reacted functionalities of the first PEG layer (e.g. acetylation of amines) has a strong influence on blood compatibility. The hydrolysis of epoxides could be achieved by reaction with 1M ethanolamine (pH 9, reaction time > 16 h), acetylation by reaction with acetic anhydride (reaction time > 6 h). It was shown that combinations of different PEG chain lengths are possible and that the total length/total molecular weight correlates to a certain extent with the blood compatibility of the materials produced.

As can be seen from Figure 27, double PEGylation enables the prevention of non-specific protein accumulation.

In the SEM images shown in Figure 27, the "naked" bead (upper-left) is heavily covered with adhering cells and protein deposits, whereas the beads coated with dPEG200+500 (bottom left) and beads coated with dPEG200+500 and loaded with protein A (bottom right) show a much less pronounced deposition of cellular components and proteins.

As can be seen in Figure 28, comparative blood compatibility tests on protein A loaded beads (ECR8415M-Ac2O-PEG200-PEG500-Protein A) show that blood compatibility is not affected by binding protein A.

### REFERENCES

1. Sanchez AP, Cunard R, Ward DM. The selective therapeutic apheresis procedures. J Clin Apher. 2013 Feb;28(1):20-9. doi: 10.1002/jca.21265. PMID: 23420592
2. Liu X , Yuan L , Li D , Tang Z , Wang Y , Chen G , Chen H , Brash JL . Blood compatible materials: state of the art. J Mater Chem B. 2014 Sep 21;2(35):5718-5738. doi: 10.1039/c4tb00881b. Epub 2014 Aug 1. PMID: 32262016.
3. Ekdahl KN, Fromell K, Mannes M, Grinnemo KH, Huber-Lang M, Teramura Y, Nilsson B. Therapeutic regulation of complement activation in extracorporeal circuits and intravascular treatments with special reference to the alternative pathway amplification loop. Immunol Rev. 2023 Jan;313(1):91-103. doi: 10.1111/imr.13148. Epub 2022 Oct 18. PMID: 36258635; PMCID: PMC10092679.
4. Bacal, C. J. O., Maina, J. W., Nandurkar, H. H., Khaleel, M., Guijt, R., Chang, Y., Dwyer, K. M., & Dumée, L. F. (2021). Blood apheresis technologies-a critical review on challenges towards efficient blood separation and treatment. Materials Advances, 2(22), 7210-7236. https://doi.org/10.1039/d1ma00859e
5. Vaganov AA, Taranushenko TE, Luzan NA, Shchugoreva IA, Kolovskaya OS, Artyushenko PV, Zamay TN, Kichkailo AS. Aptamers Regulating the Hemostasis System. Molecules. 2022 Dec 6;27(23):8593. doi: 10.3390/molecules27238593. PMID: 36500686; PMCID: PMC9739204.
6. Kovach KM, Capadona JR, Gupta AS, Potkay JA. 2014. The effects of PEG-based surface modification of PDMS microchannels on long-term hemocompatibility. J Biomed Mater Res Part A 2014:102A:4195-4205.
7. Jianhua Lv, Jing Jin, Yuanyuan Han & Wei Jiang (2019): Effect of end-grafted PEG conformation on the hemocompatibility of poly(styrene-b-(ethylene-co-butylene)-b-styrene), Journal of Biomaterials Science, Polymer Edition, DOI: 10.1080/09205063.2019.1657621.
8. M. Lauth, Y. Frère, B. Meurer, Ph. Gramain, M. Prévost (1990): Polyethylene oxide) grafted onto polystyrene-divinylbenzene macroporous resins. Covalent binding of mercuric chloride, Reactive Polymers, Volume 12, Issue 2.

## Claims

1. A whole blood compatible matrix for coupling a ligand and use in extracorporeal blood treatment, said matrix comprising a solid substrate, said substrate comprising a first layer of polyethylene glycol (PEG) and at least a second layer of polyethylene glycol,
wherein said at least second layer is positioned outside the first layer and is bound to said first layer, and wherein the polyethylene glycol of the at least second layer comprises a terminal functional group for coupling said ligand to said polyethylene glycol.

2. The matrix according to the preceding claim, wherein the solid substrate comprises particles, wherein said particles have a diameter of about 100 µm to about 1000 µm and an average pore size of about 20 nm to about 300 nm.

3. The matrix according to any one of the preceding claims, wherein the polyethylene glycol of the first layer has a molecular weight of about 100 to 7000 Daltons, preferably 100 to 5000 Daltons, more preferably 100 to 1000 Daltons, and most preferably 100 to 500 Daltons, and wherein the polyethylene glycol of the at least second layer has a molecular weight of about 100 to 7000 Daltons, preferably 200 to 3000 Daltons, more preferably 200 to 1000 Daltons, and especially preferably from 300 to 800 Daltons.

4. The matrix according to any one of the preceding claims, wherein the solid substrate is hydrophilic or hydrophobic, and comprises a synthetic polymer, a natural polymer, and/or inorganic material.

5. The matrix according to any one of the preceding claims, wherein the solid substrate is a porous synthetic polymer particle, preferably comprising a methacrylate polymer.

6. The matrix according to any one of the preceding claims, wherein the particles have a diameter of about 200 µm to 800 µm and the average pore size of the solid substrate is about 40 to 500 nm, preferably, 50 to 300 nm.

7. The matrix according to any one of the preceding claims, wherein the solid substrate comprises a synthetic methacrylate polymer particle covalently coupled to polyethylene glycols of the first layer with an average molecular weight of about 100 to 1000 Daltons and the polyethylene glycol of the at least second layer is coupled to the polyethylene glycol of the first layer and has an average molecular weight of about 300 to 2000 Daltons.

8. The matrix according to any one of the preceding claims, wherein the solid substrate comprises a first layer of in essence a single polyethylene glycol molecular weight form, such as polyethylene glycol having an average molecular weight of 100 Daltons, 200 Daltons, 300 Daltons, 400 Daltons, 500 Daltons, 600 Daltons, 700 Daltons, 750 Daltons, 900 Daltons, 1000 Daltons or 2000 Daltons, and at least a second layer of in essence a single polyethylene glycol molecular weight form, such as polyethylene glycol having an average molecular weight of 100 Daltons, 200 Daltons, 300 Daltons, 400 Daltons, 500 Daltons, 600 Daltons, 700 Daltons, 750 Daltons, 900 Daltons, 1000 Daltons, 2000 Daltons, 3000 Daltons, or 5000 Daltons.

9. The matrix according to any one of the preceding claims, wherein the polyethylene glycol of the first and/or at least second layer is a linear polyethylene glycol.

10. The matrix according to any one of the preceding claims, wherein the polyethylene glycol of the first layer has a smaller average molecular weight than the at least second layer.

11. The matrix according to any one of the preceding claims, wherein the polyethylene glycol (PEG) of the first layer is covalently bound to the solid substrate by a chemical reaction having occurred between said PEG of the first layer and a functional group of the solid substrate, wherein said functional group of the solid substrate is selected from an epoxy, acetyl, hydroxyl, amine, thiol, cystamine HCl, carboxy, aldehyde, squaric acid ester, active ester, maleimide, haloacetate, succinimidyl carbonate, octadecyl and/or iminodiacetic group, or a derivative thereof.

12. The matrix according to any one of the preceding claims, wherein the terminal functional group of the polyethylene glycol (PEG) for coupling the ligand to said PEG is selected from an epoxy, acetyl, hydroxyl, amine, maleimide, thiol, orthopyridyldisulfide (OPSS)), bromoacetamide and/or iodoacetamide group, or a derivative thereof.

13. The matrix according to any one of the preceding claims, wherein the solid substrate is capped by chemical modification, preferably by acetylation and/or hydrolysis.

14. The matrix according to any one of the preceding claims, wherein said matrix facilitates a ligand loading, obtained by coupling the functional group of the polyethylene glycol to the ligand, of 1 to 200 mg ligand/mL of matrix, preferably 10 to 100 mg ligand/mL matrix.

15. The matrix according to any one of the preceding claims, wherein the ligand is coupled to the polyethylene glycol of the at least second layer, preferably by covalent binding.

16. The matrix according to the preceding claim, wherein the ligand is configured to selectively bind or enzymatically process a target molecule in whole blood.

17. The matrix according to any one of claims 15 or 16, wherein the ligand is a polypeptide, preferably an antigen, antibody or fragment thereof, or an enzyme.

18. The matrix according to any one of claims 15 to 17, wherein the ligand is a polypeptide selected from an enzyme, antigen, antibody, or fragment thereof, configured to selectively capture and/or modify a target molecule from whole blood, wherein the resulting apheresis matrix has a target molecule binding capacity, where the target molecule is a protein, of 0.01 to 20 mg protein/mL of apheresis matrix, preferably 0.1 to 10 mg protein/mL apheresis matrix.

19. The matrix according to any one of claims 15 or 16, wherein the ligand is a nucleic acid molecule, preferably an aptamer, more preferably an RNA, a DNA, and/or a mirror-image aptamer.

20. The matrix according to any one of the preceding claims, wherein the solid substrate, the matrix and/or the matrix coupled to a ligand, are sterilizable or sterilized, preferably by heat and/or radiation.

21. A method for preparing an apheresis matrix, comprising coupling, preferably by covalent binding, the linear polyethylene glycol of the at least second layer of the matrix according to claim 1 to the ligand, preferably according to any one of claims 16-19.

22. A blood treatment device in the form of an adsorption cartridge or column, comprising the matrix according to any one of claims 15 to 20.

23. A blood treatment device according to the preceding claim, wherein the blood treatment device is located in an extracorporeal blood circuit through which the blood of a patient passes, and which comprises means for transporting blood from the patient's vascular system to the blood treatment device at a defined flow rate and for returning the treated blood back to the patient.

24. A ligand coupled to the polyethylene glycol of the matrix according to any one of claims 15 to 20 for use in the treatment of a medical condition by extracorporeal blood treatment, wherein said extracorporeal blood treatment comprises transporting blood from the patient's vascular system to said ligand at a defined flow rate and returning the treated blood back to the patient.

25. The ligand for use according to claim 24, wherein the medical condition is an inflammatory condition, an autoimmune disease, a condition associated with organ failure, transplantation and/or a degenerative disease.
